# EUROPEAN PATENT SPECIFICATION

(11) **EP 2 079 743 B1**
(45) Date of publication and mention of the grant of the patent: **25.01.2012**
(21) Application number: 07824247.6
(22) Date of filing: 19.10.2007
(51) Int. Cl.: C07D 475/00, A61K 31/519, A61P 35/00

(54) **PTERIDINE DERIVATIVES AS POLO-LIKE KINASE INHIBITORS USEFUL IN THE TREATMENT OF CANCER**
PTERIDINDERIVATE ALS INHIBITOREN DER POLO-LIKE-KINASE ZUR BEHANDLUNG VON KREBS
DÉRIVÉS DE PTÉRIDINE EN TANT QU'INHIBITEURS DES KINASES DE TYPE POLO UTILES DANS LE TRAITEMENT DU CANCER

(30) Priority: 25.10.2006 GB 0621205; 10.08.2007 GB 0715614
(43) Date of publication of application: 22.07.2009
(73) Proprietor: Chroma Therapeutics Limited, Abingdon Oxon OX14 4RY (GB)
(72) Inventor: MOFFAT, David Festus Charles, Abingdon Oxfordshire OX14 4RY (GB); PATEL, Sanjay Ratilal, Abingdon Oxfordshire OX14 4RY (GB); DAVIES, Stephen John, Abingdon Oxfordshire OX14 4RY (GB); BAKER, Kenneth William John, Abingdon Oxfordshire OX14 4RY (GB); PHILPS, Oliver James, Abingdon Oxfordshire OX14 4RY (GB)
(74) Representative: Walls, Alan James
(86) International application number: PCT/GB2007/003998
(87) International publication number: WO 2008/050096

(56) References cited:
- EP-A- 1 632 493
- US-A1- 2006 046 990
- US-A1- 2006 047 118

## Description

This invention relates to a series of amino acid esters, to compositions containing them, to processes for their preparation and to their use in medicine as Polo-like kinase 'PLK' inhibitors. Polo-like kinases (PLKs) are key enzymes that control mitotic entry of proliferating cells and regulate many aspects of mitosis necessary for successful cytokinesis. Of the four known human PLKs, PLK1 is the best characterized and is overexpressed in many tumour types with aberrant elevation frequently constituting a prognostic indicator of poor disease outcome. The compounds may be of use in the treatment of cell proliferative diseases such as cancer. The present invention encompasses compounds that are dihydropteridinine derivatives.

### Background to invention

The PLKs, a family of Ser/Thr protein kinases named after their functional and sequence similarity with the archetypal *polo* kinase from *Drosophila melanogaster,* play a variety of roles in mitosis (Nat. Rev. Mol. Cell Biol., 2001, 2, 21-32.). In yeasts (*Saccharomyces cerevisiae* and *S*. *pombe*) single PLKs exist, whereas four distinct PLKs have been identified to date in mammals. Human PLK1 (Cell Growth Differ., 1994, 5, 249-257), PLK2 (serum-inducible kinase, SNK, Mol. Cell. Biol., 1992, 12, 4164-4169), PLK3 (proliferation-related kinase, PRK J. Biol. Chem. 1997, 272, 28646-28651) and PLK4 (Onco/. Rep., 1997, 4, 505-510) are structurally homologous and contain two conserved domains, the N-terminal catalytic kinase domain, as well as a C-terminal region composed of the so-called polo boxes. Whereas PLK1, PLK2, and PLK3 are expressed in all tissues, PLK4 appears to possess unique physiological roles and the distribution of PLK4 mRNA in adults is restricted to certain tissues such as testes and thymus. PLK1 is the best characterized member of the PLK family and it appears to fulfil most of the known functions of the single PLKs present in invertebrates (Nat. Rev. Mol. Cell Biol., 2004, 5, 429-441). PLK1 protein levels fluctuate in a cell-cycle-dependent manner and its kinase activity peaks at the transition between the second gap phase and the mitosis phases (G2/M) of the eukaryotic cell division cycle. Upon exit from mitosis PLK1 levels drop as a result of ubiquitin-dependent proteolysis. PLK1 has been reported to be involved in the initiation of mitosis through activation of the cyclin-dependent kinase CDK1/cyclin B complex, *i.e*. the master switch for mitotic entry (mitosis-promoting factor, MPF Nature, 1990, 344, 503-508).

This occurs when PLK1 phosphorylates, and thus activates, the dual specificity phosphatase CDC25C, which in turn relieves premitotic MYT1- and WEE1- mediated suppression of CDK1/cyclin B activity through dephosphorylation at the CDK1 pThr14 and pTyr15 sites (Cell, 1991, 67, 197-211). Upon entry into mitosis, phosphorylation of CDC25C by PLK1 and PLK3 leads to its translocation into the nucleus. Apart from controlling entry into mitosis through CDK1 activation, PLK1 has additional roles in regulating progression through mitosis. It is involved in bipolar spindle formation, including centrosome maturation and regulation of the microtubule organizing centre, in the subsequent steps of mitosis involving sister chromatid separation, and finally in cytokinesis (Dev. Cell, 2003, 5, 127-138).

### Brief Summary of the Invention

Compounds of the invention are related to compounds disclosed in WO2004076454. They are inhibitors of PLK1 and the isoforms thereof. The compounds are thus of use in medicine, for example in the treatment of a variety of proliferative disease states, including cancers. The compounds are characterised by the presence in the molecule of an amino acid motif or an amino acid ester motif which is hydrolysable by an intracellular carboxylesterase. Compounds of the invention having the lipophilic amino acid ester motif cross the cell membrane, and are hydrolysed to the acid by the intracellular carboxylesterases. The polar hydrolysis product accumulates in the cell since it does not readily cross the cell membrane. Hence the PLK1 activity of the compound is prolonged and enhanced within the cell.

US patent publication No.2006/046990 relates to tetrahydropteridine derivatives said to be PLKI inhibitors useful for the treatment of cell proliferation diseases.

### Detailed Description of the Invention

According to the invention there is provided a compound of formula (I) as set out in claim 1 or a salt, N-oxide, hydrate or solvate thereof.

In the compounds of the invention, when R₁ is other than hydrogen, the carbon atom to which the R₁ substituent is attached is asymmetric. Preferably the stereo chemistry at that asymmetric center is R.

In another broad aspect the invention provides the use of a compound of formula (I) as defined above, or an N-oxide, salt, hydrate or solvate thereof in the preparation of a composition for inhibiting the activity of PLK1.

In one aspect of the invention, the compounds of the invention are useful for for treatment of cell proliferative diseases such as solid tumours and haemato-oncological tumours such as leukaemias and lymphomas.

The compounds of formula (I) may be used in a method for the treatment of the foregoing disease types, which comprises administering to a subject suffering such disease an effective amount of a compound of formula (I) as defined above.

### Terminology

As used herein, the term "(Cₐ-C_{b})alkyl" wherein a and b are integers, refers to a straight or branched chain alkyl radical having from a to b carbon atoms. Thus when a is 1 and b is 6, for example, the term includes methyl, ethyl, n-propyl, isopropyl, n-butyl, isobutyl, sec-butyl, t-butyl, n-pentyl and n-hexyl.

As used herein, the term "divalent (Cₐ-C_{b})alkylene radical", wherein a and b are integers, refers to a saturated hydrocarbon chain having from a to b carbon atoms and two unsatisfied valences.

As used herein, the term "(Cₐ-C_{b})alkenyl" wherein a and b are integers, refers to a straight or branched chain alkenyl moiety with a to b carbon atoms; having at least one double bond of either E or Z stereochemistry where applicable. The term includes, for example, vinyl, allyl, 1- and 2-butenyl and 2-methyl-2-propenyl.

As used herein, the term "divalent (Cₐ-C_{b})alkenylene radical" means a hydrocarbon chain having from a to b carbon atoms, at least one double bond, and two unsatisfied valences.

As used herein the term "Cₐ-C_{b} alkynyl", wherein a and b are integers refers to straight chain or branched chain hydrocarbon groups having from two to six carbon atoms and having in addition one triple bond. This term would include, for example, ethynyl, 1-propynyl, 1- and 2-butynyl, 2-methyl-2-propynyl, 2-pentynyl, 3-pentynyl, 4-pentynyl, 2-hexynyl, 3-hexynyl, 4-hexynyl and 5-hexynyl.

As used herein, the term "divalent (Cₐ-C_{b})alkynylene radical", wherein a and b are integers refers to a divalent hydrocarbon chain having from two to six carbon atoms, and at least one triple bond.

As used herein, the term "carbocyclic" refers to a mono-, bi- or tricyclic radical having up to 16 ring atoms, all of which are carbon, and includes aryl and cycloalkyl.

As used herein, the term "cycloalkyl" refers to a monocyclic saturated carbocyclic radical having from 3-8 carbon atoms and includes, for example, cyclopropyl, cyclobutyl, cyclopentyl, cyclohexyl, cycloheptyl and cyclooctyl.

As used herein, the unqualified term "aryl" refers to a mono-, bi- or tri-cyclic carbocyclic aromatic radical, and includes radicals having two monocyclic carbocyclic aromatic rings which are directly linked by a covalent bond. Illustrative of such radicals are phenyl, biphenyl and napthyl.

As used herein, the unqualified term "heteroaryl" refers to a mono-, bi- or tri-cyclic aromatic radical containing one or more heteroatoms selected from S, N and O, and includes radicals having two such monocyclic rings, or one such monocyclic ring and one monocyclic aryl ring, which are directly linked by a covalent bond. Illustrative of such radicals are thienyl, benzthienyl, furyl, benzfuryl, pyrrolyl, imidazolyl, benzimidazolyl, thiazolyl, benzthiazolyl, isothiazolyl, benzisothiazolyl, pyrazolyl, oxazolyl, benzoxazolyl, isoxazolyl, benzisoxazolyl, isothiazolyl, triazolyl, benztriazolyl, thiadiazolyl, oxadiazolyl, pyridinyl, pyridazinyl, pyrimidinyl, pyrazinyl, triazinyl, indolyl and indazolyl.

As used herein, the unqualified term "heterocyclyl" or "heterocyclic" includes "heteroaryl" as defined above, and in its non-aromatic meaning relates to a mono-, bi- or tri-cyclic non-aromatic radical containing one or more heteroatoms selected from S, N and O, and to groups consisting of a monocyclic non-aromatic radical containing one or more such heteroatoms which is covalently linked to another such radical or to a monocyclic carbocyclic radical. Illustrative of such radicals are pyrrolyl, furanyl, thienyl, piperidinyl, imidazolyl, oxazolyl, isoxazolyl, thiazolyl, thiadiazolyl, pyrazolyl, pyridinyl, pyrrolidinyl, pyrimidinyl, morpholinyl, piperazinyl, indolyl, morpholinyl, benzfuranyl, pyranyl, isoxazolyl, benzimidazolyl, methylenedioxyphenyl, ethylenedioxyphenyl, maleimido and succinimido groups.

A "divalent phenylene, pyridinylene, pyrimidinylene, pyrazinylene, piperidinylene, piperazinylene, pyrrolidenylene, pyrrolene, cyclopropylene, cyclobutylene, cyclopentylene, cyclohexylene or 3-aza-bicyclo[3.1.0]hexylene, radical" is a benzene, pyridine, pyrimidine, pyrazine, piperidine, piperazine, pyrrolidene, pyrrole, cyclopropyl, cyclobutylene, cyclopentyl, cyclohexyl or 3-aza-bicyclo[3.1.0]hexyl ring, with two unsatisfied valencies, and includes 1,3-phenylene, 1,4-phenylene, and the following:

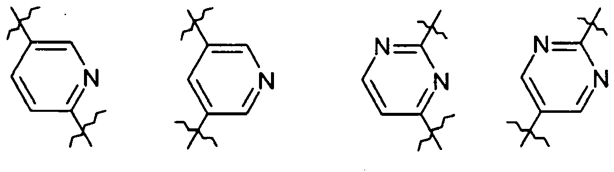

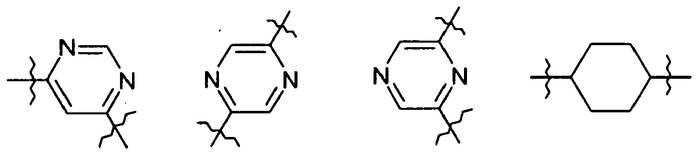

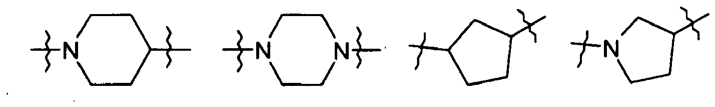

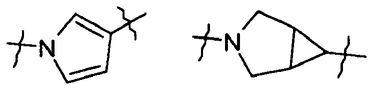

Unless otherwise specified in the context in which it occurs, the term "substituted", as applied to any moiety herein, means substituted with up to four compatible substituents, each of which independently may be, for example, (C₁-C₆)alkyl, (C₁-C₆)alkoxy, hydroxy, hydroxy(C₁-C₆)alkyl, mercapto, mercapto(C₁-C₆)alkyl, (C₁-C₆)alkylthio, phenyl, halo (including fluoro, bromo and chloro), trifluoromethyl, trifluoromethoxy, nitro, nitrile (-CN), oxo, -COOH, -COOR^{A}, -COR^{A}, -SO₂R^{A}, -CONH₂, -SO₂NH₂, -CONHR^{A}, -SO₂NHR^{A}, -CONR^{A}R^{B}, -SO₂NR^{A}R^{B}, -NH₂, -NHR^{A}, -NR^{A}R^{B}, -OCONH₂, -OCONHR^{A}, -OCONR^{A}R^{B}, -NHCOR^{A}, -NHCOOR^{A}, -NR^{B}COOR^{A}, -NHSO₂OR^{A}, -NR^{B}SO₂OH, -NR^{B}SO₂OR^{A}, -NHCONH₂, -NR^{A}CONH₂, -NHCONHR^{B}, -NR^{A}CONHR^{B}, -NHCONR^{A}R^{B}, or -NR^{A}CONR^{A}R^{B} wherein R^{A} and R^{B} are independently a (C₁-C₆)alkyl, (C₃-C₆) cycloalkyl, phenyl or monocyclic heteroaryl having 5 or 6 ring atoms, or R^{A} and R^{B} when attached to the same nitrogen atom form a cyclic amino group (for example morpholino, piperidinyl, piperazinyl, or tetrahydropyrrolyl). An "optional substituent" may be one of the foregoing substituent groups.

As used herein the term "salt" includes base addition, acid addition and quaternary salts. Compounds of the invention which are acidic can form salts, including pharmaceutically acceptable salts, with bases such as alkali metal hydroxides, e.g. sodium and potassium hydroxides; alkaline earth metal hydroxides e.g. calcium, barium and magnesium hydroxides; with organic bases e.g. N-methyl-D-glucamine, choline tris(hydroxymethyl)amino-methane, L-arginine, L-lysine, N-ethyl piperidine, dibenzylamine and the like. Those compounds (I) which are basic can form salts, including pharmaceutically acceptable salts with inorganic acids, e.g. with hydrohalic acids such as hydrochloric or hydrobromic acids, sulphuric acid, nitric acid or phosphoric acid and the like, and with organic acids e.g. with acetic, tartaric, succinic, fumaric, maleic, malic, salicylic, citric, methanesulphonic, p-toluenesulphonic, benzoic, benzenesunfonic, glutamic, lactic, and mandelic acids and the like.

Compounds of the invention which contain one or more actual or potential chiral centres, because of the presence of asymmetric carbon atoms, can exist as a number of diastereoisomers with R or S stereochemistry at each chiral centre. The invention includes all such diastereoisomers and mixtures thereof.

The term "ester" or "esterified carboxyl group" in connection with substituent R₇ above means a group RₓO(C=O)- in which Rₓ is the group characterising the ester, notionally derived from the alcohol RₓOH.

### The substituents R₁-R₃'

R₁ is hydrogen, (C₁-C₆)alkyl, for example methyl, ethyl, n- or iso-propyl, (C₂-C₆)alkenyl, for example allyl, (C₂-C₆)alkynyl, for example -CH₂C≡CH or (C₃-C₆)cycloalkyl, for example cyclopropyl, cyclopentyl or cyclohexyl. In one subclass of compounds of the invention R₁ is ethyl.

R₂ is hydrogen, (C₁-C₆)alkyl, for example methyl, ethyl, n- or iso-propyl, (C₂-C₆)alkenyl, for example allyl, (C₂-C₆)alkynyl, for example -CH₂C≡CH or (C₃-C₆)cycloalkyl, for example cyclopropyl, cyclopentyl or cyclohexyl, or C₆₋₁₄ aryl for example phenyl or naphthyl. In one subclass of compounds of the invention R₂ is cyclopentyl.

R₃ and R₃' are independently selected from hydrogen, -CN, hydroxyl, halogen, (C₁-C₆)alkyl, for example methyl, ethyl, n- or iso-propyl, (C₂-C₆)alkenyl, for example allyl, (C₂-C₆)alkynyl, for example -CH₂C≡CH or (C₃-C₆)cycloalkyl, for example cyclopropyl, cyclopentyl or cyclohexyl, -NR₅R₆ and C₁-C₄ alkoxy, wherein R₅ and R₆ are independently hydrogen or optionally substituted (C₁-C₆)alkyl, for example methyl or ethyl. In one subclass of compounds of the invention R₃ is methoxy, fluoro or chloro, and R'₃ is hydrogen, fluoro or chloro.

### The ring A

Ring A is a phenyl, pyridinyl or pyrimidinylring.

Ring A may be substituted by any of the optional substituents referred to above, for example chloro, bromo or fluoro, trifluoromethyl, methoxy, and trifluoromethoxy.

### The substituent T

This substituent contains the alpha amino acid or alpha amino acid ester moiety of formula (X) or (Y), linked through a linker radical to ring A.

The ester compounds of the invention are converted by intracellular esterases to the carboxylic acid. Both the esters and carboxylic acids may have PLK inhibitory activity in their own right. The compounds of the invention therefore include not only the ester, but also the corresponding carboxylic acid hydrolysis products.

The ester group R₇ present in substituent T must be one which in the compound of the invention is hydrolysable by one or more intracellular carboxylesterase enzymes to a carboxylic acid group. Intracellular carboxylesterase enzymes capable of hydrolysing the ester group of a compound of the invention to the corresponding acid include the three known human enzyme isotypes hCE-1, hCE-2 and hCE-3. Although these are considered to be the main enzymes other enzymes such as biphenylhydrolase (BPH) may also have a role in hydrolysing the conjugates. In general, if the carboxylesterase hydrolyses the free amino acid ester to the parent acid it will also hydrolyse the ester motif when covalently conjugated to the modulator. Hence, the broken cell assay described herein provides a straightforward, quick and simple first screen for esters which have the required hydrolysis profile. Ester motifs selected in that way may then be re-assayed in the same carboxylesterase assay when conjugated to the rest of the molecule via the chosen conjugation chemistry, to confirm that it is still a carboxylesterase substrate in that background.

Subject to the requirement that they be hydrolysable by intracellular carboxylesterase enzymes, examples of particular ester groups R₇ include those of formula -(C=O)OR₁₀ wherein R₁₀ is R₁₁R₁₂R₁₃C- wherein
(i) R₁₁ is hydrogen or optionally substituted (C₁-C₃)alkyl-(Z¹)ₐ-[(C₁-C₃)alkyl]_{b}- or (C₂-C₃)alkenyl-(Z¹)ₐ-[(C₁-C₃)alkyl]_{b}- wherein a and b are independently 0 or 1 and Z¹ is -O-, -S-, or -NR₁₄- wherein R₁₄ is hydrogen or (C₁-C₃)alkyl; and R₁₂ and R₁₃ are independently hydrogen or (C₁-C₃)alkyl-;
(ii) R₁₁ is hydrogen or optionally substituted R₁₅R₁₆N-(C₁-C₃)alkyl- wherein R₁₅ is hydrogen or (C₁-C₃)alkyl and R₁₆ is hydrogen or (C₁-C₃)alkyl; or R₁₅ and R₁₆ together with the nitrogen to which they are attached form an optionally substituted monocyclic heterocyclic ring of 5- or 6- ring atoms or bicyclic heterocyclic ring system of 8 to 10 ring atoms, and R₁₂ and R₁₃ are independently hydrogen or (C₁-C₃)alkyl-;or
(iii) R₁₁ and R₁₂ taken together with the carbon to which they are attached form an optionally substituted monocyclic carbocyclic ring of from 3 to 7 ring atoms or bicyclic carbocyclic ring system of 8 to 10 ring atoms, and R₁₃ is hydrogen.

Within these classes, R₁₀ may be, for example, methyl, ethyl, n- or iso-propyl, n-, sec- or tert-butyl, cyclohexyl, allyl, phenyl, benzyl, 2-, 3- or 4-pyridylmethyl, N-methytpiperidin-4-yl, tetrahydrofuran-3-yl, methoxyethyl, indanyl, norbonyl, dimethylaminoethyl, or morpholinoethyl. Currently preferred is where R₁₀ is cyclopentyl or tert-butyl.

### The ring D

When R is a group of formula (Y), examples of R include: 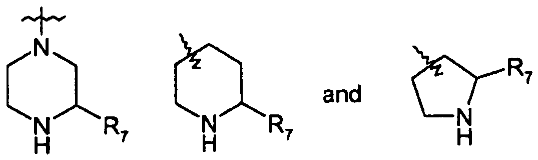 wherein R₇ is as defined and discussed above.

### The group R₈

The group R₈ is present in the compounds of the invention when R in formula (I) is a radical of formula (X)

R₈ may be, for example, optionally substituted (C₁-C₆)alkyl, (C₃-C₆)cycloalkyl, phenyl or pyridyl, for example methyl, ethyl, n-or isopropyl, cyclopropyl, cyclopentyl, or cyclohexyl. R₈ may also be, for example hydrogen or -(C=O)R₁₆, wherein R₁₆ is optionally substituted (C₁-C₆)alkyl such as methyl, ethyl, n-or isopropyl, or n-, iso- or sec-butyl, (C₃-C₆)cycloalkyl such as cyclopropyl, cyclopentyl, cyclohexyl, phenyl, pyridyl, thienyl, phenyl(C₁-C₆alkyl)-, thienyl(C₁-C₆alkyl)- or pyridyl(C₁-C₆alkyl)- such as benzyl, 4-methoxyphenylmethylcarbonyl, thienylmethyl or pyridylmethyl.

R₈ may also be, for example -(C=O)OR₁₇, or -(C=O)NHR₁₇ wherein R₁₇ is hydrogen or optionally substituted (C₁-C₆)alkyl such as methyl, ethyl, or n-or isopropyl.

Currently it is preferred that R₈ be hydrogen.

For compounds of the invention which are to be administered systemically, esters with a slow rate of esterase cleavage are preferred, since they are less susceptible to pre-systemic metabolism. Their ability to reach their target tissue intact is therefore increased, and the ester can be converted inside the cells of the target tissue into the acid product. However, for local administration, where the ester is either directly applied to the target tissue or directed there by, for example, inhalation, it will often be desirable that the ester has a rapid rate of esterase cleavage, to minimise systemic exposure and consequent unwanted side effects. If a carbon atom to which the group R is attached is unsubstituted, ie R is attached to a methylene (-CH₂)- radical, then the esters tend to be cleaved more rapidly than if that carbon is substituted, or is part of a ring system such as a phenyl or cyclohexyl ring.

### The radical -L¹-Y¹-

This radical (or bond) arises from the particular chemistry strategy chosen to link the amino acid ester motif R in substituent T to ring A of the inhibitor. Clearly the chemistry strategy for that coupling may vary widely, and thus many combinations of the variables Y¹ and L¹ are possible. However, when the inhibitor is bound to the enzyme at its active site, the amino acid ester motif generally extends in a direction away from the enzyme, and thus minimises or avoids interference with the binding mode of the inhibitor. Hence the precise combination of variable making up the linking chemistry between the amino acid ester motif and the rest of the molecule will often be irrelevant to the primary binding mode of the compound as a whole.

With the foregoing general observations in mind, taking the variables making up the radical -L¹-Y¹- in turn:
Y¹ may be, for example, -NR₃-, -S-, -O-, -C(=O)NR₃-, - NR₃C(=O)-, or -C(=O)O-, wherein R₃ is hydrogen or optionally substituted C₁-C₆ alkyl such as -CH₂CH₂OH;
In the radical L¹, examples of Alk¹ and Alk² radicals, when present, include -CH₂-, -CH₂CH₂- -CH₂CH₂CH₂-, -CH₂CH(OH)CH₂-, -CH₂CH₂CH₂CH₂-. -CH=CH-, -CH=CHCH₂-, -CH₂CH=CH-, CH₂CH=CHCH₂-, -C≡C-, -C≡CCH₂-, -CH₂C≡C-, and CH₂C≡CCH₂. Additional examples of Alk¹ and Alk² include, in either orientation, -CH₂W-, -CH₂CH₂W-, -CH₂CH₂WCH₂-, -CH₂CH₂WCH(CH₃)-, -CH₂WCH₂CH₂-, -CH₂WCH₂CH₂WCH₂-, and -WCH₂CH₂- where W is -O-, -S-, -NH-, -N(CH₃)-, or -CH₂CH₂N(CH₂CH₂OH)CH₂-. Further examples of Alk¹ and Alk² include divalent cyclopropyl, cyclopentyl and cyclohexyl radicals.
Alk¹ and Alk² when present may also be branched chain alkyl such as -CH(CH₃)-, -C(CH₃)₂-, or in either orientation -CH₂CH(CH₃)-, -CH₂C(CH₃)₂-.

In L¹, when n is 0, the radical is a hydrocarbon chain (optionally substituted for example by hydroxyl) and perhaps having an ether, thioether or amino linkage). Presently it is preferred that there be no optional substituents in L¹. When both m and p are 0, L¹ is a divalent mono- or bicyclic carbocyclic or heterocyclic radical with 5 - 13 ring atoms (optionally substituted). When n is 1 and at least one of m and p is 1, L¹ is a divalent radical including a hydrocarbon chain or chains and a mono- or bicyclic carbocyclic or heterocyclic radical with 5 - 13 ring atoms (optionally substituted). When present, Q may be, for example, a divalent phenylene, pyridinylene, pyrimidinylene, pyrazinylene, piperidinylene, piperazinylene, pyrrolidenylene, pyrrolene, cyclopropylene, cyclobutylene, cyclopentylene, cyclohexylene or 3-aza-bicyclo[3.1.0]hexylene, radical, but 1,4-phenylene, 1,4-piperidinylene, or 1,4- piperazinyl are presently preferred.

Specific examples of the radical -L¹-Y¹- include those present in the compounds of the Examples herein.

A particular subclass of compounds of the invention consists of those of formula (IA) 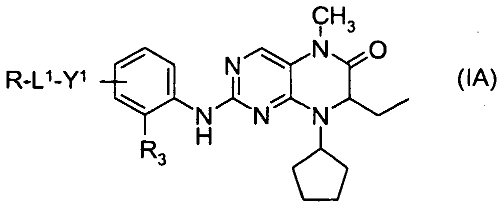 wherein R₃ is methoxy, fluoro or chloro, and the remaining variables are as defined and discussed above.

As mentioned above, the compounds with which the invention is concerned are inhibitors of PLK1 kinase activity and are therefore of use for treatment of cell proliferative diseases such as cancer.

It will be understood that the specific dose level for any particular patient will depend upon a variety of factors including the activity of the specific compound employed, the age, body weight, general health, sex, diet, time of administration, route of administration, rate of excretion, drug combination and the severity of the particular disease undergoing treatment. Optimum dose levels and frequency of dosing will be determined by clinical trial.

The compounds with which the invention is concerned may be prepared for administration by any route consistent with their pharmacokinetic properties. The orally administrable compositions may be in the form of tablets, capsules, powders, granules, lozenges, liquid or gel preparations, such as oral, topical, or sterile parenteral solutions or suspensions. Tablets and capsules for oral administration may be in unit dose presentation form, and may contain conventional excipients such as binding agents, for example syrup, acacia, gelatin, sorbitol, tragacanth, or polyvinyl-pyrrolidone; fillers for example lactose, sugar, maize-starch, calcium phosphate, sorbitol or glycine; tabletting lubricant, for example magnesium stearate, talc, polyethylene glycol or silica; disintegrants for example potato starch, or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, for example sorbitol, syrup, methyl cellulose, glucose syrup, gelatin hydrogenated edible fats; emulsifying agents, for example lecithin, sorbitan monooleate, or acacia; non-aqueous vehicles (which may include edible oils), for example almond oil, fractionated coconut oil, oily esters such as glycerine, propylene glycol, or ethyl alcohol; preservatives, for example methyl or propyl p-hydroxybenzoate or sorbic acid, and if desired conventional flavouring or colouring agents.

For topical application to the skin, the drug may be made up into a cream, lotion or ointment. Cream or ointment formulations which may be used for the drug are conventional formulations well known in the art, for example as described in standard textbooks of pharmaceutics such as the British Pharmacopoeia.

For topical application by inhalation, the drug may be formulated for aerosol delivery for example, by pressure-driven jet atomizers or ultrasonic atomizers, or preferably by propellant-driven metered aerosols or propellant-free administration of micronized powders, for example, inhalation capsules or other "dry powder" delivery systems. Excipients, such as, for example, propellants (e.g. Frigen in the case of metered aerosols), surface-active substances, emulsifiers, stabilizers, preservatives, flavourings, and fillers (e.g. lactose in the case of powder inhalers) may be present in such inhaled formulations. For the purposes of inhalation, a large number of apparata are available with which aerosols of optimum particle size can be generated and administered, using an inhalation technique which is appropriate for the patient. In addition to the use of adaptors (spacers, expanders) and pear-shaped containers (e.g. Nebulator®, Volumatic®), and automatic devices emitting a puffer spray (Autohaler®), for metered aerosols, in particular in the case of powder inhalers, a number of technical solutions are available (e.g. Diskhaler®, Rotadisk®, Turbohaler® or the inhalers for example as described in European Patent Application EP 0 505 321).

For topical application to the eye, the drug may be made up into a solution or suspension in a suitable sterile aqueous or non aqueous vehicle. Additives, for instance buffers such as sodium metabisulphite or disodium edeate; preservatives including bactericidal and fungicidal agents such as phenyl mercuric acetate or nitrate, benzalkonium chloride or chlorhexidine, and thickening agents such as hypromellose may also be included.

The active ingredient may also be administered parenterally in a sterile medium. Depending on the vehicle and concentration used, the drug can either be suspended or dissolved in the vehicle. Advantageously, adjuvants such as a local anaesthetic, preservative and buffering agents can be dissolved in the vehicle.

The compounds of the invention may be used in conjunction with a number of known pharmaceutically active substances. For example, the compounds of the invention may be used with cytotoxics, HDAC inhibitors, kinase inhibitors, aminopeptidase inhibitors, protease inhibitors, bcl-2 antagonists, inhibitors of mTor and monoclonal antibodies (for example those directed at growth factor receptors). Preferred cytotoxics include, for example, taxanes, platins, anti-metabolites such as 5-fluoracil, topoisomerase inhibitors and the like. The medicaments of the invention comprising amino acid derivatives of formula (I), tautomers thereof or pharmaceutically acceptable salts, N-oxides, hydrates or solvates thereof therefore typically further comprise a cytotoxic, an HDAC inhibitor, a kinase inhibitor, an aminopeptidase inhibitor and/or a monoclonal antibody.

Further, the present invention provides a pharmaceutical composition comprising:
(a) a compound (I), or a pharmaceutically acceptable salt, N-oxide, hydrate or solvate thereof;
(b) a cytotoxic agent, an HDAC inhibitor, a kinase inhibitor, an aminopeptidase inhibitor, a protease inhibitor, a bcl-2 antagonist, an inhibitor of mTor and/or a monoclonal antibody; and
(c) a pharmaceutically acceptable carrier or diluent.

Also provided is a product comprising:
(a) a compound (I), or a pharmaceutically acceptable salt, N-oxide, hydrate or solvate thereof; and
(b) a cytotoxic agent, an HDAC inhibitor, a kinase inhibitor, an aminopeptidase inhibitor, a protease inhibitor, a bcl-2 antagonist, an inhibitor of mTor and/or a monoclonal antibody,
for the separate, simultaneous or sequential use in the treatment of the human or animal body.

### Synthesis

There are multiple synthetic strategies for the synthesis of the compounds (I) with which the present invention is concerned, but all rely on known chemistry, known to the synthetic organic chemist. Thus, compounds according to formula (I) can be synthesised according to procedures described in the standard literature and are well-known to those skilled in the art. Typical literature sources are *"*Advanced organic chemistry", 4th Edition(Wiley), J March; *"*Comprehensive Organic Transformation", 2nd Edition (Wiley), R.C. Larock , *"*Handbook of Heterocyclic Chemistry', 2nd Edition (Pergamon), A.R. Katritzky; review articles such as found in *"Synthesis", "Acc. Chem. Res*.", "*Chem*. *Rev*", or primary literature sources identified by standard literature searches online or from secondary sources such as *"Chemical Abstracts"* or *"Beilstein".*

The compounds of the invention may be prepared by a number of processes some of which are described specifically in the Examples below. In the reactions described below, it may be necessary to protect reactive functional groups, for example hydroxyl, amino and carboxy groups, where these are desired in the final product, to avoid their unwanted participation in the reactions [see for example, "Protecting Groups in Organic Synthesis", 3rd Edition, (Wiley), T.W. Greene]. Conventional protecting groups may be used in conjunction with standard practice. In some instances deprotection may be the final step in the synthesis of a compound of general formula (I), and the processes according to the invention described herein after are understood to extend to such removal of protecting groups.

### Abbreviations

AcOH = acetic acid
Boc or boc = *tert*-butoxycarbonyl
BOC₂O = Di-*tert*-butyldicarbonate
Cbz = benzyloxycarbonyl
DBU = 1,8-diazabicyclo[5.4.0]undec-7-ene
DCE = dichloroethane
DCM = dichloromethane
DIPEA = diisopropylethylamine
DMAP = dimethylaminopyridine
DMF = dimethylformamide
DMSO = dimethyl sulfoxide
EDC = 1-ethyl-3-(3-dimethylaminopropyl)carbodiimide
EtOAc = ethyl acetate
EtOH = ethanol
Et₂O = diethyl ether
Et₃N = triethylamine
H₂SO₄ = sulphuric acid
HCl = hydrochloric acid
HOBt = N-hydroxybenzotriazole
K₂CO₃ = potassium carbonate
LiOH = lithium hydroxide
MeOH = methanol
MgSO₄ = magnesium sulphate
Na₂CO₃ = sodium carbonate
NaH = sodium hydride
NaHCO₃ = sodium hydrogen carbonate
Nal = sodium iodide
NaOH = sodium hydroxide
NBS = *N*-bromo succinimide
NBu₄Br = tetrabutylammonium bromide
NMM = *N*-methyl morpholine
Pd(dppf)Cl₂ = dichloro-(1,2-bis-(diphenylphosphino)ethane)-palladium(II)
Pd/C = palladium on carbon
PPh₃ = triphenyl phosphine
PyBrOP = Bromo-tris-pyrrolidinophosphoniumhexafluorophosphate
STAB = sodium triacetoxyborohydride
TBTU = *O*-benzotriazol-1-yl-*N*,*N*,*N*',*N*'-tetramethyluronium tetrafluoroborate
TFA = trifluoroacetic acid
THF = tetrahydrofuran
aq = aqueous
g = gram(s)
LCMS = high performance liquid chromatography/mass spectrometry
mg = milligram(s)
min = minutes
mL = milliliter(s)
µL = microlitre(s)
mol = mole(s)
mmol = millimole(s)
NMR = nuclear magnetic resonance
RT or rt = room temperature
sat = saturated

Commercially available reagents and solvents (HPLC grade) were used without further purification. Solvents were removed using a Buchi rotary evaporator. Microwave irradiation was carried out using a Biotage Initiator™ Eight microwave synthesiser. Purification of compounds by flash chromatography column was performed using silica gel, particle size 40-63µm (230-400 mesh) obtained from Fluorochem. Purification of compounds by preparative HPLC was performed on Gilson systems using reverse phase Axial™ prep Luna C18 columns (10µm, 100 x 21.2mm), gradient 0-100% B (A = water / 0.05% TFA, B = acetonitrile / 0.05% TFA) over 10 min, flow = 25mUmin, UV detection at 254nm.

¹H NMR spectra were recorded on a Bruker 300 MHz AV spectrometer in deuterated solvents. Chemical shifts (δ) are in parts per million. Thin-layer chromatography (TLC) analysis was performed with Kieselgel 60 F₂₅₄ (Merck) plates and visualized using UV light.

Analytical HPLC/MS was performed on an Agilent HP1100 LC system using reverse phase Luna C18 columns (3µm, 50 x 4.6mm), gradient 5-95% B (A = water / 0.1% Formic acid, B = acetonitrile / 0.1 % Formic acid) over 2.25 min, flow = 2.25mUmin. UV spectra were recorded at 220 and 254nm using a G1315B DAD detector. Mass spectra were obtained over the range m/z 150 to 800 on a LC/MSD SL G1956B detector. Data were integrated and reported using ChemStation and ChemStation Data Browser softwares.

### Intermediates

The intermediates for the preparation of the examples described herein are shown below (Figure 1):

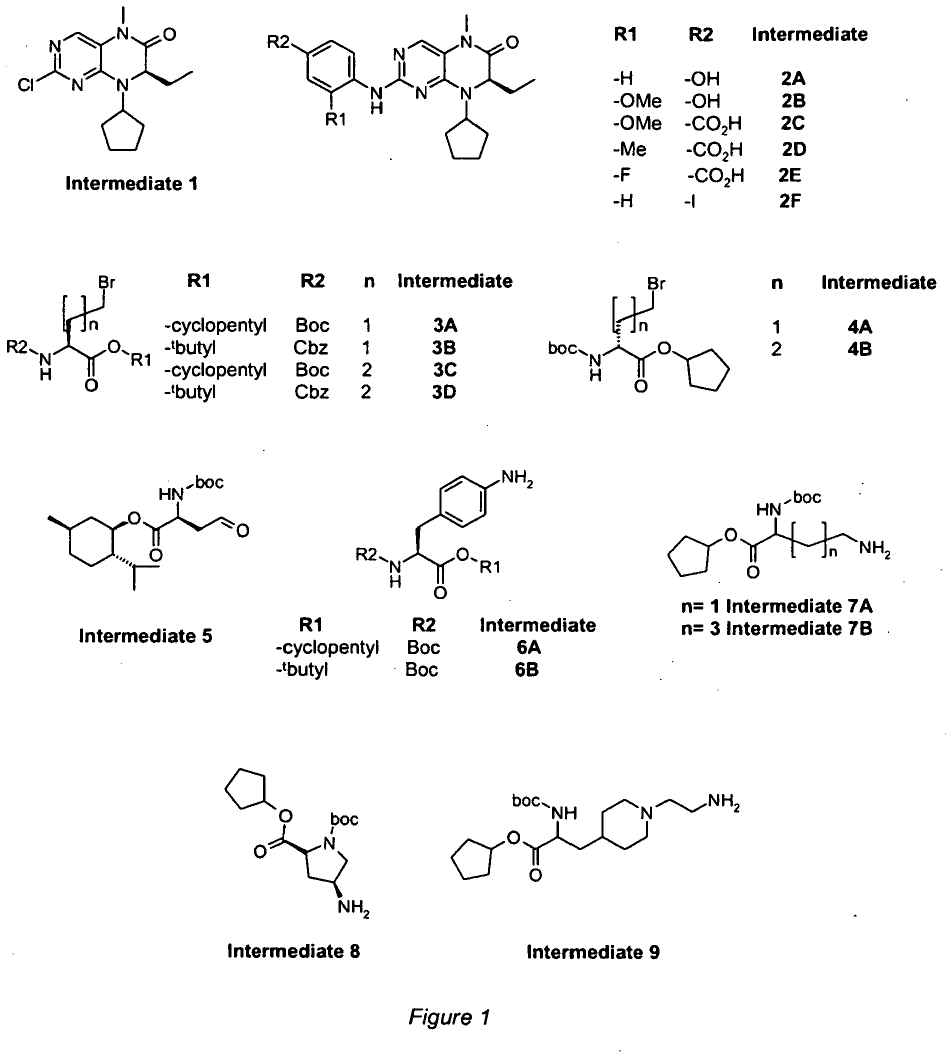

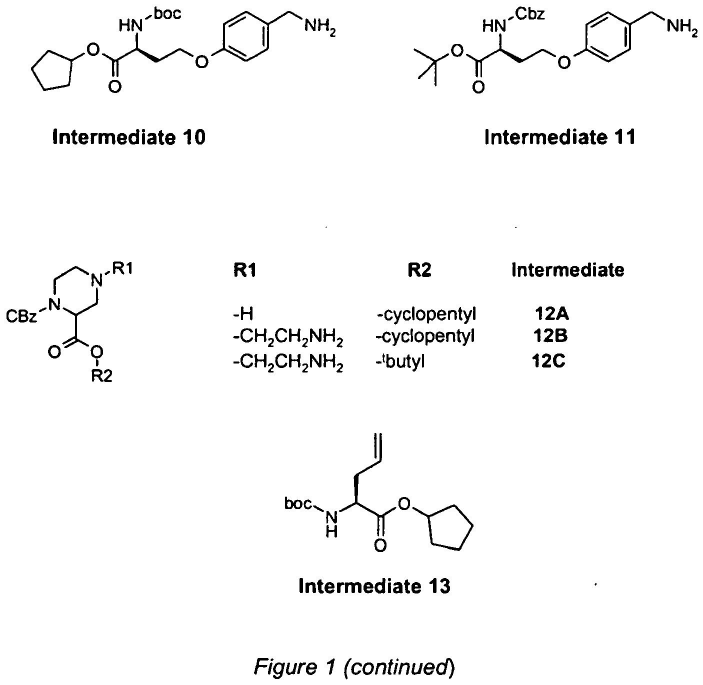

### Intermediate 1:

### (7R)-2-Chloro-8-cyclopentyl-7-ethyl-5-methyl-7,8-dihydropteridin-6(5H)-one

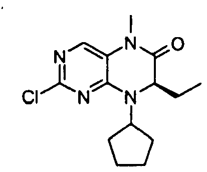

The title intermediate was prepared using methodology described in WO2004076454.

### Intermediates 2A - 2F

### General Procedure

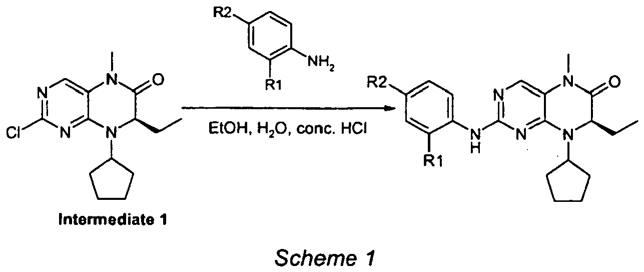

### Intermediate 2A:

### (7R)-8-Cyclopentyl-7-ethyl-2-[(4-hydroxyphenyl)amino]-5-methyl-7,8-dihydropteridin-6(5H)-one

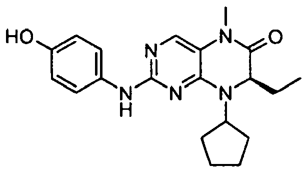

The title intermediate was prepared from Intermediate 1 according to the general procedure (Scheme 1).

To a solution of (7*R*)-2-chloro-8-cyclopentyl-7-ethyl-5-methyl-7,8-dihydropteridin-6(5*H*)-one [Intermediate 1] (200mg, 0.68mmol) in EtOH (2mL), water (8mL) and concentrated HCl (0.2mL) was added 4-aminophenol (148mg, 1.36mmol). The reaction mixture was refluxed for 18 hours and concentrated under reduced pressure. The residue was partitioned between sat. NaHCO₃ (20mL) and a mixture of MeOH/DCM (1:3, 20mL). The aqueous layer was separated and extracted with MeOH/DCM (1:3, 20 mL). The combined organic layers were dried (MgSO₄) and concentrated under reduced pressure to leave a brown solid. Trituration with Et₂O afforded the titled intermediate as a grey solid (125mg, 50% yield). ESMS: m/z 368 [M+H]⁺. ¹H NMR (DMSO-*d*₆. 300 MHz) 8.90 (1H, s), 8.64 (1H, s), 7.74 (1H, s), 7.43 (2H, d, J=8.9 Hz), 6.64 (2H, d, J=8.9 Hz), 4.39-4.29 (1H, m), 4.16 (1H, dd, J=3.6, 7.8 Hz), 3.22 (3H, s), 1.99-1.54 (10H, m), 0.77 (3H, t, J=7.4 Hz).

The intermediates in the table below were prepared by methods analogous to the method described above.

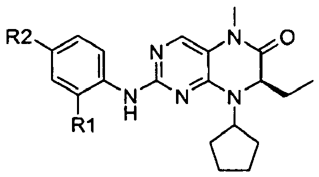

| **Intermediate** | **R1** | **R2** | **Name** | **ESMS** |
|---|---|---|---|---|
| 2B | -OMe | -OH | (7*R*)-8-Cyclopentyl-7-ethyl-2-[(4-hydroxy 2-methoxyphenyl)amino]-5-methyl-7,8-dihydropteridin-6(5*H*)-one | m/z 398 [M+H]⁺ |
| 2C | -OMe | -CO₂H | 4-{[(7*R*)-8-Cyclopentyl-7-ethyl-5-methyl-6-oxo-5,6,7,8-tetrahydropteridin-2-yl] amino}-3-methoxybenzoic acid | m/z 426 [M+H]⁺ |
| 2D | -Me | -CO₂H | 4-{[(7*R*)-8-Cyclopentyl-7-ethyl-5-methyl-6-oxo-5,6,7,8-tetrahydropteridin-2-yl] amino}-3-methylbenzoic acid | m/z 410 [M+H]⁺ |
| 2E | -F | -CO₂H | 4-{[(7*R*)-8-Cyclopentyl-7-ethyl-5-methyl-6-oxo-5,6,7,8-tetrahydropteridin-2-yl] amino}-3-fluorobenzoic acid | m/z 414 [M+H]⁺ |
| 2F | -H | -I | (7*R*)-8-Cyclopentyl-7-ethyl-2-[(4-iodophenyl)amino]-5-methyl-7,8-dihydro pteridin-6(5*H*)-one | m/z 478 [M+H]⁺ |

### Intermediate 3A:

### Cyclopentyl (2S)-4-bromo-2-[(tert-butoxycarbonyl)aminolbutanoate

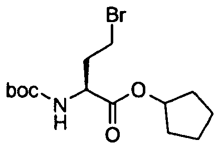

The title intermediate was prepared according to the procedure outlined below (Scheme 2).

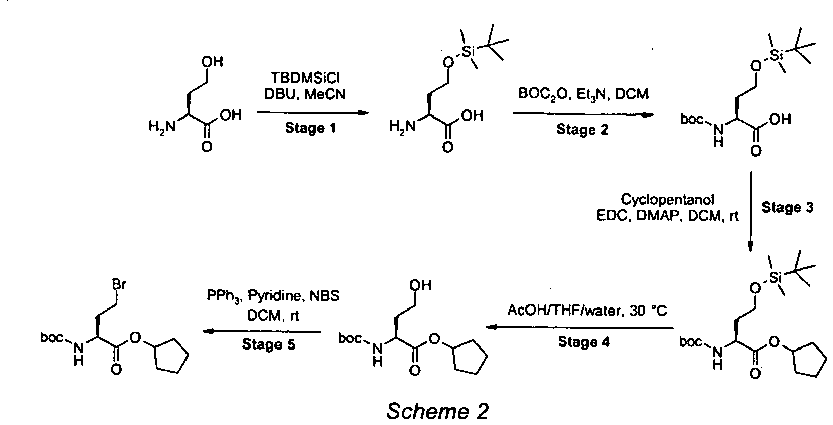

### Stage 1- O-[tert-butyl(dimethyl)silyl]-L-homoserine

To a suspension of L-homoserine (1.00g, 8.40mmol) in acetonitrile (10mL) at 0 °C was added DBU (1.32mL, 8.80mmol,). *tert*-Butyl-dimethyl silyl chloride (1.33g, 8.80mmol) was then added portionwise over 5 minutes and the reaction mixture allowed to warm to RT and stirred for 16 hours. The white solid was filtered and washed with acetonitrile to give the product (1.80g, 92% yield). ESMS: m/z 234 [M+H]⁺.

### Stage 2- N-(tert-butoxycarbonyl)-O-[tert-butyl(dimethyl)silyl]-L-homoserine

To a suspension of *O*-[*tert*-butyl(dimethyl)silyl]-L-homoserine (1.80g, 7.70mmol) in DCM (100mL) at 0°C was added Et₃N (2.15mL, 15.4mmol) and BOC₂O (1.77g, 8.10mmol). The reaction mixture was stirred at RT for 16 hours. The DCM was removed under reduced pressure and the residue was re-dissolved in EtOAc (20ml) and brine (10ml). The EtOAc layer was dried (MgSO₄) and concentrated under reduced pressure to give the crude product which was taken forward without further purification (2.53g, 99% yield). ESMS: m/z 356 [M+H]⁺.

**Stage 3**- Cyclopentyl *N*-(*tert*-butoxycarbonyl)-*O*-[*tert*-butyl(dimethyl)silyl]-L-homoserinate To a solution of *N*-(tert-butoxycarbonyl)-*O*-[*tert*-butyl(dimethyl)silyl]-L-homoserine (2.53g, 7.6mmol) in DCM (50mL) at 0°C was added cyclopentanol (1.39mL, 15.3mmol), EDC (1.61g, 8.40mmol) and DMAP (93mg, 0.76mmol). The reaction mixture was stirred for 16 hours at RT before concentration under reduced pressure. The crude residue was dissolved in EtOAc (100 mL) and washed with 1M HCl (30ml), 1 M Na₂CO₃ (30ml) and brine (20ml). The organic layer was dried (MgSO₄) and concentrated under reduced pressure. The residue was purified by column chromatography (25% EtOAc/heptane) to afford the product (2.24g, 73% yield). ESMS: m/z 402 [M+H]⁺.

### Stage 4- Cyclopentyl N-(tert-butoxycarbonyl)-L-homoserinate

A solution of cyclopentyl *N*-(*tert*-butoxycarbonyl)-*O*-[*tert*-butyl(dimethyl)silyl]-L-homoserinate (1.51g, 3.90mmol) in acetic acid:THF:water (3:1:1, 100mL) was stirred at 30°C for 16 hours. EtOAc (200mL) was added and washed with 1 M Na₂CO₃(10ml), 1 M HCl (10ml) and brine (10ml). The EtOAc layer was dried (MgSO₄) and concentrated under reduced pressure to afford the product as a clear oil which solidified on standing (1.00g, 95% yield). ESMS: m/z 310 [M+Na]⁺.

### Stage 5 - Cyclopentyl (2S)-4-bromo-2-[(tert-butoxycarbonyl)amino]butanoate

To a suspension of NBS (1.86g, 10.4mmol) in DCM (16mL) was added a solution of triphenyl phosphine (2.56g, 9.70mmol) in DCM (7mL). The solution was stirred for 5 minutes after addition. Pyridine (0.34mL, 4.20mmol) was added followed by a solution of cyclopentyl *N*-(*tert*-butoxycarbonyl)-L-homoserinate (1.00g, 3.5mmol) in DCM (9mL). The solution was stirred at RT for 18 hours, concentrated under reduced pressure and the residual solvent azeotroped with toluene (3 x 16mL). The residue was triturated with Et₂O (10mL) and EtOAc:heptane (1:9, 2 x 10mL). The combined organic solutions were concentrated onto silica and purified by column chromatography (10%-25% EtOAc/heptane) to afford the title intermediate (1.02g, 84% yield). ESMS: m/z 351 [M+H]⁺. ¹H NMR (300 MHz, C*D*Cl₃) 5.30-5.05 (2H, m), 4.45-4.30(1H, m), 3.45 (2H, t, J=7.3 Hz), 2.50-2.30 (1H, m), 2.25- 2.10 (1H, m), 1.95-1.60 (8H, br m) and 1.47 (9H, s).

### Intermediate 3B:

### tert-butyl (2S)-2-{[(benzyloxy)carbonyl]amino}-4-bromobutanoate

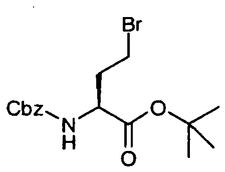

The title intermediate was prepared according to the procedure outlined below (Scheme 3).

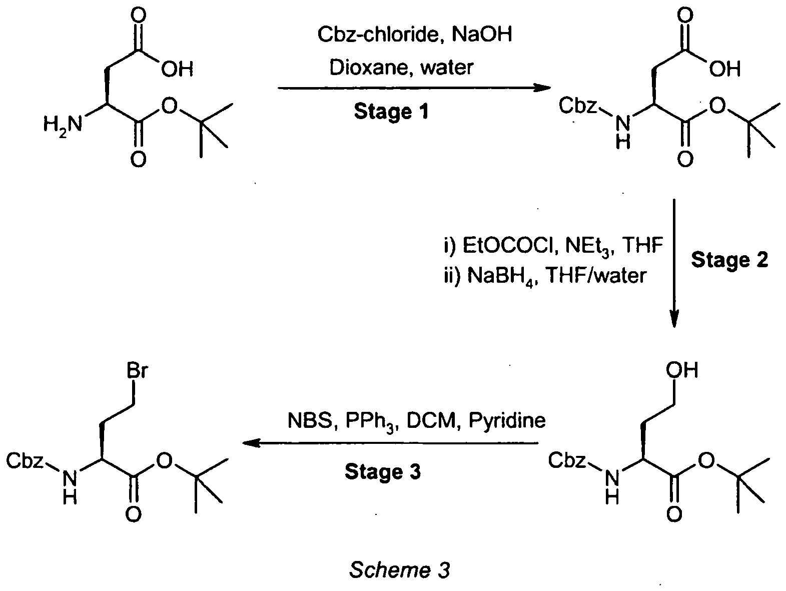

### Stage 1- (3S)-3-{[(Benzyloxy)carbonyl]amino}-4-tert-butoxy-4-oxobutanoic acid

To a solution of (3*S*)-3-amino-4-*tert*-butoxy-4-oxobutanoic acid (900mg, 4.75mmol) and sodium hydroxide (280mg, 7.13mmol) in 25% water/dioxane (50mL) at 0°C was added benzyl chloroformate (2g, 4.13mmol) in dioxane (10mL). The mixture was stirred at 0°C for 1 hour and then at RT overnight. Water (10mL) was added and the mixture was extracted with EtOAc (2 x 20mL). The organic phase was back extracted with a saturated aqueous solution of NaHCO₃ (2 x 10mL). The combined aqueous layers were acidified to pH 1 with 1M HCl, and extracted with EtOAc (3 x 10mL). The combined organic layers were dried (MgSO₄) and concentrated under reduced pressure. The residue was purified by column chromatography (35% EtOAc/heptane) to give the product as a colourless oil (0.76g, 50% yield). ESMS: m/z 346 [M+23]⁺

### Stage 2- tert-Butyl N-[(benzyloxy)carbonyl]-L-homoserinate

To a solution of (3*S*)-3-([(benzyloxy)carbonyl]amino}-4-*tert*-butoxy-4-oxobutanoic acid (600mg, 1.87mmol) in anhydrous THF (20mL) at -20°C was slowly added Et₃N (32µL, 2.24mmol) and ethyl chloroformate (21µL, 2.24mmol). The mixture was stirred at -20°C for 2 hours. The solid formed was filtered off and washed with THF (2 x 10mL). The filtrate was added dropwise to a solution of sodium borohydride (0.2g, 5.61 mmol) at 0°C over 10 minutes and then allowed to warm to RT. The mixture was stirred for an additional 4 hours. The solvent was removed under reduced pressure and the residue was diluted with water (10mL), acidified to pH 5 with 1M HCl and extracted with EtOAc (2 x 20ml). The combined organic fractions were washed with 10% aqueous NaOH (10mL), water (10mL) and brine (10mL). The organic layer was dried (MgSO₄) and concentrated under reduced pressure to give the product as a clear oil (0.3g, 51% yield). ESMS: m/z 332 [M+23]⁺.

### Stage 3- tert-butyl (2S)-2-{[(benzyloxy)carbonyl]aminol-4-bromobutanoate

To a solution of NBS (520mg, 2.91mmol) in DCM (10mL) was slowly added a solution of triphenylphosphine (0.71g, 2.72mmol) in DCM (10mL). The mixture was stirred at RT for 5 minutes before pyridine (94µL, 1.16mmol) and a solution of *tert*-butyl *N-*[(benzyloxy)carbonyl]-L-homoserinate (0.30g, 0.97mmol) in DCM (20mL) were added dropwise. The mixture was stirred at RT for another 18 hours. The solvent was removed under reduced pressure, the residue was azeotroped with toluene (2 x 15mL) and triturated with Et₂O (2 x 25mL) and 10% EtOAc in heptanes. The filtrate from the triturations were combined and concentrated under reduced pressure. The crude product was purified by column chromatography (15% EtOAc/heptanes) to give the title intermediate as a clear oil (0.16g, 44% yield). ESMS: m/z 395 [M+23] ⁺. ¹H NMR (300 MHz, C*D*Cl₃), δ ppm 7.39-7.30 (5H, m), 5.40 (1H, d, J=6.8Hz), 5.12 (2H, s), 4.38 (1H, q, J=7.7Hz), 3.47-3.38 (2H, m), 5.49-2.33 (1H, m), 2.28-2.13 (1H, m) and 1.48 (9H, s).

### Intermediate 3C:

### Cyclopentyl 5-bromo-N-(tert-butoxycarbonyl)-L-norvalinate

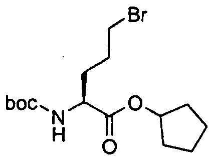

The title intermediate was prepared according to the procedure outlined below (Scheme 4).

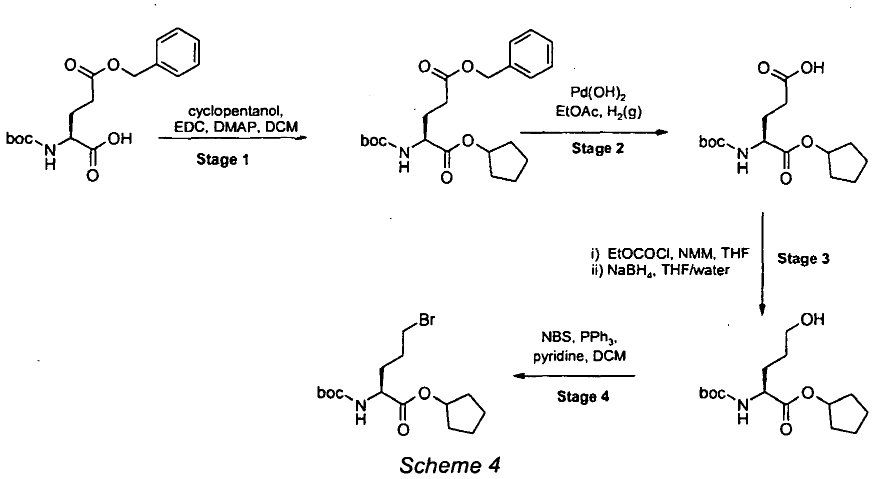

### Stage 1- 5-Benzyl 1-cyclopentyl N-(tert-butoxycarbonyl)-L-glutamate

To a solution of (2*S*)-5-(benzyloxy)-2-[(*tert*-butoxycarbonyl)amino]-5-oxopentanoic acid (15g, 44.5mmol) in DCM (220mL) at 0°C was added cyclopentanol (4.8mL, 53.3mmol), EDC (9.4g, 48.9mmol) and DMAP (543mg, 4.4mmol). The reaction mixture was allowed to warm to RT and stirred for a further 12 hours. The reaction mixture was diluted with DCM (200mL) and washed with 1M HCl (50mL), 1M Na₂CO₃ (30mL) and brine (50mL). The organic layer was dried (MgSO₄) and concentrated under reduced pressure. The residue was purified by column chromatography (25% EtOAc/heptane) to give the product as a white solid (12.4g, 69% yield). ESMS: m/z 406 [M+H]⁺.

### Stage 2- 1-Cyclopentyl N-(tert-butoxycarbonyl)-L-glutamatic acid

5-Benzyl 1-cyclopentyl *N*-(*tert*-butoxycarbonyl)-L-glutamate (12.4g, 30.5mmol) was dissolved in EtOAc (200mL) and purged with nitrogen before addition of Pd(OH)₂ on carbon catalyst (1.3g, 20% w/w). The reaction flask was then purged with hydrogen gas for a period of 5 minutes before leaving under a balloon of hydrogen for 5 hours. The catalyst was removed by filtration through Celite^{®}, washing thouroughly with EtOAc (50mL). The solvent was removed under reduced pressure to give the product as a clear oil (7.73g, 85% yield). ESMS: m/z 316 [M+H]⁺.

### Stage 3- Cyclopentyl N-(tert-butoxycarbonyl)-5-hydroxy-L-norvalinate

To a stirred solution of 1-cyclopentyl *N*-(*tert*-butoxycarbonyl)-L-glutamatic acid (6.73g, 21.4mmol) in THF(150mL) at -20°C was added NMM (3.05mL, 27.8mmol) and ethyl chloroformate (2.45mL, 25.6mmol). The reaction mixture was stirred at -20°C for 2 hours. The solid was removed by filtration was added dropwise over 20 minutes to a solution of sodium borohydride (2.43g, 64.1mmol) in THF (20mL) and water (5mL) at 0°C. The reaction mixture was allowed to warm to RT and left for a further 4 hours. The mixture was acidified to pH 5 with 1M HCl and the THF removed under reduced pressure. The aqueous solution was extracted with EtOAc (3 x 100mL), dried (MgSO₄), and concentrated under reduced pressure. The residue was purified by column chromatography (0-5% MeOH/DCM) to give the product as a clear oil (5.0g, 78% yield). ESMS: m/z 302 [M+H]⁺.

### Stage 4- Cyclopentyl 5-bromo-N-(tert-butoxycarbonyl)-L-norvalinate

To a suspension of NBS (3.54g, 19.9mmol) in DCM (30mL) was added a solution of triphenylphosphine (4.87g, 18.8mmol) in DCM (15mL). The solution was stirred for a further 5 minutes before addition of pyridine (644µL, 7.96mmol) and a solution of cyclopentyl *N*-(*tert*-butoxycarbonyl)-5-hydroxy-L-norvalinate (2.0g, 6.64mmol) in DCM (20mL). The solution was stirred for 18 hours, concentrated under reduced pressure and the residual solvent azeotroped with toluene (3 x 30mL). The residue was triturated with Et₂O (30mL) and 10% EtOAc/heptane (2 x 30mL). The combined Et₂O and EtOAc/heptane solutions were concentrated onto silica and purified by column chromatography (10%-25% EtOAc/heptane) to give the title intermediate as a clear oil (1.34g, 55% yield). ESMS: m/z 365 [M+H]⁺. ¹H NMR (300 MHz, C*D*Cl3), δ: 5.25 (1H, m), 5.05 (1H, bd), 3.45 (2H, m), 2.00-1.55 (12H, bm) and 1.45 (9H, s).

### Intermediate 3D:

### tert-butyl N-[(benzyloxy)carbonyl]-5-bromo-L-norvalinate

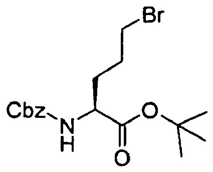

The title intermediate was prepared according to the procedure outlined for intermediate 3B [Scheme 3] starting with (4*S*)-4-amino-5-*tert*-butoxy-5-oxopentanoic acid. ESMS: m/z 409 [M+Na]⁺.

### Intermediate 4A:

### Cyclopentyl (2R)-4-bromo-2-[(tert-butoxycarbonyl)amino]butanoate

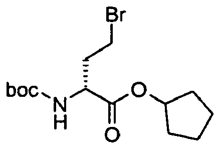

The title intermediate was prepared according to the procedure outlined for intermediate 3A [Scheme 2] starting with D-homoserine. ESMS: m/z 351 [M+H]⁺.

### Intermediate 4B:

### Cyclopentyl 5-bromo-N-(tert-butoxycarbonyl)-D-norvalinate

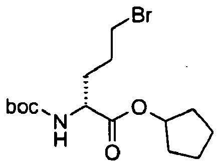

The title intermediate was prepared according to the procedure outlined for intermediate 3C [Scheme 4] starting with of (2*R*)-5-(benzyloxy)-2-[(*tert*-butoxycarbonyl)aminol-5-oxopentanoic acid. ESMS: m/z 365 [M+H]⁺.

### Intermediate 5

### (1R,2S,5R)-2-isopropyl-5-methylcyclohexyl (2S)-2-[(tert-butoxycarbonyl)aminol-4-oxobutanoate

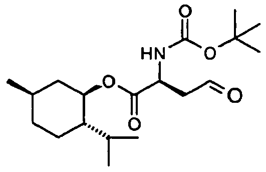

The title intermediate was prepared according to the procedure outlined below (Scheme 5).

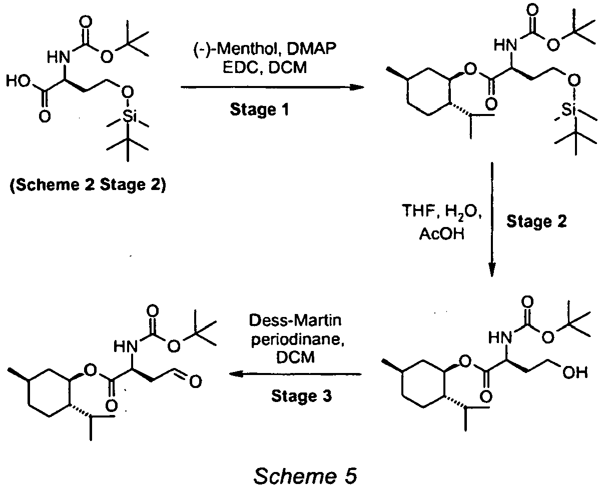

### Stage 1- (1R,2S,5R)-2-isopropyl-5-methylcyclohexyl N-(tert-butoxycarbonyl)-O-[tert-butyl(dimethyl)silyl]-L-homoserinate

To a suspension of *N*-(*tert*-butoxycarbonyl)-*O*-[*tert-butyl*(dimethyl)silyl]-L-homoserine [Scheme 2 Stage 2] (6.22g, 19mmol) in DCM (120mL) at 0°C was added (-)-menthol (5.85g, 37.0mmol), DMAP (228mg, 1.87mmol) and EDC (3.93g, 20.3mmol). The solution was allowed to warm to RT and stirred for a further 18 hours. The reaction mixture was concentrated under reduced pressure. The residue was purified by column chromatography (20% EtOAc/heptane) to give the product as a clear oil (4.86g, 55% yield). ESMS: m/z 394 [M+Na]⁺.

### Stage 2- (1R,2S,5R)-2-isopropyl-5-methylcyclohexyl N-(tert-butoxycarbonyl)-L-homoserinate

A suspension of (1*R*,2*S*,5*R*)-2-isopropyl-5-methylcyclohexyl *N*-(*tert*-butoxycarbonyl)-*O-*[*tert*-butyl(dimethyl)silyl]-L-homoserinate (4.86g,14.0mmol) in THF / water / acetic acid (60mL : 60mL : 180mL) was heated at 30°C for 20 hours. The reaction was diluted with EtOAc (60mL) and washed with sat NaHCO₃ solution (20mL), 1M HCl (30mL) and brine (30mL). The organic layer was dried (MgSO₄) and concentrated under reduced pressure to afford the product (3.45g 69% yield). ESMS: m/z 380 [M+Na]⁺.

### Stage 3- (1R,2S,5R)-2-isopropy)-5-methytcyclohexyl (2S)-2-[(tert butoxycarbonyl) amino]-4-oxobutanoate

To a suspension of (1*R*,2*S*,5*R*)-2-isopropyl-5-methylcyclohexyl *N*-(tert-butoxycarbonyl)-L-homoserinate (500mg, 1.40mmol) in DCM (20mL) at 0°C was added Dess-Martin periodinane (595mg, 1.54mmol). The reaction was allowed to warm to RT and stirred for 3 hours. To the solution was added 1:1 Na₂SO₃ / NaHCO₃ saturated solution (30mL) and the mixture stirred for 15 min. The organic layer was separated and the aqueous layer extracted with DCM (2 x 10mL). The combined organic layers were washed with 1:1 Na₂SO₃ / NaHCO₃ solution (15mL), dried (MgSO₄) and concentrated under reduced pressure to give the title intermediate as a colourless oil (480g 97% yield). ESMS: m/z 378 [M+Na]⁺. ¹H NMR (CDCl₃) δ: 7.90 (1H, m), 5.30 (1H, d J=4.7Hz), 4.70-4.57 (2H, m), 4.45 (1H, br. s), 2.92 (2H, t, J=5.7Hz), 1.91-1.68 (6H, m), 1.58 (9H, s), 1.05-0.85 (4H, m) and 0.66 (6H, d, J=7.0Hz).

### Intermediate 6A

### Cyclopentyl 4-amino-N-(tert-butoxycarbonyl)-L-phenylalaninate

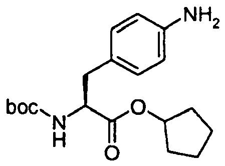

The title intermediate was prepared according to the procedure outlined below (Scheme 6).

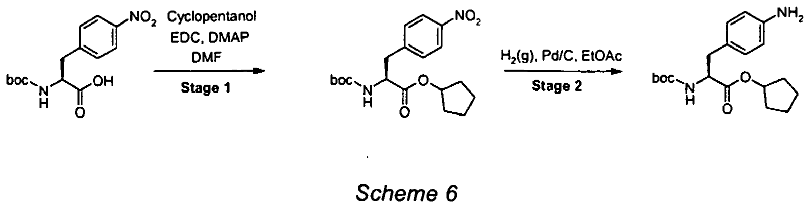

### Stage 1- Cyclopentyl N-(tert-butoxycarbonyl)-4-nitro-L-phenylalaninate

To a solution of *N*-(*tert*-butoxycarbonyl)-4-nitro-L-phenylalanine (1.00g, 3.23mmol) in DMF (10mL) at 0°C was added cyclopentanol (0.585mL, 6.44mmol), DMAP (39mg, 0.32mmol) and EDC (0.655g, 3.39mmol). The reaction mixture was allowed to warm to RT and stirred for a further 16 hours. The mixture was partitioned between water (200mL) and EtOAc (200mL). The organic layer was extracted with water (3x50mL), dried (MgSO₄) and concentrated under reduced pressure. The residue was purified by column chromatography (33% heptane/EtOAc) to afford the product as a pale yellow oil (1.12g, 95% yield). ESMS: m/z 365 [M+H]⁺.

### Stage 2- Cyclopentyl 4-amino-N-(tert-butoxycarbonyl)-L-phenylalaninate

To a solution of cyclopentyl *N*-(*tert*-butoxycarbonyl)-4-nitro-L-phenylalaninate (480mg, 1.32mmol) in EtOAc (10mL) was added 10% Pd /C (48mg, 10% w/w). The flask was evacuated and put under a hydrogen atmosphere for two hours. The reaction was evacuated and the mixture filtered through Celite^{®}, washing with excess EtOAc (20mL). The filtrate was concentrated under reduced pressure to afford the title intermediate as a pink oil (432mg, 98% yield). ESMS: m/z 335 [M+H]⁺. ¹H NMR (300 MHz, CDCl₃) δ: 6.62 (2H, d, J = 8.4Hz), 5.15-5.25 (1H, m, CH), 4.95 (1H, d, J = 4.2Hz), 4.40-4.55 (1H, m), 6.94 (2H, d, J = 8.1Hz), 3.62 (2H, br s), 2.97 (2H, d, J = 5.7Hz), 1.50-1.96 (9H, m) and 1.44 (9H, s).

### Intermediate 6B

### tert-Butyl 4-amino-N-(tert-butoxycarbonyl)-L-phenylalaninate

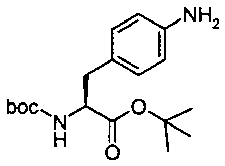

The title intermediate was prepared according to the procedure outlined below (Scheme 7).

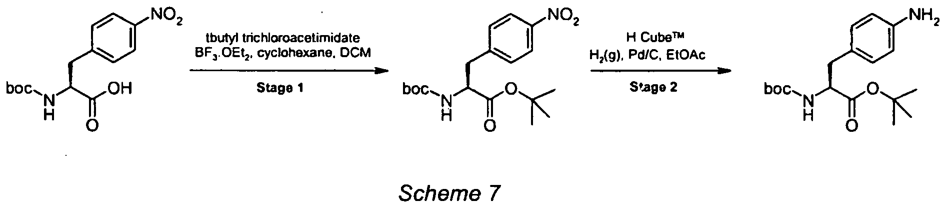

### Stage 1- tert-Butyl N-(tert-butoxycarbonyl)-4-nitro-L-phenylalaninate

To a solution of *N*-(tert-butoxycarbonyl)-4-nitro-L-phenylalanine (500mg, 1.61 mmol) in 66% DCM / cyclohexane (30mL) at 0°C was added boron trifluoride diethyl etherate (10µL) followed immediately by dropwise addition over 10 minutes of *tert*-butyl trichloroacetimidate (704mg, 3.22mmol) in cyclohexane (10mL). The mixture was allowed to warm to RT and stirred for 30 minutes before quenching with NaHCO₃ powder (80mg). The crude mixture was filtered through Celite^{®} and the filtrate concentrated under reduced pressure to give a residue. The residue was purified by column chromatography (20% EtOAc / heptane) to give the product as a yellow solid (320mg, 54% yield). ESMS: m/z 389 [M+Na]⁺.

### Stage 2- tert-Butyl 4-amino-N-(tert-butoxycarbonyl)-L-phenylalaninate

Stage 1 product (0.53g, 1.40mmol) was dissolved in MeOH (29mL) to make a 0.05M solution. The solution was passed through an H-Cube™ continuous hydrogenator (Thales Nanotechnology, HC-2, SS). The reaction was performed using a 30mm CatCart™ (10% Pd/C) in full H₂ mode. A flow rate of 1mL/min was maintained, with a temperature of 25°C and H₂ pressure of 1 bar. The product was eluted into 2M NaOH

(20mL) and the MeOH removed under reduced pressure. The aqueous solution was extracted with EtOAc (2 x 20mL). The combined organic layers were dried (MgSO₄) and concentrated under reduced pressure to afford the title intermediate as a yellow oil. (0.15g, 31% yield). ESMS: m/z 359 [M+Na]⁺. ¹H NMR (300 MHz, MeOD) δ: 6.97 (2H, d, J=8.5 Hz), 6.68 (2H, d, J=8.3 Hz), 4.15 (1H, t, J=5.9 Hz), 2.85 (2H, dd, J=19.0, 7.2 Hz) and 1.42 (18H, s).

### Intermediate 7A

### Cyctopentyl (2S)-4-amino-2-[(tert-butoxycarbonyl)amino]butanoate

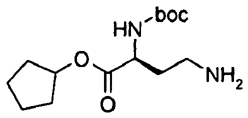

The title intermediate was prepared according to the procedure outlined below (Scheme 8).

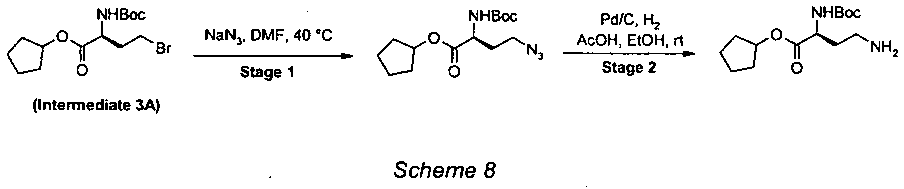

### Stage 1- Cyclopentyl (2S)-4-azido-2-[(tert-butoxycarbonyl)amino]butanoate

To a solution of cyclopentyl (2*S*)-4-bromo-2-[(*tert*-butoxycarbonyl)amino]butanoate [Intermediate 3A] (1.00g, 2.90mmol) in DMF (30mL) was added sodium azide (0.93g, 14.3mmol). The reaction mixture was stirred at 40°C for 32 hours and concentrated under reduced pressure. The residue was partitioned between Et₂O (100mL) and sat. Na₂CO₃ (100mL). The organic layer was separated, washed with sat. Na₂CO₃ (100mL), and brine (100mL), dried (MgSO₄), and concentrated under reduced pressure to give the product as a yellow oil (1.05g). This product was used without further purification. ESMS: m/z 335 [M+Na]⁺

### Stage 2- Cyclopentyl (2S)-4-amino-2-[(tert-butoxycarbonyl)amino]butanoate

To a solution of crude cyclopentyl (2*S*)-4-azido-2-[(*tert*-butoxycarbonyl)amino]butanoate (1.05g, 2.90mmol) in ethanol (50mL) was added acetic acid (0.16mL, 2.90mmol). The reaction mixture was flushed 3 times with nitrogen. Pd/C (50 mg, 10% w/w) was added. The mixture was flushed 3 times with nitrogen and finally stirred under an atmosphere of hydrogen at RT for 2 hours. The reaction mixture was filtered through a short pad of Celite^{®} and the filtrate was concentrated under reduced pressure. The residue was partitioned between EtOAc (50mL) and sat. Na₂CO₃ (50mL). The organic layer was separated, washed with brine (50mL), dried (MgSO₄), and concentrated under reduced pressure to leave a yellow oil. Purification by column chromatography (2% ammonia : 5% MeOH in DCM) afforded the title intermediate as a colorless oil (638mg, 78% yield over 2 steps). ESMS: m/z 287 [M+H]⁺. ¹H NMR (300 MHz, C*D*Cl3) δ: 5.55 (1H, br d), 5.21 (1H, m), 4.35 (1H, m), 2.81 (2H, m), 1.89 (2H, m), 1.81-1.55 (8H, m) and 1.45 (9H, s).

### Intermediate 7B

### Cyclopentyl N²-(tert-butoxycarbonyl)lysinate

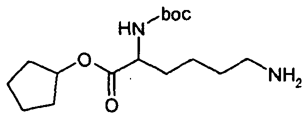

The title intermediate was prepared according to the procedure outlined below (Scheme 9).

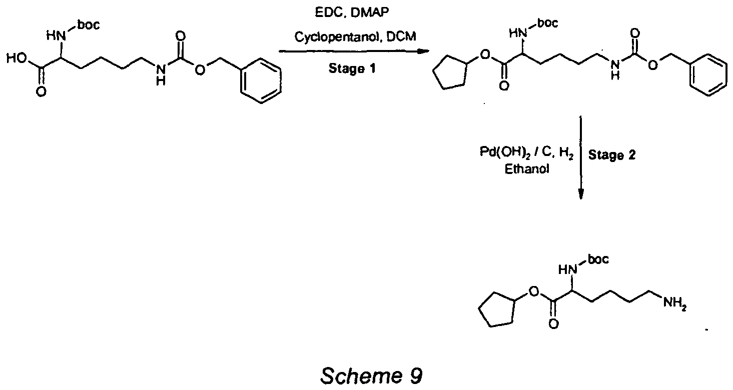

### Stage 1- Cyclopentyl N⁶-[(benzyloxy)carbonyl]-N²-(tert-butoxycarbonyl)lysinate

To a solution of *N*⁶-[(benzyloxy)carbonyl]-*N*²-(*tert*-butoxycarbonyl)lysine (1.00g, 2.63mmol) in anhydrous DCM (20mL) at 0°C was added DMAP (32mg, 0.26mmol), cyclopentanol (0.48mL, 5.23mmol) and EDC (552mg, 2.89mmol). The reaction was allowed to warm to room RT and stirred for a futher 16 hours. The mixture was diluted with DCM (50mL) and washed with brine (50mL). The organic layer was dried (MgSO₄) and concentrated under reduced pressure to give crude product as an oil (1.18g, 100% yield) which was used without further purification. ESMS: m/z 471 [M+Na]⁺.

### Stage 2- Cyclopentyl N²-(tert-butoxycarbonyl)lysinate

To a solution of cyclopentyl *N*⁶-[(benzy[oxy)carbonyl]-N²-(*tert*-butoxycarbonyl)lysinate (1.18g, 2.63mmol) in ethanol (5mL) was carefully added palladium hydroxide on carbon (235mg, 20%w/w) under an atmosphere of nitrogen. The reaction mixture was evacuated and placed under an atmosphere of H₂. This was repeated a further two times and the reaction allowed to stir under and atmosphere of H₂ for 2 hours. The reaction mixture was filtered through Celite^{®} and concentrated to give the title intermediate (250mg). ESMS: m/z 315 [M+H]⁺. ¹H NMR (300 MHz, DMSO) δ: 6.70-6.77 (1H, m), 5.13-5.15 (1H, m), 4.08-4.09 (1H, m), 2.88-2.90 (2H, m), 1.82 (2H, m), 1.57-1.66 (10H, m) and 1.03-1.37 (11H, m).

### Intermediate 8

### 1-tert-Butyl 2-cyclopentyl (2S,4S)-4-aminopyrrolidine-1,2-dicarboxylate

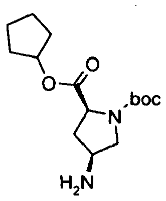

The title intermediate was prepared according to the procedure outlined below (Scheme 10).

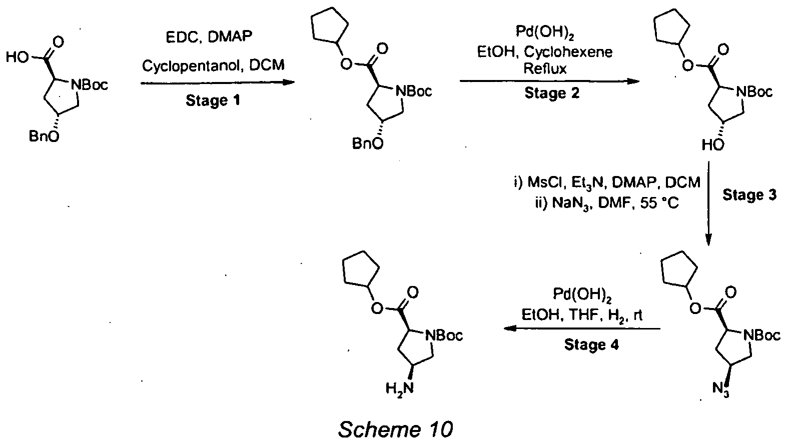

### Stage 1- 1-tert-Butyl 2-cyclopentyl (2S,4R)-4-(benzytoxy)pyrrolidine-1,2-dicarboxylate

To a solution of (4*R*)-4-(benzyloxy)-1-(*tert*-butoxycarbonyl)-L-proline (5.06g, 15.7mmol) in DCM (50mL) at 0°C was added cyclopentanol (2.9mL, 31.4mmol), DMAP (192mg, 1.60mmol) and EDC (3.32g, 17.3mmol). The reaction mixture was allowed to warm to RT and stirred for a further 18 hours. The mixture was washed with sat, Na₂CO₃ (30mL), 1M HCl (30mL) and brine (30mL). The organic layer was dried (MgSO₄) and concentrated under reduced pressure to leave a pale yellow oil. Purification by column chromatography (15% EtOAc/heptane) afforded the product as a colourless oil (5.21g, 85% yield). ESMS: m/z 412 [M+Na]⁺ and 801 [2M+Na]⁺.

### Stage 2- 1-tert-Butyl 2-cyclopentyl (2S,4R)-4-hydroxypyrrolidine-1,2-dicarboxylate

To a solution of *1*-*tert*-butyl 2-cyclopentyl (2*S*,4*R*)-4-(benzyloxy)pyrrolidine-1,2-dicarboxylate (5.21 g, 13.4 mmol) in EtOH:cyclohexene (5:1, 120 mL) was carefully added palladium hydroxide on carbon (521 mg, 20%w/w) The reaction mixture was evacuated and flushed with nitrogen 3 times and refluxed for 21 hours. The reaction mixture was filtered through Celite^{®} and the filtrate was concentrated under reduced pressure to leave a pale yellow oil. Purification by column chromatography (50% EtOAc/heptane) afforded the product as a pale pink oil (3.77g, 100 % yield). ESMS: m/z 621 [2M+Na]⁺.

### Stage 3- 1-tert-Butyl 2-cyclopentyl (2S,4S)-4-(2I⁵-triaz-1-en-2-yn-1-yl)pyrrolidine-1,2-dicarboxylate

To a solution of 1-*tert*-butyl 2-cyclopentyl (2*S*,4*R*)-4-hydroxy-pyrrolidine-1,2-dicarboxylate (3.07g, 10.3mmol) in DCM (100mL) at 0°C was added Et₃N (2.90mL, 20.5mmol), DMAP (125mg, 1.02mmol) and methanesulfonyl chloride (0.87mL, 11.3mmol). The reaction mixture was allowed to warm to RT and stirred for 1 hour. The mixture was washed with water (50mL) and brine (50mL). The organic layer was dried (MgSO₄) and concentrated under reduced pressure. The residue was dissolved in DMF (100mL) and sodium azide (100mg, 15.5mmol) was added. The reaction mixture was stirred at 60°C for 3 days, allowed to cool to RT and partitioned between water (200mL) and EtOAc (200mL). The organic layer was separated, washed with brine (200mL), dried (MgSO₄) and concentrated under reduced pressure to leave a pale yellow oil. Purification by column chromatography (30% EtOAc/heptane) afforded the title compound as a colourless oil (3.26g, 98% yield). ESMS: m/z 671 [2M+Na]⁺

### Stage 4- 1-tert-Butyl 2-cyclopentyl (2S,4S)-4-aminopyrrolidine-1,2-dicarboxylate

To a solution of 1-*tert*-butyl 2-cyclopentyl (2*S*,4*S*)-4-(2I⁵-triaz-1-en-2-yn-1-yl)pyrrolidine-1,2-dicarboxylate (3.26g, 10.0mmol) in EtOH:THF (5:1, 120mL) was added palladium hydroxide on carbon (326mg, 20% w/w). The reaction mixture was evacuated and placed under an atmosphere of H₂. This was repeated a further two times and the reaction allowed to stir under and atmosphere of H₂ for 16 hours The reaction mixture was filtered through Celite^{®} and the filtrate was concentrated under reduced pressure to leave a pale yellow oil. Purification by column chromatography (5-10% MeOH/DCM) afforded the title intermediate as a thick colourless oil (1.34g, 45% yield). ESMS: m/z 299 [M+H]⁺ and 597 [2M+Na]⁺. ¹H NMR (300 MHz, C*D*Cl₃) δ:5.27-5.19 (1H, m), 4.31-4.18 (1H, m), 3.75-3.63 (1H, m), 3.57-3.50 (2H, m), 3.31-3.22 (1H, m), 2.52-2.43 (1H, m) and 1.91-1.38 (15H, m).

### Intermediate 9

### Cyclopentyl 3-[1-(2-aminoethyl)piperidin-4-yl]-N-(tert-butoxycarbonyl)alaninate

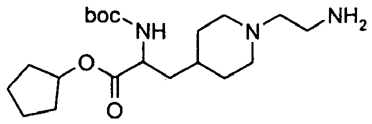

The title intermediate was prepared according to the procedure outlined below (Scheme 11).

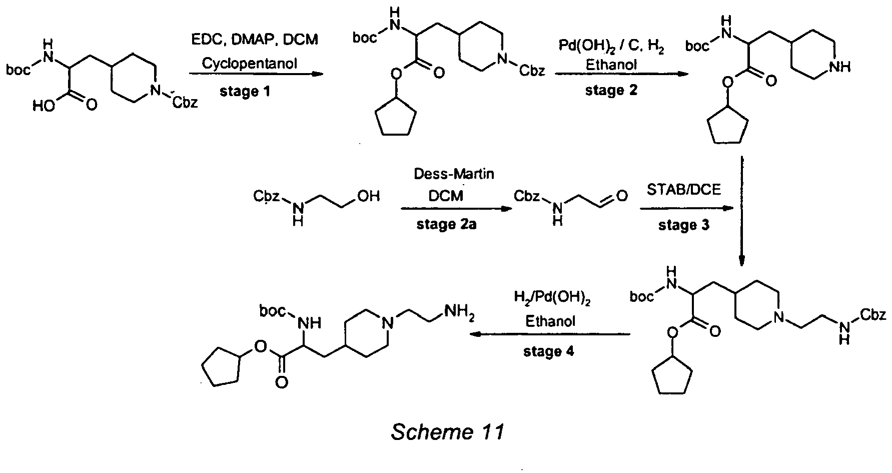

### Stage 1- Benzyl 4-{2-[(tert-butoxycarbonyl)amino]-3-(cyclopentyloxy)-3-oxopropyl} piperidine-1-carboxylate

To a solution of 3-{1-[(benzyloxy)carbonyl]piperidin-4-yl}-*N*-(*tert*-butoxycarbonyl)alanine (250mg, 0.62mmol) in DCM (5mL) at 0°C was added cyclopentanol (0.11mL, 1.23mmol), DMAP (9.6mg, 0.06mmol), and EDC (180mg, 0.68mmol). The reaction was allowed to warm to RT and stirred for a further 16 hours. The reaction mixture was diluted with water (30mL) and EtOAc (30mL). The aqueous layer was re-extracted with EtOAc (2 x 30mL) and the combined organic layers washed with brine, dried (MgSO₄) and concentrated under reduced pressure to give crude product (340mg, >100% yield) which was used without further purification. ESMS: m/z 475 [M+H]⁺.

### Stage 2- Cyclopentyl N-(tert-butoxycarbonyl)-3-piperidin-4-ylalaninate

To a solution of *N*-(*tert*-butoxycarbonyl)-3-piperidin-4-ylalanine (340mg, 0.72mmol) in ethanol (5mL) was carefully added palladium hydroxide on carbon (68mg, 20% w/w) under an atmosphere of nitrogen. The reaction mixture was evacuated and placed under an atmosphere of H₂. This was repeated a further two times and the reaction allowed to stir under and atmosphere of H₂ for 3 hours. The reaction mixture was filtered through Celite^{®} and concentrated under reduced pressure to give the product (250mg, >100% yield). ESMS: m/z 341 [M+H]⁺.

### Stage 2a- Benzyl (2-oxoethyl)carbamate

To a solution of benzyl (2-hydroxyethyl) carbamate (210mg, 1.08mmole) in DCM (3mL) at -78°C was added Dess-Martin periodinane (504mg, 1.19mmole). The reaction was allowed to warm to RT and stirred for a further 2 hours. The reaction was quenched by the addition of a saturated solution of 1:1 Na₂SO₃/NaHCO₃ (20mL) and then extracted with DCM (3 x 30mL). The combined organics were dried (MgSO₄) and concentrated under reduced pressure to give the desired product (150mg, 70% yield) which required no further purification. ¹H NMM (300 MHz, CDCl₃) δ: 9.59 (1H, s), 7.28-7.30 (5H, m), 5.06 (2H, s) and 4.08 (2H, d, J=5.0Hz).

### Stage 3- Cyclopentyl 3-[1-(2-{[(benzyloxy)carbonyl]amino}ethyl)piperidin-4-yl]-N-(tert-butoxycarbonyl)alaninate

To a solution of cyclopentyl *N*-(*tert*-butoxycarbonyl)-3-piperidin-4-ylalaninate (250mg, 0.74mmol) in DCE (5mL) was added benzyl (2-oxoethyl)carbamate (131mg, 0.67mmol). The reaction was allowed to stir for 30 mins and then STAB (424mg, 2.01 mmol) was added. The reaction was stirred for a further 16 hours and then quenched by the addition of sat. NaHCO₃ solution (10ml). The mixture was extracted with DCM (3 x 30mL), the organic layers combined, dried (MgSO₄) and concentrated under reduced pressure to give the product (240mg, 69% yield). ESMS: m/z 518 [M+H]⁺.

### Stage 4- Cyclopentyl 3-[1-(2-aminoethyl)piperidin-4-yl]-N-(tert-butoxycarbonyl)alaninate

To a solution of cyclopentyl 3-[1-(2-{[(benzyloxy)carbonyl]amino}ethyl)piperidin-4-yl]-*N-*(*tert*-butoxycarbonyl)alaninate (240mg, 0.46mmol) in ethanol (5mL) was carefully added palladium hydroxide on carbon (48mg, 20%w/w) under an atmosphere of nitrogen. The reaction mixture was evacuated and placed under an atmosphere of H₂. This was repeated a further two times and the reaction allowed to stir under an atmosphere of H₂ for 3 hours. A further portion of palladium hydroxide on carbon (48mg, 20%w/w) was added and the reaction stirred for an additional 16 hours. The reaction mixture was filtered through Celite^{®} and concentrated under reduced pressure to give the title intermediate (250mg). ESMS: m/z 384 [M+H]⁺.

### Intermediate 10

### Cyclopentyl O-[4-(aminomethyl)phenyl]-N-(tert-butoxycarbonyl)-L-homoserinate

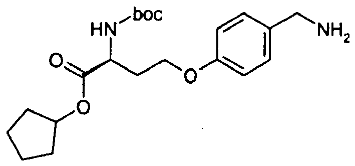

The title intermediate was prepared according to the procedure outlined below (Scheme 12).

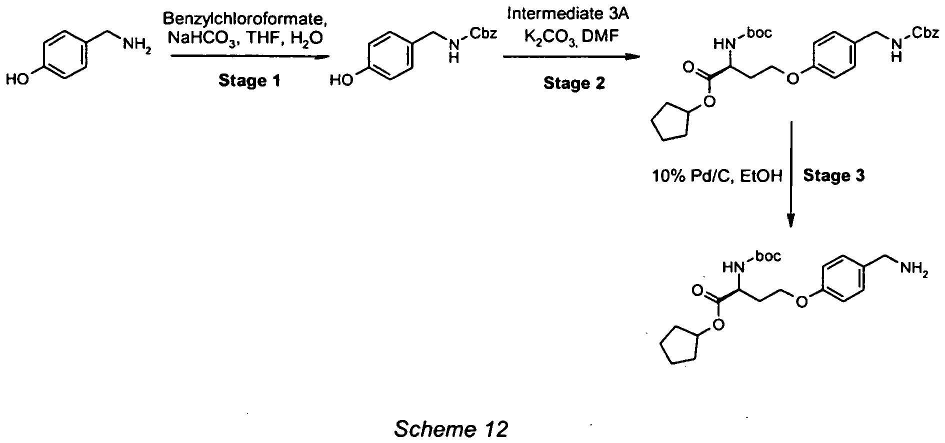

### Stage 1- Benzyl (4-hydroxybenzyl)carbamate

To a suspension of 4-(aminomethyl)phenol (300mg, 2.44mmol) in 10% THF/H₂O (10mL) was added NaHCO₃ (266mg, 3.17mmol). The mixture was cooled to 0°C and benzylchloroformate (344µL, 2.44mmol) added slowly. The reaction was stirred for 1.5 hours at RT. The reaction mixture was partitioned between water (40mL) and EtOAc (40mL). The organic layer was separated and the aqueous layer was re-extracted with EtOAc (20mL). The combined organic layers were dried (MgSO₄) and concentrated under reduced pressure. The residue was triturated with heptane to afford the product as a white solid (610mg, 97% yield). ESMS: m/z 258 [M+H]⁺

### Stage 2- (S)-4-[4-(Benzyloxycarbonylamino-methyl)-phenoxy]-2-tert butoxycarbonyl amino-butyric acid cyclopentyl ester

To a solution of benzyl (4-hydroxybenzyl)carbamate (150mg, 0.58mmol) in DMF (5mL) was added potassium carbonate (107mg, 0.77mmol) and cyclopentyl (2*S*)-4-bromo-2-[(*tert*-butoxycarbonyl)amino] butanoate [intermediate 3A] (219mg, 0.64mmol). The reaction was heated for 20 hours at 60°C. The reaction mixture was concentrated under reduced pressure and then partitioned between water (30mL) and EtOAc (30mL). The aqueous layer was extracted with EtOAc (20mL) and the combined organic layers were dried (MgSO₄) and concentrated under reduced pressure. The residue was purified by column chromatography (10-50% EtOAc/heptane) to afford the product (250mg, 74% yield). ESMS: m/z 527 [M+H]⁺

### Stage 3- Cyclopentyl O-[4-(aminomethyl)phenyl]-N-(tert-butoxycarbonyl)-L-homoserinate

To a solution of (*S*)-4-[4-(Benzyloxycarbonylamino-methyl)-phenoxy]-2-tert-butoxycarbonylamino-butyric acid cyclopentyl ester (250mg, 0.47mmol) in ethanol (8mL) was added a slurry of Pd/C (50mg, 20% w/w) in EtOH (2mL). The reaction was evacuated and put under a H₂ atmosphere for 2 hours. The reaction mixture was filtered through Celite^{®} and washed with ethanol (15mL). The filtrate was concentrated under reduced pressure to afford the title intermediate (110mg, 59% yield). ESMS: m/z 393 [M+H]⁺. ¹H NMR (300 MHz, C*D*Cl₃) δ: 7.21 (2H, d, 8.1Hz), 6.84 (2H, d, J=8.4Hz), 5.38 (1H, m), 5.22 (1H, m), 4.42 (1H, d, J=6.3Hz), 4.03 (2H, t, 6Hz), 3.80 (2H, s), 3.72 (1H, m), 2.29-1.51 (9H, m) 1.45 (9H, s), 1.28-1.20 (2H, m).

### Intermediate 11

### tert-Butyl O-[4-(aminomethyl)phenyl]-N-[(benzyloxy)carbonyl]-L-homoserinate

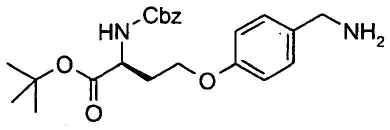

The title intermediate was prepared according to the procedure outlined below (Scheme 13).

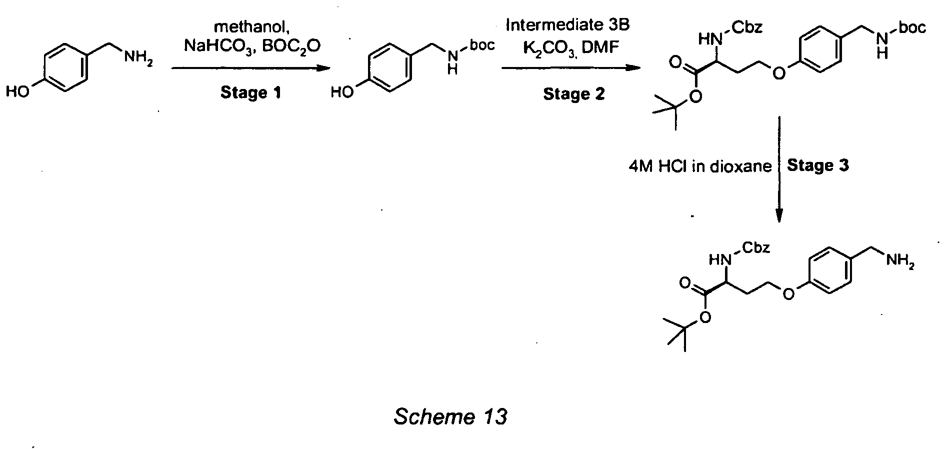

### Stage 1- tert-Butyl (4-hydroxybenzyl)carbamate

To a solution of 4-(aminomethyl)phenol (200mg, 1.62mmol) in MeOH (2.5mL) was added sodium bicarbonate (476mg, 5.68mmol) and BOC₂O (390mg, 1.79mmol). The solution was stirred at RT for 72 hours. The reaction mixture was partitioned between water (20mL) and EtOAc (20mL). The organic layer was separated and the aqueous layer was extracted with EtOAc (10mL). The combined organic layers were dried (MgSO₄) and concentrated under reduced pressure to give the product as a yellow oil (360mg). ESMS: m/z 224 [M+H]⁺.

### Stage 2- tert-Butyl N-[(benzyloxy)carbonyl]-O-(4-{[(tert-butoxycarbonyl)amino]methyl} phenyl)-L-homoserinate

Procedure as in Stage 2 Scheme 12 using intermediate 3B.
ESMS: m/z 515 [M+H]⁺.

**Stage 3**- *tert* Butyl *O*-[4-(aminomethyl)phenyl]-*N*-[(benzyloxy)carbonyl]-L-homoserinate *tert*-butyl *N*-[(benzyloxy)carbonyl]-*O*-(4-{[(*tert*-butoxycarbonyl)amino]methyl} phenyl)-L-homoserinate (200mg, 0.39mmol) was dissolved in 4M HCl/dioxane (1.5mL) and stirred at 0°C for 20 minutes. The reaction mixture was filtered through Celite^{®} and washed with ethanol (15mL). The residue was diluted with EtOAc (15mL) and the pH adjusted to 12 with 1M NaOH solution. The aqueous layer was extracted with EtOAc (3 x 10mL) and the combined organics were dried (MgSO₄) and concentrated under reduced pressure to afford the title intermediate as a colourless oil (152mg, 95% yield). ESMS: m/z 224 [M+H]⁺. ¹H NMR (300 MHz, C*D*Cl₃) δ: 7.36 (5H, s), 7.19 (2H, d, J=8.5Hz), 6.83 (2H, d, J=8.3Hz), 5.12 (2H, s), 4.45 (1H, br. s.), 4.25 (2H, d, J=5.3Hz), 4.04 (2H, t, J=6.0Hz), 2.11-2.46 (2H, m) and 1.48 (9H, s).

### Intermediate 12A

### 1-Benzyl 2-cyclopentyl Piperazine-1,2-dicarboxylate

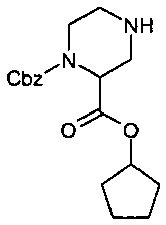

The title intermediate was prepared according to the procedure outlined below (Scheme 14).

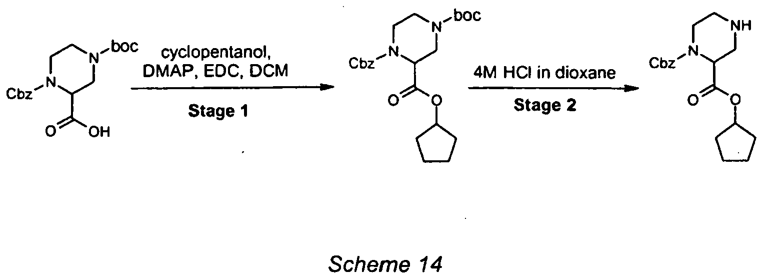

### Stage 1- 1-Benzyl 4-tert-butyl 2-cyclopentyl piperazine-1,2,4-tricarboxylate

To a solution of 1-[(benzyloxy)carbonyl]-4-(*tert*-butoxycarbonyl)piperazine-2-carboxylic acid (1.00g, 2.85mmol) in DCM (20mL) at 0°C was added cyclopentanol (520µL, 5.70mmol), EDC (602mg, 3.14mmol) and DMAP (35mg, 0.29mmol). The reaction mixture was stirred for 48 hours at RT then the solvent removed under reduced pressure. The crude residue was dissolved in EtOAc (30mL) and washed with 1M HCl (15mL), 1M Na₂CO₃ (15mL) and brine (10mL). The organic layer was dried (MgSO₄) and the solvent removed under reduced pressure to give the product (1.23g, 95% yield). ESMS: m/z 433 [M+H]⁺.

### Stage 2- 1-Benzyl 2-cyclopentyl piperazine-1,2-dicarboxylate

1-Benzyl 2-cyclopentyl piperazine-1,2-dicarboxylate (200mg, 0.39mmol) was dissolved in 4M HCl/dioxane (3mL) and stirred at 0°C for 1 hour. The reaction mixture was concentrated under reduced pressure to afford the title intermediate as a colourless oil (145mg). ESMS: m/z 333 [M+H]⁺. ¹H NMR (300 MHz, C*D*Cl₃) δ: 7.20-7.28 (5H, m), 5.16-5.17 (1H, m), 5.01-5.09 (2H, m), 4.49-4.60 (1H, m), 3.82 (1H, t J=14.8Hz), 3.43 (1H, t J=12.9Hz), 2.26-3.12 (4H, m) and 1.51-1.76 (8H, m).

### Intermediate 12B

### 1-Benzyl 2-cyclopentyl 4-(2-aminoethyl)piperazine-1,2-dicarboxylate

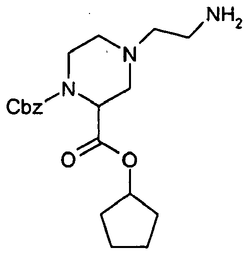

The title intermediate was prepared according to the procedure outlined below (Scheme 15).

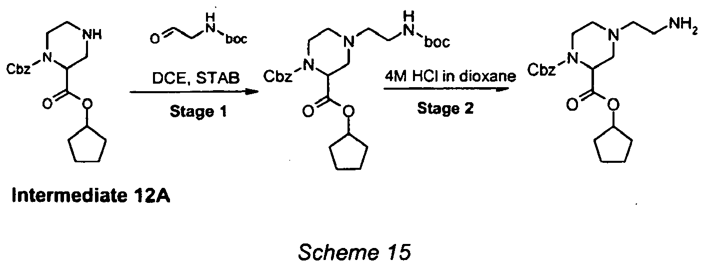

### Stage 1- 1-tert-Butyl 2-cyclopentyl 4-{2-[(tert-butoxycarbonyl)amino]ethyl}piperazine-1,2-dicarboxylate

To a solution of 1-benzyl 2-cyclopentyl piperazine-1,2-dicarboxylate [Intermediate 12A] (165mg, 0.50mmol) in DCE (8 mL) was added the *tert*-butyl (2-oxoethyl)carbamate (72mg, 0.45mmol). After stirring at RT for 10 minutes AcOH (35µL) and STAB (287mg, 1.35mmol) were added. After stirring for 1 hour the mixture was quenched with sat NaHCO₃ (2mL) and diluted with DCM (10mL). The organic layer was washed with 1 M HCl (10mL), 1M Na₂CO₃ (10mL) and brine (10mL), dried (MgSO₄) and evaporated under reduced pressure to isolate the crude product (240mg). ESMS: m/z 476 [M+H]⁺.

### Stage 2- 1-Benzyl 2-cyclopentyl 4-(2-aminoethyl)piperazine-1,2-dicarboxylate Procedure as in [Scheme 14 Stage 2].

ESMS: m/z 376 [M+H]⁺. ¹H NMR (300 MHz, C*D*Cl₃) δ: 7.17-7.31 (5H, m), 4.98-5.20 (3H, m), 4.44-4.91 (2H, m), 3.83 (1H, t, J=14.8 Hz), 3.04-3.52 (4H, m), 1.91-2.46 (4H, m) and 1.44-1.85 (8H, m).

### Intermediate 12C

### 1-Benzyl 2-tert-butyl 4-(2-aminoethyl)piperazine-1,2-dicarboxylate

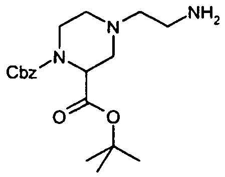

The title intermediate was prepared according to the procedure outlined below (Scheme 16).

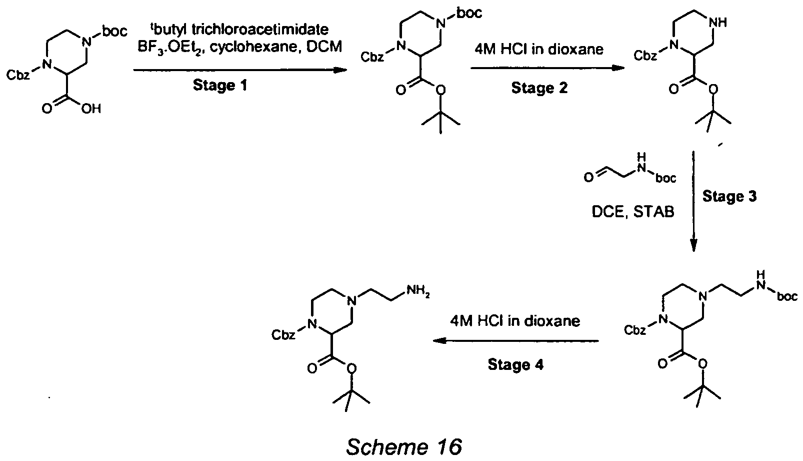

### Stage 1- 1-Benzyl 2,4-di-tert-butyl piperazine-1,2,4-tricarboxylate

To a solution of 1-[(benzyloxy)carbonyl]-4-(*tert*-butoxycarbonyl)piperazine-2-carboxylic acid (500mg, 1.37mmol) in DCM (10mL) and cyclohexane (10mL) at 0°C was added boron trifluoride triethyl etherate followed immediately by slow addition of ^{t}butyl trichloroacetimidate (600mg, 2.74mmol) in cyclohexane (10mL) over 15min. The reaction was allowed to warm to RT and stirred for 30min. Sodium hydrogen carbonate (80mg) was added, and stirring continued for a further 10 minutes before filtering through Celite^{®}. The Celite^{®} was washed thoroughly with DCM and the filtrate solvent removed under reduced pressure. The residue was purified by column chromatography (10% EtOAc/heptane) to afford the product as a white solid (0.240g, 42% yield). ESMS: m/z 443 [M+H]⁺.

### Stage 2- 1-Benzyl 2-tert-butyl piperazine-1,2-dicarboxylate

1-Benzyl 2,4-di-*tert*-butyl piperazine-1,2,4-tricarboxylate (240mg, 0.57mmol) was dissolved in 4M HCl/dioxane (1.5mL) and stirred at RT for 1 hour. The mixture was diluted in EtOAc (10mL) and washed in 2M NaOH. The organic layer was then dried (MgSO₄) and evaporated under reduced pressure to give the crude product (240mg). ESMS: m/z 321 [M+H]⁺.

### Stage 3- 1-Benzyl 2-tert-butyl 4-{2-[(tert-butoxycarbonyl)amino]ethyl}piperazine-1,2-dicarboxylate

Procedure as in [Scheme 15 Stage 1]
ESMS: m/z 464 [M+H]⁺.

### Stage 4- 1-Benzyl 2-tert-butyl 4-(2-aminoethyl)piperazine-1,2-dicarboxylate

Procedure as in [Scheme 13 Stage 3]
ESMS: m/z 364 [M+H]⁺. ¹H NMR (300 MHz, C*D*Cl₃) δ: 7.17-7.31 (5H, m), 4.98-5.20 (3H, m), 4.44-4.91 (2H, m), 3.83 (1H, t, J=14.8 Hz), 3.04-3.52 (4H, m), 1.91-2.46 (4H, m) and 1.35 (9H, s).

### Intermediate 13

### Cyclopentyl (2S)-2-[(tert-butoxycarbonyl)amino]pent-4-enoate

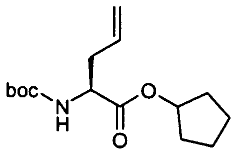

The title intermediate was prepared according to the procedure outlined below (Scheme 17).

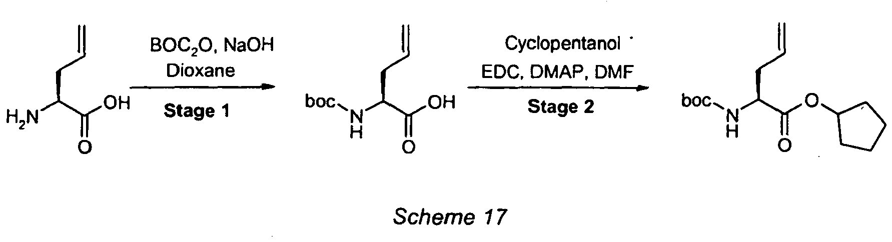

### Stage 1- (2S)-2-[(tert-Butoxycarbonyl)amino]pent-4-enoic acid

To a solution of (2*S*)-2-aminopent-4-enoic acid (1.00g, 8.70mmol) in 1 M NaOH (20mL) and dioxane (10mL) at 0°C was added BOC₂O (2.28g, 10.5mmol). The reaction mixture was allowed to warm to RT and stirred for an additional 18 hours. The pH was checked and adjusted to basic when necessary. The reaction mixture was concentrated under reduced pressure and the aqueous phase washed with Et₂O (2 x 10mL) to remove the excess BOC₂O: The aqueous phase was acidified to pH2 with 2M H₂SO₄ and extracted with EtOAc (4 x 20mL) while saturating the aqueous each time with sodium chloride. The combined organic layers were dried (MgSO₄) and concentrated under reduced pressure to afford the product (2.2g, 100% yield). ESMS m/z: 238 [M+Na]⁺.

### Stage 2- Cyclopentyl (2S)-2-[(tert-butoxycarbonyl)amino)pent-4-enoate

To a solution of (*S*)-2-*tert*-butoxycarbonylamino-pent-4-enoic acid (2.20g, 10.2mmol) in DCM (50mL) was added DMAP (125mg, 1.02mmol), cyclopentanol (1.1mL, 12.2mmol) and EDC (2.15g, 11.2mmol). The reaction was stirred for 65 hours and concentrated under reduced pressure. Purification by column chromatography (5% EtOAc/heptane) afforded the titled intermediate as a clear oil (1.75g, 60% yield). ¹H NMR (300 MHz, C*D*Cl₃) δ: 5.61-5.79 (1H, m), 5.21 (1H, dd, *J*=8.3, 3.4 Hz), 5.15 (1H, dd, *J*=2.9, 1.2 Hz), 5.10 (1H, d, *J*=1.3 Hz), 4.25-4.38 (1H, m), 2.49 (1H, dd, *J*=12.8, 6.4 Hz), 1.53-1.92 (8H, m) and 1.44 (9H, s).

### Example 1

### Cyclopentyl 4-{[(7R)-8-cyclopentyl-7-ethyl-5-methyl-6-oxo-5,6,7,8-tetrahydropteridin-2-yl]amino}-L-phenylalaninate

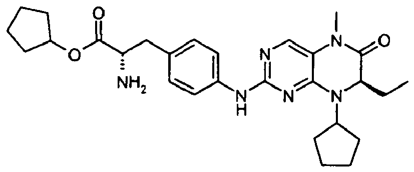

The titled example was prepared according to the procedure outlined below (Scheme 18).

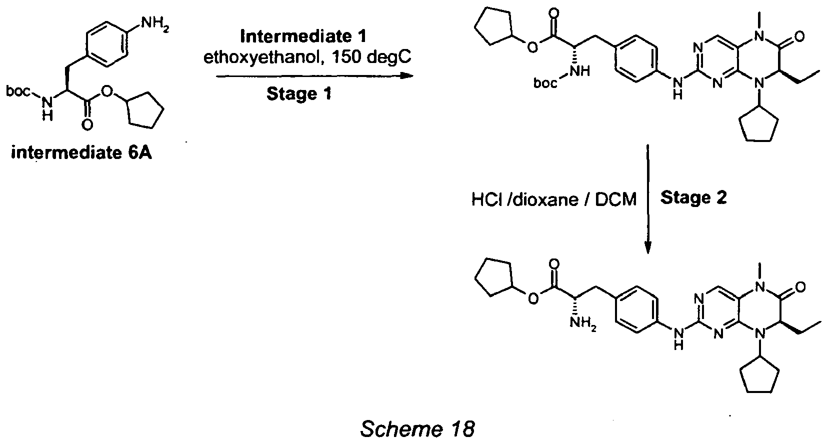

### Stage 1- Cyclopentyl N-(tert-butoxycarbonyl)-4-{[(7R)-8-cyclopentyl-7-ethyl-5-methyl-6-oxo-5,6,7,8-tetrahydropteridin-2-yl]amino}-L-phenylalaninate

To a solution of (7*R*)-2-chloro-8-cydopentyl-7-ethyl-5-methyl-7,8-dihydropteridin-6(5*H*)-one [Intermediate 1] (100mg, 0.34mmol) in 2-ethoxyethanol (2mL) was added cyclopentyl 4-amino-*N*-(*tert*-butoxycarbonyl)-L-phenylalaninate [Intermediate 6A] (170mg, 0.51mmol). The reaction mixture was heated at 150°C for 4 hours, cooled and concentrated under reduced pressure to give a brown residue. The residue was purified by column chromatography (5% methanol/ 1% NH₄OH in EtOAc) to afford the product as a yellow solid (89mg, 43% yield). ESMS: m/z 607 [M+H]".

### Stage 2- Cyclopentyl 4-{[(7R)-8-cyclopentyl-7-ethyl-5-methyl-6-oxo-5,6,7,8-tetrahydropteridin-2-yl]amino}-L-phenylalaninate

To a solution of (S)-2-tert-butoxycarbonylamino-3-[4-((R)-8-cyclopentyl-7-ethyl-5-methyl-6-oxo-5,6,7,8-tetrahydropteridin-2-yl]amino}-L-phenylalaninate (32mg, 0.05mmol) in DCM (3mL) was added 4M HCl/dioxane (3mL). The reaction mixture was stirred at RT for 4 hours and concentrated under reduced pressure to give a brown residue. The pH of the residue was adjusted to 9 with saturated NaHCO₃ solution (3mL) and then extracted with EtOAc (3 x 10mL). The combined organics were dried (MgSO₄) and concentrated under reduced pressure to afford the title example as a white solid (9mg, 34% yield). ESMS: m/z 507 [M+H]⁺. ¹H NMR (300 MHz, C*D*Cl₃) δ: 7.43 (2H, d, J = 7.7Hz), 7.04-7.33 (3H, m), 5.06-5.24 (1H, m), 4.02-4.18 (1H, m), 4.20-4.44 (2H, m), 3.14 (2H, m), 2.83-11.04 (1H, s), 1.32-2.14 (18H, m) and 0.80 (3H, t, J = 7.4Hz).

### Example 2

### Cyclopentyl (2S,4E)-2-amino-5-(4-{[(7R)-8-cyclopentyl-7-ethyl-5-methyl-6-oxo-5,6,7,8-tetrahydropteridin-2-yl]amino}phenyl)pent-4-enoate

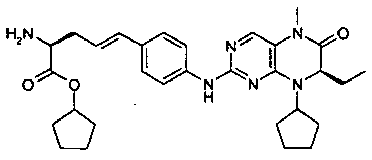

The title example was prepared according to the procedure outlined below (Scheme 19):

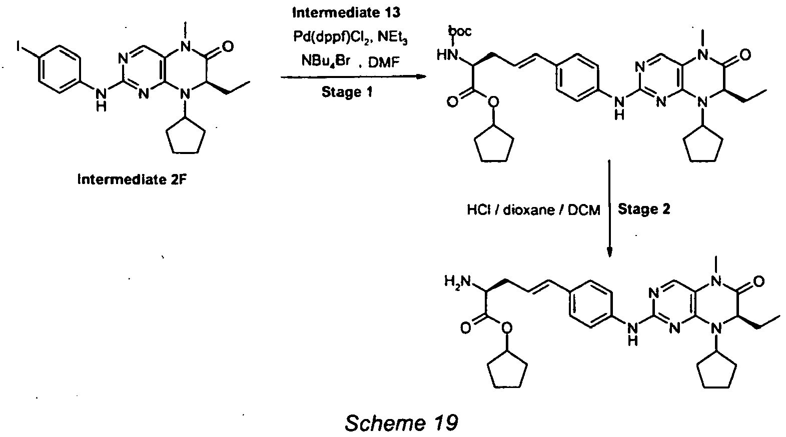

### Stage 1- (7R)-8-Cyclopentyl-7-ethyl-2-[(4-iodophenyl)amino]-5-methyl-7,8-dihydropteridin-6(5H)-one

To a solution of cyclopentyl (2*S*)-2-[(*tert*-butoxycarbonyl)amino]pent-4-enoate [Intermediate 13] (175mg, 0.62mmol) in DMF (3mL) was added (7*R*)-8-cyclopentyl-7-ethyl-2-[(4-iodo-phenyl)amino]-5-methyl-7,8-dihydro pteridin-6(5*H*)-one [Intermediate 2F] (197mg, 0.41mmol), Pd(dppf)Cl₂ (34mg, 0.04mmol), Et₃N (0.13mL, 0.90mmol) and NBu₄Br (133mg, 0.40mmol). The reaction mixture was heated at 120°C for 1h in the microwave and concentrated under reduced pressure. The crude residue was absorbed onto silica and purified by column chromatography (40% EtOAc/heptane) to give the product (72mg, 30% yield). ESMS m/z: 633 [M+H]⁺.

### Stage 2- Cyclopentyl (2S,4E)-2-[(tert-butoxycarbonyl)amino]-5-(4-{[(7R)-8-cyclopentyl-7-ethyl-5-methyl-6-oxo-5,6,7,8-tetrahydropteridin-2-yl]amino}phenyl)pent-4-enoate

To a solution of (7*R*)-8-Cyclopentyl-7-ethyl-2-[(4-iodophenyl)amino]-5-methyl-7,8-dihydropteridin-6(5*H*)-one (36mg, 0.06mmol) in DCM (2mL) was added 4M HCl/dioxane (20gi, 0.08mmol). The reaction mixture was stirred at RT for 2h and then concentrated under reduced pressure. The residue was redissolved in DCM (10ml) washed with 1 M NaHCO₃ (10mL), dried (MgSO₄) and evaporated under reduced pressure. Purification by reverse phase chromatography afforded the title example as a yellow oil (3mg, 10% yield). ESMS m/z: 533 [M+H]⁺. ¹H NMR (300 MHz, MeOD) δ: 7.55-7.62 (1H, m), 7.39-7.51 (4H, m), 6.61 (1H, d, J=15.6 Hz), 6.17 (1H, ddd, J=15.4_{,} 7.6, 7.3 Hz), 5.26-5.34 (1H, m), 4.39 (1H, dd, J=6.3, 3.3 Hz), 4.32 (1H, t, J=8.8 Hz), 4.17 (1H, t, J=6.2 Hz), 3.25 (3H, s), 2.83 (2H, t, J=6.7 Hz), 1.79-2.06 (9H, m), 1.54-1.78 (9H, m) and 0.86 (3H, t, J=7.4 Hz)

### Example 3

### Cyclopentyl O-(4-{[(7R)-8-cyclopentyl-7-ethyl-5-methyl-6-oxo-5,6,7,8-tetrahydropteridin-2-yl]amino}phenyl)-L-homoserinate

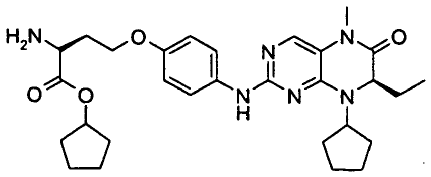

The titled example was prepared according to the general procedure outlined below (Scheme 20).

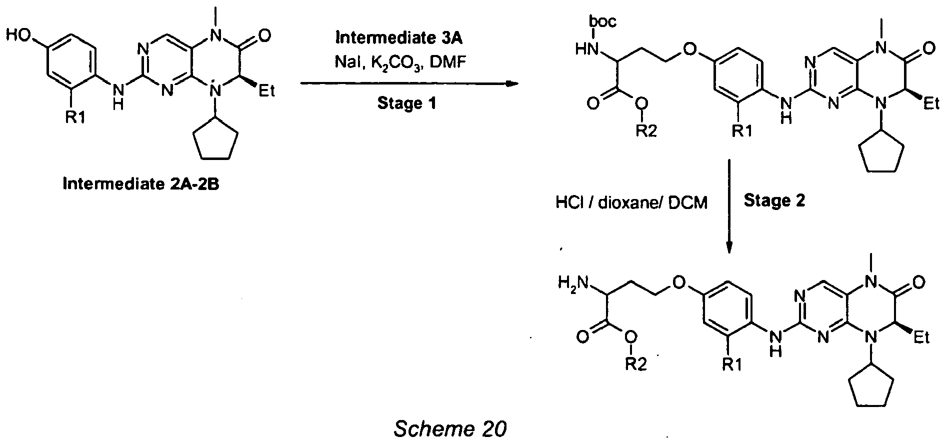

### Stage 1- Cyclopentyl N-(tert-butoxycarbonyl)-O-(4-{[(7R)-8-cyclopentyl-7-ethyl-5-methyl-6-oxo-5,6,7,8-tetrahydropteridin-2-yl]amino}phenyl)-L-homoserinate

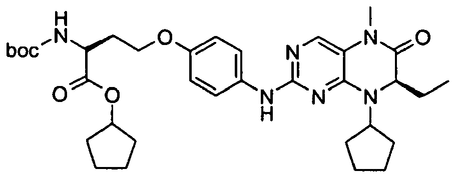

To a solution of (7*R*)-8-cyclopentyl-7-ethyl-2-[(4-hydroxyphenyl)amino]-5-methyl-7,8-dihydropteridin-6(5h)-one [Intermediate 2A] (120mg, 0.33mmol) in DMF (2mL) was added cyclopentyl (2*S*)-4-bromo-2-[(*tert*-butoxycarbonyl)amino]butanoate [Intermediate 3A] (114mg, 0.33mmol) and K₂CO₃ (90mg, 0.65mmol). The reaction mixture was stirred for 40 hours at 40°C and then the reaction mixture was diluted with EtOAc (25mL). The mixture was washed with water (2 x 25mL) and brine (25mL). The organic layer was dried (MgSO₄) and concentrated under reduced pressure to leave a brown oil. Purification by column chromatography (100% EtOAc) afforded the product as a pale brown solid (177 mg, 85% yield). ESMS: m/z 637 [M+H]⁺.

### Stage 2- Cyclopentyl O-(4-{[(7R)-8-cyclopentyl-7-ethyl-5-methyl-6-oxo-5,6,7,8-tetrahydropteridin-2-yl]amino}phenyl)-L-homoserinate

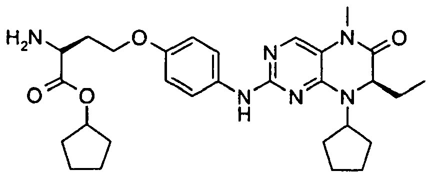

Cyclopentyl *N*-(*tert*-butoxycarbonyl)-*O*-(4-{[(7*R*)-8-cyclopentyl-7-ethyl-5-methyl-6-oxo-5,6,7,8-tetrahydropteridin-2-yl]amino}phenyl)-L-homoserinate (177mg, 0.28mmol) was suspended in a solution of 4M HCl / dioxane (2mL). The reaction mixture was stirred at RT for 30 minutes and concentrated under reduced pressure to leave a thick yellow oil. Trituration with Et₂O afforded an off-white solid, which was partitioned between DCM (25mL) and sat. Na₂CO₃ (25mL). The organic layer was separated, dried (MgSO₄) and concentrated under reduced pressure to afford the title example as an off-white solid (90 mg, 60% yield). ESMS: m/z 537 [M+H]⁺. ¹H NMR (300 MHz, MeO*D*) δ: 7.55 (1H, s), 7.32 (2H, d, J=9.0 Hz), 6.76 (2H, d, J=9.0 Hz), 5.15-5.09 (1H, m), 4.28-4.19 (1H, m), 4.11 (1H, dd, J=3.6, 7.5 Hz), 4.01-3.95 (2H, m), 3.55 (1H, t, J=6.5 Hz), 3.20 (3H, s), 2.11-1.51 (20H, m) and 0.75 (3H, t, J=7.5 Hz).

The example in the following table was prepared by methods analogous to the method described above (Scheme 20) using the appropriate intermediates.

| **Example** | **Intermediates Used** | **Name** | **ESMS** |
|---|---|---|---|
| **4** | 2B & 3A | Cyclopentyl *O*-(4-{[(7*R*)-8-cyclopentyl-7-ethyl-5-methyl-6-oxo-5,6,7,8-tetrahydro pteridin-2-yl]amino}-3-methoxyphenyl)-L-homoserinate | m/z 567 [M+H]⁺ |

### Example 5

### Cyclopentyl (2S)-2-amino-4-[(4-{[(7R)-8-cyclopentyl-7-ethyl-5-methyl-6-oxo-5,6,7,8-tetrahydropteridin-2-yl]amino}-3-methoxybenzoyl)amino]butanoate

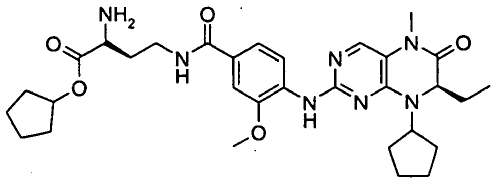

The titled example was prepared according to the general procedure outlined below (Scheme 21).

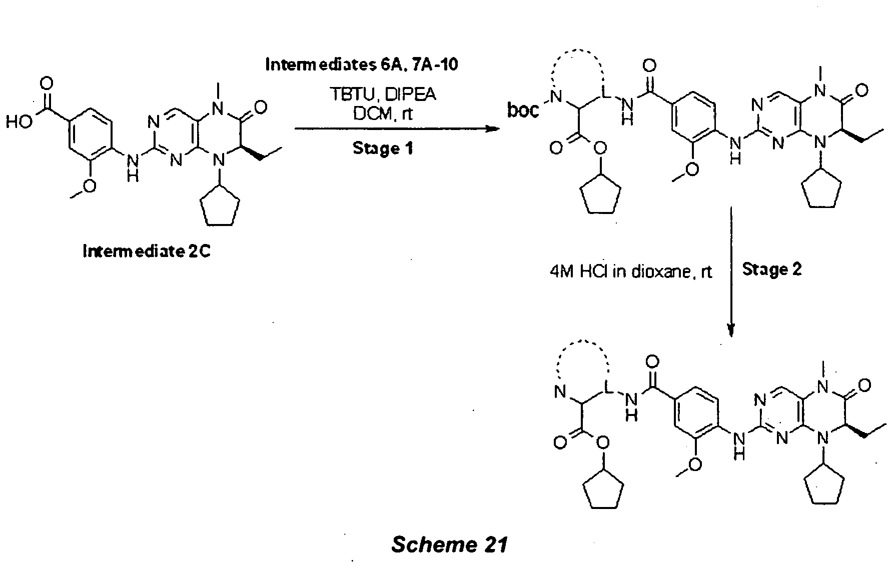

### Stage 1- Cyclopentyl (2S)-2-[(tert-butoxycarbonyl)amino]-4-[(4-{[(7R)-8-cyclopentyl-7-ethyl-5-methyl-6-oxo-5,6,7,8-tetrahydropteridin-2-yl]amino}-3-methoxybenzoyl)amino] butanoate

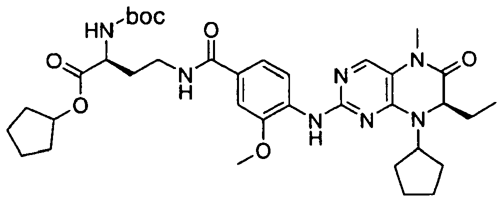

To a solution of 4-{[(7*R*)-8-cyclopentyl-7-ethyl-5-methyl-6-oxo-5,6,7,8-tetrahydro-pteridin-2-yl] amino}-3-methoxybenzoic acid [Intermediate 2C] (200mg, 0.47mmol) in DCM (5mL) was added *O*-benzotriazol-1-yl-*N*,*N*,*N*',*N*'-tetramethyluronium tetrafluoroborate (166mg, 0.52mmol) and DIPEA (0.16mL, 0.94mmol). The reaction mixture was stirred at RT for 30 minutes before adding cyclopentyl (2*S*)-4-amino-2-[(*tert-*butoxycarbonyl)amino]butanoate [Intermediate 7A] (269mg, 0.84mmol). The reaction mixture was stirred at RT for a further 18 hours then diluted with DCM (20mL), and washed with water (2 x 20mL). The organic layer was dried (MgSO₄) and concentrated under reduced pressure to leave a yellow oil. Purification by column chromatography (100 % EtOAc) afforded the product as a yellow solid (228mg, 70% yield). ESMS: m/z 694 [M+H]⁺.

### Stage 2- Cyclopentyl (2S)-2-amino-4-[(4-{[(7R)-8-cyclopentyl-7-ethyl-5-methyl-6-oxo-5,6,7,8-tetrahydropteridin-2-yl]amino}-3-methoxybenzoyl)amino]butanoate

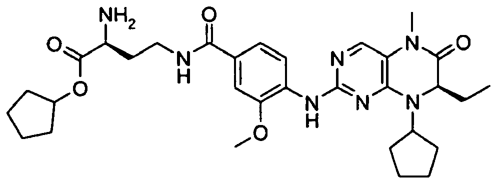

Cyclopentyl (2*S*)-2-[(*tert*-butoxycarbonyl)amino]-4-[(4-{[(7*R*)-8-cyclopentyl-7-ethyl-5-methyl-6-oxo-5,6,7,8-tetrahydropteridin-2-yl]amino}-3-methoxy benzoyl)amino]butanoate (228mg, 0.33mmol) was dissolved in DCM (20mL) and 4M HCl/dioxane (10mL) was added. The reaction mixture was stirred at RT for 2 hours and concentrated under reduced pressure. The residue was taken up in EtOAc (50mL), washed with sat. Na₂CO₃ (25mL), brine (25mL). The organic layer was dried (MgSO₄) and concentrated under reduced pressure to afford the title example as a white solid (180mg, 92% yield). ESMS: m/z 594 [M+H]⁺. ¹H NMR (300 MHz, C*D*Cl₃) δ: 8.55 (1H, d, J=8.4 Hz), 7.70 (2H, br s), 7.62 (1H, s), 7.48 (1H, d, J=1.5 Hz), 7.32 (1H, dd, J=2.0, 8.6 Hz), 5.23-5.19 (1H, m), 4.55-4.49 (1H, m), 4.24 (1H, dd, J=3.6, 7.8 Hz), 3.99 (3H, s), 3.92-3.80 (1H, m), 3.59-3.47 (2H, m), 3.35 (3H, s), 2.14-1.60 (22H, m) and 0.90 (3H, t, J=7.4 Hz).

The examples in the following table were prepared by methods analogous to the method described above (Scheme 21) using the appropriate intermediates.

| **Example** | **Stage 1 Intermediates used** | **Name** | **ESMS** |
|---|---|---|---|
| **6** | 2C&8 | Cyclopentyl (4*S*)-4-[(4-{[(7*R*)-8-cyclopentyl-7-ethyl-5-methyl-6-oxo-5,6,7,8-tetrahydropteridin-2-yl]amino}-3-methoxybenzoyl)amino]-L-prolinate | m/z 606 [M+H]⁺ |
| **7** | 2C & 6A | Cyclopentyl 4-[(4-{[(7*R*)-8-cyclopentyl-7-ethyl-5-methyl-6-oxo-5,6,7,8-tetrahydropteridin-2-yl]amino)-3-methoxybenzoyl)amino]-L-phenylalaninate | m/z 656 [M+H]+ |
| **8** | 2C & 7B | Cyclopentyl *N*⁶-(4-{[(7*R*)-8-cyclopentyl-7-ethyl-5-methyl-6-oxo-5,6,7,8-tetrahydropteridin-2-yl]amino}-3-methoxybenzoyl)lysinate | m/z 622 [M+H]+ |
| **9** | 2C&10 | Cyclopentyl *O*-(4-{[(4-([(7*R*)-cyclopentyl-7-ethyl-5-methyl-6-oxo-5,6,7,8-tetrahydropteridin-2-yl]amino}-3-methoxybenzoyl)amino]methyl}phenyl)-L-homoserinate | m/z 700 [M+H]⁺ |
| **10** | 2C&9 | Cyclopentyl 3-(1-{2-[(4-{[(7*R*)-8-cyclopentyl-7-ethyl-5-methyl-6-oxo-5,6,7,8-tetrahydropteridin-2-yl]amino}-3-methoxybenzoyl)amino]ethyl)piperidin-4-yl)alaninate | m/z 691 [M+H]⁺ |
| ***11** | 2C & 6B | *tert*-Butyl 4-[(4-{[(7*R*)-8-cyclopentyl-7-ethyl-5-methyl-6-oxo-5,6,7,8-tetrahydropteridin-2-yl]amino}-3-methoxybenzoyl)amino]-L-phenylalaninate | m/z 644 [M+H]⁺ |

| | | | |
|---|---|---|---|
| * In order to achieve selective Boc deprotection [Scheme 21 Stage 2] the mixture was stirred at 0°C for 30 minutes instead of RT for 2 hours. | | | |

### Example 12

### tert-Butyl O-(4-{([4-{[(7R)-8-cyclopentyl-7-ethyl-5-methyl-6-oxo-5,6,7,8-tetrahydro pieridin-2-yl]amino}-3-methoxybenzoyl)amino]methyl}phenyl)-L-homoserinate

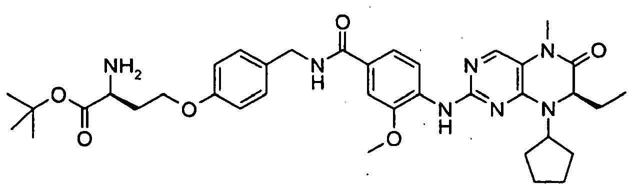

The titled example was prepared according to the general procedure outlined below (Scheme 22).

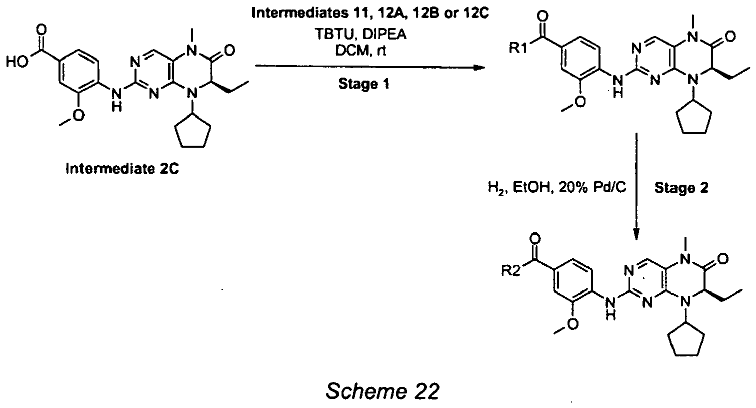

### Stage 1- tert-Butyl N-[(benzyloxy)carbonyl]-O-(4-{[(4-{[(7R)-8-cyclopentyl-7-ethyl-5-methyl-6-oxo-5,6,7,8-tetrahydropteridin-2-yl]amino}-3-methoxybenzoyl)amino]-methyl}phenyl)-L-homoserinate

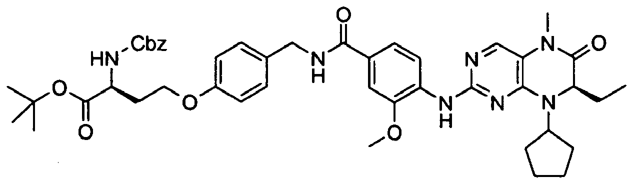

Procedure as in [Scheme 21 Stage 1] using intermediates 2C and 11.

### Stage 2- tert-Butyl O-(4-{[(4-{[(7R)-8-cyclopentyl-7-ethyl-5-methyl-6-oxo-5,6,7,8-tetrahydropteridin-2-yl]amino}-3-methoxybenzoyl)amino]methyl}phenyl)-L-homoserinate

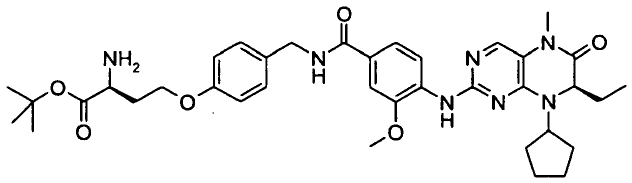

To a solution of *tert*-butyl *N*-[(benzyloxy)carbonyl]-*O*-(4-{[(4-{[(7*R*)-8-cyclopentyl-7-ethyl-5-methyl-6-oxo-5,6,7,8-tetrahydropteridin-2-yl]amino}-3-methoxybenzoyl)amino]-methyl}phenyl)-L-homoserinate (132mg, 0.16mmol) in EtOH (5mL) under a nitrogen atmosphere was added Pd/C (30mg, 20% w/w). The reaction mixture was evacuated and placed under an atmosphere of H₂. This was repeated a further two times and the reaction allowed to stir under an atmosphere of H₂ for 1 hour. The reaction mixture was filtered through Celite^{®} and the filtrate concentrated under reduced pressure and purified by column chromatography (10% MeOH / DCM) to yield the titled example as a white solid (42mg, 38% yield). ESMS: m/z 688 [M+H]⁺. ¹H NMR (300 MHz, C*D*Cl₃) δ: 8.52 (1H, d, J=8.5Hz), 7.56-7.70 (2H, m), 7.48 (1H, d, J=1.3Hz), 7.27-7.32 (2H, m), 6.88 (2H, d, J=8.5Hz), 6.53 (1H, t, J=5.5Hz), 4.44-4.62 (3H, m), 4.21 (1H, dd, J=7.7, 3.6Hz), 3.95 (3H, s), 3.59 (1H, dd, J=7.6, 5.0Hz), 3.31 (3H, s), 2.08-2.28 (4H, m), 1.66-2.01 (10H, m), 1.47 (9H, m) and 0.87 (3H, t, J=7.5Hz).

The examples in the following table were prepared by methods analogous to the method described above (Scheme 22) using the appropriate intermediates.

| **Example** | **Stage 1 Intermediates used** | **Name** | **ESMS** |
|---|---|---|---|
| **13** | 2C & 12A | Cyclopentyl 4-(4-{[(7*R*)-8-cyclopentyl-7-ethyl-5-methyl-6-oxo-5,6,7,8-tetrahydropteridin-2-yl]amino)-3-methoxybenzoyl)piperazine-2-carboxylate | m/z 606 [M+H]⁺ |
| **14** | 2C & 12B | Cyclopentyl 4-{2-[(4-{[(7*R*)-8-cyclopentyl-7-ethyl-5-methyl-6-oxo-5,6,7,8-tetrahydro pteridin-2-yl]amino}-3-methoxybenzoyl) amino]ethyl}piperazine-2-carboxylate | m/z 325 [(M+2)/2]⁺ |
| **15** | 2C & 12C | *tert*-butyl 4-{2-[(4-{[(7*R*)-8-cyclopentyl-7-ethyl-5-methyl-6-oxo-5,6,7,8-tetrahydro pteridin-2-yl]amino}-3-methoxybenzoyl) amino]ethyl}piperazine-2-carboxylate | m/z 637 [M+H]⁺ |

### Example 16

### Cyclopentyl (2S)-2-amino-4-{3-[(4-{[(7R)-8-cyclopentyl-7-ethyl-5-methyl-6-oxo-5,6,7,8-tetrahydropteridin-2-yl]amino}-3-methoxybenzoyl)amino]pyrrolidin-1-yl}butanoate

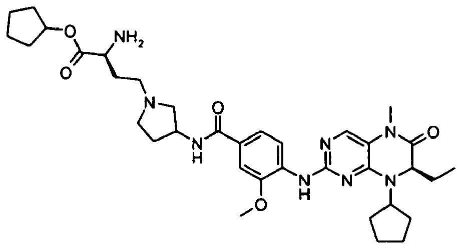

The titled example was prepared according to the general procedure outlined below (Scheme 23).

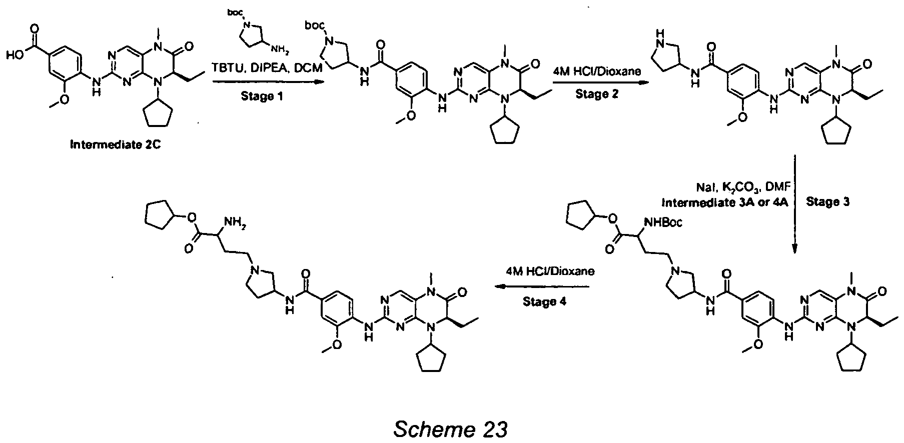

### Stage 1- tert-Butyl 3-[(4-{[(7R)-8-cyclopentyl-7-ethyl-5-methyl-6-oxo-5,6,7,8-tetrahydro pteridin-2-yl]amino}-3-methoxybenzoyl)amino]pyrrolidine-1-carboxylate

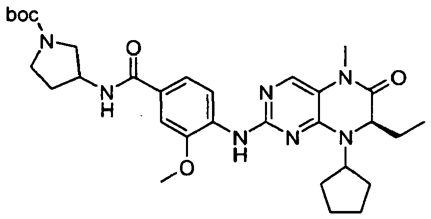

To a solution of 4-{[(7*R*)-8-cyclopentyl-7-ethyl-5-methyl-6-oxo-5,6,7,8-tetrahydropteridin-2-yl] amino}-3-methoxybenzoic acid [Intermediate 2C] (200mg, 0.47mmol) in DCM (10mL) was added TBTU (170mg, 0.52mmol) and DIPEA (163µl, 0.94mmol). The mixture was stirred at RT for 30 minutes. *tert*-Butyl 3-aminopyrrolidine-1-carboxylate (98µl, 0.56mmol) was added and the reaction mixture was stirred at RT for another 2 hours. The mixture was diluted with DCM (10mL), washed with water (2 x 20m L) and brine (10mL). The organic layer was dried (MgSO₄) and concentrated under reduced pressure. The residue was purified by column chromatography (0-1% MeOH in DCM) to afford the product as a yellow solid (220mg, 78% yield). ESMS: m/z 594 [M+H]⁺.

### Stage 2- 4-{[(7R)-8-Cyclopentyl-7-ethyl-5-methyl-6-oxo-5,6,7,8-tetrahydropteridin-2-yl]amino}-3-methoxy-N-pyrrolidin-3-ylbenzamide

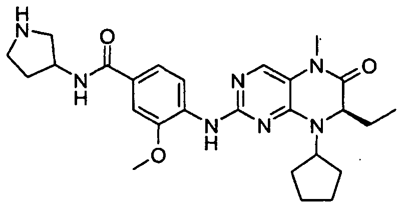

*tert*-Butyl 3-[(4-{((7*R*)-8-cyclopentyl-7-ethyl-5-methyl-6-oxo-5,6,7,8-tetrahydro pteridin-2-yl]amino}-3-methoxybenzoyl)amino]pyrrolidine-1-carboxylate (22mg, 0.36mmol) was dissolved in 4M HCl / dioxane (6mL) and stirred at RT for 1 hour. The reaction was concentrated under reduced pressure to afford the product as a white solid (120mg, 68% yield). ESMS: m/z 494 [M+H]⁺.

### Stage 3- Cyclopentyl (2S)-2-[(tert-butoxycarbonyl)amino]-4-{3-[(4-{[(7R)-8-cyclopentyl-7-ethyl-5-methyl-6-oxo-5,6,7,8-tetrahydropteridin-2-yl]amino}-3-methoxybenzoyl)amino] pyrrolidin-1-yl}butanoate

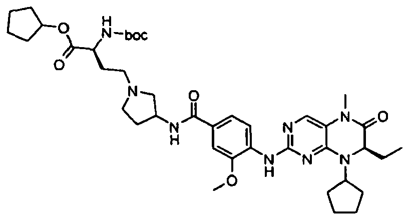

To a stirred solution of 4-{[(7*R*)-8-cyclopentyl-7-ethyl-5-methyl-6-oxo-5,6,7,8-tetrahydro pteridin-2-yl]amino)-3-methoxy-*N*-pyrrolidin-3-ylbenzamide (120mg,0.25mmol) in DMF (5mL) was added K₂CO₃ (140mg, 1.0mmol), Nal (75µl, 0.5mmol) and (S)-cyclopentyl (2*S*)-4-bromo-2-[(*tert*-butoxycarbonyl)amino]butanoate [intermediate 3A] (130mg, 0.37mmol). The reaction mixture was stirred at 80°C overnight and then diluted with EtOAc (10mL). The mixture was washed with water (2 x 10mL) and brine (10mL). The organic layer was dried (MgSO₄) and concentrated under reduced pressure. The residue was purified by column chromatography (0-2% MeOH/DCM) to afford the product as a pale yellow solid (140mg, 71% yield). ESMS: m/z 522 [M+H]⁺.

### Stage 4- Cyclopentyl (2S)-2-amino-4-{3-[(4-{[(7R)-8-cyclopentyl-7-ethyl-5-methyl-6-oxo-5,6,7,8-tetrahydropteridin-2-yl]amino}-3-methoxybenzoyl)amino]pyrrolidin-1-yl}butanoate

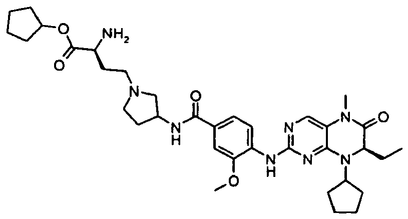

Cyclopentyl (2*S*)-2-[(*tert*-butoxycarbonyl)amino]-4-{3-[(4-{[(7*R*)-8-cyclopentyl-7-ethyl-5-methyl-6-oxo-5,6,7,8-tetrahydropteridin-2-yl]amino}-3-methoxybenzoyl)amino] pyrrolidin-1-yl}butanoate (140mg, 0.18mmol) was dissolved in 4M HCl/dioxane (5mL) and stirred at RT for 2 hours. The reaction was concentrated under reduced pressure. The residue was triturated with Et₂O, filtered and dried under reduced pressure to afford the title example as a white solid (60mg, 50% yield). ESMS: m/z 663 [M+H]⁺. 1H NMR (300 MHz, CDCl₃) δ: 8.56 (1H, d, J=8.5 Hz), 7.69 (1H, s), 7.61 (1H, s), 7.51 (1H, d, J=1.7 Hz), 7.44 (1H, d, J=8.7Hz), 6.66 - 6.72 (1H, m), 5.09 - 5.16 (1H, m), 4.47 - 4.69 (2H, m), 4.23 (1H, dd, J=7.9, 3.8 Hz), 3.99 (3H, s), 3.48 (1H, t, J=6.2 Hz), 3.34 (3H,s), 2.95 - 3.05 (1H, m), 2.83 (1H, d, J=10.0 Hz), 2.46 - 2.71 (3H, m), 2.11 - 2.45 (3H, m), 1.47 - 2.05 (22H, m), 0.89 (3H, t, J=7.5 Hz).

The example in the following table was prepared by methods analogous to the method described above (Scheme 23) using the appropriate intermediates.

| **Example** | **Stage 3 Intermediate used** | **Name** | **ESMS** |
|---|---|---|---|
| 17 | 4A | Cyclopentyl (2*R*)-2-amino-4{3-[(4-{[(7*R*)-8-cyclopentyl-7-ethyl-5-methyl-6-oxo-5,6,7,8-tetrahydropteridin-2-yl]amino}-3-methoxybenzoyl) amino]pyrrolidin-1-yl}butanoate | m/z 663 [M+H]⁺ |

### Example 18

### Cyclopentyl (2S)-2-amino-4-{6-[(4-{[(7R)-8-cyclopentyl-7-ethyl-5-methyl-6-oxo-5,6,7,8-tetrahydropteridin-2-yl]amino}-3-methoxybenzoyl)amino]-3-azabicyclo[3.1.0]hex-3-yl} butanoate

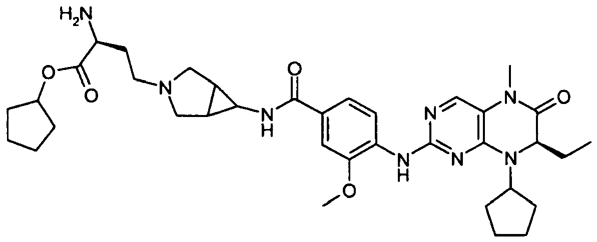

The titled example was prepared according to the procedure outlined below (Scheme 24):

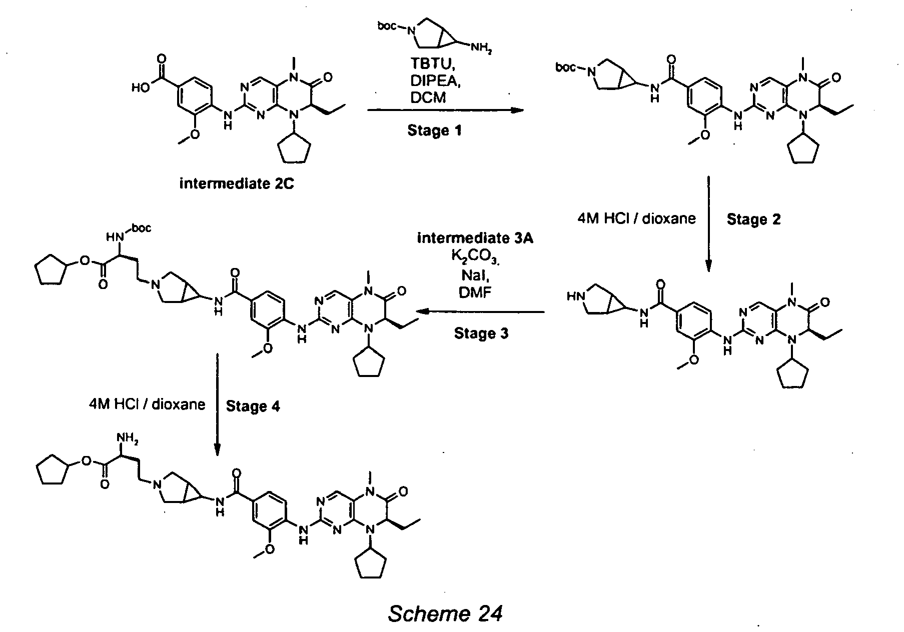

### Stage 1- tert-butyl 6-[(4-{[(7R)-8-cyclopentyl-7-ethyl-5-methyl-6-oxo-5,6,7,8-tetrahydro pteridin-2-yl]am ino}-3-methoxybenzoyl)am ino]-3-azabicyclo[3.1.0]hexane-3-carboxylate

To a stirred solution of 4-{[(7*R*)-8-cyclopentyl-7-ethyl-5-methyl-6-oxo-5,6,7,8-tetrahydropteridin-2-yl] amino}-3-methoxybenzoic acid [Intermediate 2C] (200mg, 0.47mmol) in DCM (10mL) was added DIPEA (0.16mL, 0.94mmol) and TBTU (167mg, 0.52mmol). The reaction stirred at RT for 30 minutes before addition of *tert*-butyl 6-amino-3-azabicyclo[3.1.0]hexane-3-carboxylate [WO2006123121] (111mg, 0.56mmol). The reaction was stirred for a further 30 minutes and then the mixture was diluted with DCM (15mL) and washed with water (2 x 5mL).The organic layer was dried (MgSO₄) and concentrated under reduced pressure. The resulting solid was triturated with Et₂O to afford the product as a white solid (230mg, 81% yield). ESMS: m/z 606 [M+H]⁺,

### Stage 2- N-3-azabicyclo[3.1.0]hex-6-yl-4-{[(7R)-8-cyclopentyl-7-ethyl-5-methyl-6-oxo-5,6,7,8-tetrahydropteridin-2-yl]amino}-3-methoxybenzamide

*tert*-Butyl 6-[(4-{[(7*R*)-8-cyclopentyl-7-ethyl-5-methyl-6-oxo-5,6,7,8-tetrahydro pteridin-2-yl]amino}-3-methoxybenzoyl)amino]-3-azabicyclo[3.1.0]hexane-3-carboxylate (230mg, 0.38mmol) was suspended in 4M HCl/dioxane (5mL) and the reaction mixture was stirred at RT for 1.5 hours and concentrated under reduced pressure. The residue was triturated with Et₂O and then partitioned between DCM (5mL) and sat Na₂CO₃ (5mL). The organic layer washed with sat Na₂CO₃, dried (MgSO₄) and concentrated under reduced pressure to afford the product as a white solid (152mg, 80% yield). ESMS: m/z 506 [M+H]⁺.

### Stage 3- Cyclopentyl (2S)-2-[(tert-butoxycarbonyl)amino]-4-{6-[(4-{[(7R)-8-cyclopentyl-7-ethyl-5-methyl-6-oxo-5,6,7,8-tetrahydropteridin-2-yl]amino}-3-methoxybenzoyl)amino]-3-azabicyclo[3.1.0]hex-3-yl}butanoate

To a stirred solution of *N*-3-azabicyclo[3.1.0]hex-6-yl-4-{[(7*R*)-8-cyclopentyl-7-ethyl-5-methyl-6-oxo-5,6,7,8-tetrahydropteridin-2-yl]amino}-3-methoxybenzamide (152mg, 0.30mmol) in DMF (3mL) was added cyclopentyl (2S)-4-bromo-2-[(*tert*-butoxycarbonyl) amino]butanoate [Intermediate 3A] (157mg, 0.45mmol), K₂CO₃ (166mg, 1.20mmol) and Nal (90mg, 0.60mmol). The mixture was heated at 80°C for 24 hours. The reaction mixture was concentrated under reduced pressure, the resulting residue was dissolved in EtOAc (10mL) and washed with brine (10mL). The organic layer was dried (MgSO₄) and concentrated under reduced pressure to afford the title product as a brown solid (228mg, 98% yield). ESMS: m/z 775 [M+H]⁺.

### Stage 4- Cyclopentyl (2S)-2-amino-4-{6-[(4-{[(7R)-8-cyclopentyl-7-ethyl-5-methyl-6-oxo-5,6,7,8-tetrahydropteridin-2-yl]amino}-3-methoxybenzoyl)amino]-3-azabicyclo[3.1.0]hex-3-yl} butanoate

Cyclopentyl (2*S*)-2-[(*tert*-butoxycarbonyl)amino]-4-{6-[(4-{[(7*R*)-8-cyclopentyl-7-ethyl-5-methyl-6-oxo-5,6,7,8-tetrahydropteridin-2-yl]amino}-3-methoxybenzoyl)amino]-3-aza bicyclo[3.1.0]hex-3-yl}butanoate (228mg, 0.29mmol) was suspended in 4M HCl/dioxane (5mL) and the reaction mixture was stirred at RT for 1.5 hours and concentrated under reduced pressure. The residue was purified using preperative HPLC and then the product concentrated by freeze drying for 60 hours. The resulting solid was dissolved in DCM (5mL) and Na₂CO₃ (5mL) and stirred for 20 minutes. The organic layer was separated, dried (MgSO₄) and concentrated under reduced pressure to afford the title example as a clear oil (23mg, 12% yield). ESMS: m/z 675 [M+H]⁺. ¹H NMR (300 MHz, C*D*Cl₃) δ: 8.47 (1H, d, J=8.5Hz), 7.43-7.65 (3H, m), 7.25 (1H, d, J=6.6Hz), 5.23 (2H, s), 5.16 (1H, t, J=5.9 Hz), 4.34-4.49 (1H, m), 4.15 (1H, dd, J=7.9, 3.8Hz), 3.92 (2H, s), 3.55 (1H, dd, J=8.4, 3.9 Hz), 3.22-3.31 (4H, m), 3.18 (1H, d, J=9.0Hz), 2.92 (1H, br. s), 2.57 (2H, t, J=8.3Hz), 2.34-2.44 (2H, m), 1.38-2.15 (20H, m) and 0.81 (3H, t, J=7.4Hz).

### Example 19

### Cyclopentyl 5-{4-[(4-{[(7R)-8-cyclopentyl-7-ethyl-5-methyl-6-oxo-5,6,7,8-tetrahydropteridin-2-yl]amino}-3-methoxybenzoyl)amino]phenyl}-L-norvalinate

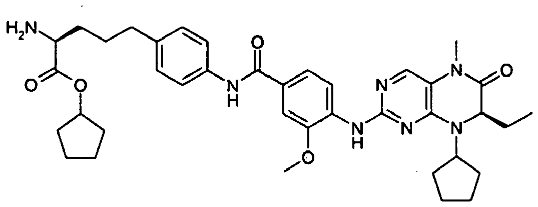

The title compound was prepared by the following methodology (Scheme 25):

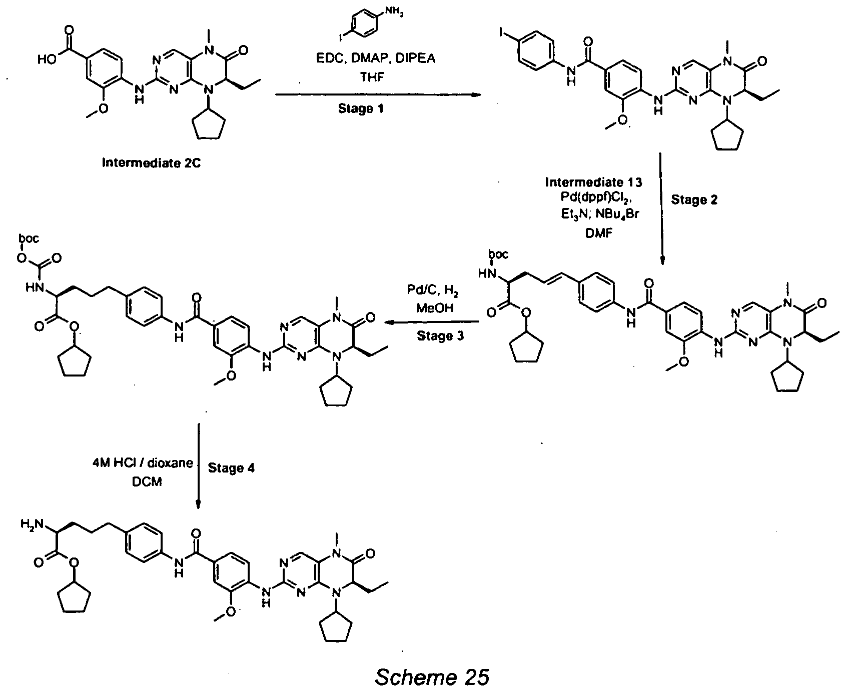

### Stage 1- 4-{[(7R)-8-cyclopentyl-7-ethyl-5-methyl-6-oxo-5,6,7,8-tetrahydropteridin-2-yl]amino}-N-(4-iodophenyl)-3-methoxybenzamide

To a stirred solution of 4-{[(7*R*)-8-cyclopentyl-7-ethyl-5-methyl-6-oxo-5,6,7,8-tetrahydro pteridin-2-yl] amino}-3-methoxybenzoic acid [Intermediate 2C] (200mg, 0.47mmol) in THF (4mL) was added 4-iodoaniline (154mg, 0.71 mmol), DMAP (6mg, 0.05mmol), DIPEA (0.25mL, 1.41mmol) and EDC (99mg, 0.52mmol). The reaction mixture was stirred at RT overnight, washed with water (10mL), dried (MgSO₄), and concentrated under reduced pressure. Purification by column chromatography (40-50% EtOAc /heptane) afforded the product (81.9mg, 28% yield). ESMS m/z: 627 [M+H]⁺.

### Stage 2- Cyclopentyl (2S,4E)-2-[(tert-butoxycarbonyl)amino]-5-{4-[(4-{[(7R)-8-cyclopentyl-7-ethyl-5-methyl-6-oxo-5,6,7,8-tetrahydropteridin-2-yl]amino}-3-methoxy benzoyl)amino]phenyl}pent-4-enoate

To a stirred solution of 4-{[(7*R*)-8-cyclopentyl-7-ethyl-5-methyl-6-oxo-5,6,7,8-tetrahydropteridin-2-yl]amino}-*N*-(4-iodophenyl)-3-methoxybenzamide (81.9mg, 0.13mmol) in DMF (3mL) was added cyclopentyl (2S)-2-[(*tert*-butoxycarbonyl)amino] pent-4-enoate [intermediate 13] (56mg, 0.20mmol), Pd(dppf)Cl₂ (11mg, 0.01mmol), Et₃N (40µl, 0.29mmol) and NBu₄Br (42mg, 0.13mmol). The reaction mixture was heated at 120°C for 1h in the microwave and concentrated under reduced pressure. The crude residue was loaded on silica and purified by column chromatography (40% EtOAc/heptane) to give the product (50mg, 30% yield). ESMS m/z: NI.

### Stage 3- Cyclopentyl N-{[(tert-butoxycarbonyl)oxy]carbonyl}-5-{4-[(4-{[(7R)-8-cyclopentyl -7-ethyl-5-methyl-6-oxo-5,6,7,8-tetrahydropteridin-2-yl]amino}-3-methoxybenzoyl) amino] phenyl}-L-norvalinate

Cyclopentyl (2*S*,4*E*)-2-[(*tert*-butoxycarbonyl)amino]-5-{4-[(4-{[(7*R*)-8-cyclopentyl-7-ethyl-5-methyl-6-oxo-5,6,7,8-tetrahydropteridin-2-yl]amino}-3-methoxybenzoyl)amino] phenyl}pent-4-enoate (50mg, 0.06mmol) in MeOH (5mL) was was passed through an H-Cube™ continuous hydrogenator (Thales Nanotechnology, HC-2, SS). The reaction was performed using a 30mm CatCart™ (10% Pd/C) in full H₂ mode. A flow rate of 1mL/min was maintained for 30 min, with a temperature of 25°C and H₂ pressure of 1 bar. The solution was then evaporated to dryness to afford the product (50mg, 100% yield). ESMS m/z: 784 [M+H]⁺.

### Stage 4- Cyclopentyl 5-{4-[(4-{[(7R)-8-cyclopentyl-7-ethyl-5-methyl-6-oxo-5,6,7,8-tetrahydropteridin-2-yl]amino}-3-methoxybenzoyl)amino]phenyl}-L-norvalinate

To a solution of cyclopentyl *N*-{[(*tert*-butoxycarbonyl)oxy]carbonyl}-5-{4-[(4-{[(7*R*)-8-cyclopentyl -7-ethyl-5-methyl-6-oxo-5,6,7,8-tetrahydropteridin-2-yl]amino}-3-methoxybenzoyl) amino] phenyl}-L-norvalinate (25mg, 0.03mmol) in DCM (1mL) was added 4M HCl/dioxane (30µl, 0.12mmol). The reaction mixture was stirred at RT for 1hour and evaporated under reduced pressure. Purification by preperative HPLC afforded the title example as a white solid (3mg, 14% yield). ESMS m/z: 342 [(M+2)/2]⁺. 1H NMR (300 MHz, MeO*D*) δ; ppm 8.14 (1H, d, J=8.1 Hz), 7.66-7.78 (4H, m), 7.26-7.54 (3H, m), 5.37 (1H, dd, J=4.0, 1.9 Hz), 4.53 (1H, dd, J=6.8, 3.4 Hz), 4.45 (1H, t, J=8.2 Hz), 4.09 (2H, s), 3.38 (3H, s), 2.73-2.95 (2H, m), 1.90-2.21 (10H, m), 1.62-1.87 (12H, m) and 0.94 (3H, t, J=7.5 Hz).

### Example 20

### Cyclopentyl(2S,4E)-2-amino-5-{4-[(4-{[(7R)-8-cyclopentyl-7-ethyl-5-methyl-6-oxo-5,6,7,8-tetrahydropteridin-2-yl]amino}-3-methoxybenzoyl)amino]phenyl}pent-4-enoate

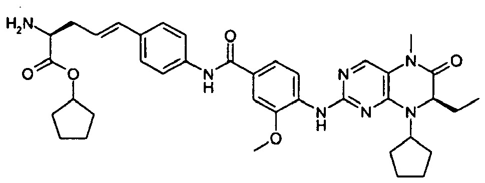

The title compound was prepared by the following methodology (Scheme 26):

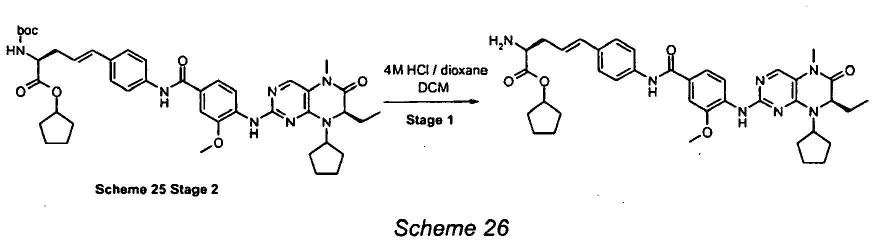

### Stage 1- Cyclopentyl (2S,4E)-2-amino-5-{4-[(4-{[(7R)-8-cyclopentyl-7-ethyl-5-methyl-6-oxo-5,6,7,8-tetrahydropteridin-2-yl]amino}-3-methoxybenzoyl)amino]phenyl}pent-4-enoate

To a stirred solution of cyclopentyl (2*S*,4*E*)-2-[(*tert*-butoxycarbonyl)amino]-5-{4-[(4-{[(7*R*)-8-cyclopentyl-7-ethyl-5-methyl-6-oxo-5,6,7,8-tetrahydropteridin-2-yl]amino}-3-methoxy benzoyl)amino]phenyl}pent-4-enoate [Scheme 25, Stage 2] (36mg, 0.06mmol) in DCM (2mL) was added 4M HCl/dioxane (20µL, 0.08mmol). The reaction mixture was stirred at RT for 2 hours, concentrated under reduced pressure and redissolved in DCM (10mL). The organic layer was washed with 1M NaHCO₃ (10mL), dried (MgSO₄) and evaporated to dryness. Purification by preperative HPLC afforded the titled example as a yellow oil (3mg, 10% yield). ESMS m/z: 342 [M/2]⁺. ¹H NMR (300 MHz, MeOD) δ: 7.55-7.62 (1H, m), 7.39-7.51 (4H, m), 6.61 (1H, d, J=15.6 Hz), 6.17 (1H, ddd, J=15.4, 7.6, 7.3 Hz), 5.26-5.34 (1H, m), 4.39 (1H, dd, J=6.3, 3.3 Hz), 4.32 (1H, t, J=8.8 Hz), 4.17 (1H, t, J=6.2 Hz), 3.25 (3H, s), 2.83 (2H, t, J=6.7 Hz), 1.79-2.06 (9H, m), 1.54-1.78 (9H, m) and 0.86 (3H, t, J=7.4 Hz).

### Example 21

### Cyclopentyl 5-{4-[(4-{[(7R)-8-cyclopentyl-7-ethyl-5-methyl-6-oxo-5,6,7,8-tetrahydropteridin-2-yl]amino}-3-methoxybenzoyl)amino]phenyl}-4-hydroxy-L-norvalinate

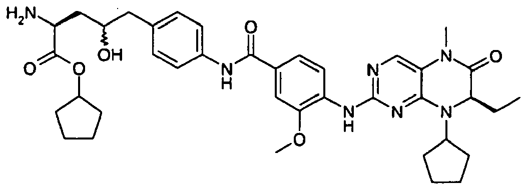

The title compound was prepared by the following methodology (Scheme 27):

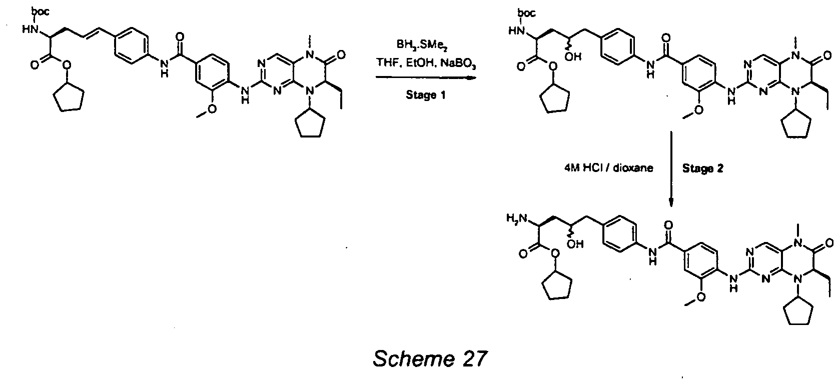

### Stage 1- Cyclopentyl N-(tert-butoxycarbonyl)-5-{4-[(4-{[(7R)-8-cyclopentyl-7-ethyl-5-methyl-6-oxo-5,6,7,8-tetrahydropteridin-2-yl]amino}-3-methoxybenzoyl)amino]phenyl}-4-hydroxy-L-norvalinate

To a solution of cyclopentyl (2*S*,4*E*)-2-[(*tert*-butoxycarbonyl)amino]-5-{4-[(4-{[(7*R*)-8-cyclopentyl-7-ethyl-5-methyl-6-oxo-5,6,7,8-tetrahydropteridin-2-yl]amino}-3-methoxy benzoyl)amino]phenyl}pent-4-enoate [Scheme 25, Stage 2] (130mg, 0.17mmol) in THF (2mL) at 0°C was added borane-dimethylsulfide complex (80µl, 0.87mmol). The mixture was stirred for 5 hours at 0°C before adding ethanol (0.3mL), water (0.27mL) and sodium perborate tetrahydrate (133mg, 0.87mmol). The reaction was stirred at 0°C for a further 3 hours and then at RT for 8 hours. The reaction mixture was concentrated, extracted in EtOAc (3 x 50mL), dried (MgSO₄) and concentrated to give the product (90mg, 75% yield). ESMS: m/z 800 [M+H]⁺.

### Stage 2- Cyclopentyl 5-{4-[(4-{[(7R)-8-cyclopentyl-7-ethyl-5-methyl-6-oxo-5,6,7,8-tetrahydro- pteridin-2-yl]amino}-3-methoxybenzoyl)amino]phenyl}-4-hydroxy-L-norvalinate

Procedure as in [Scheme 26, Stage2]
ESMS: m/z 700 [M+H]⁺. ¹H NMR (300 MHz, MeOD) δ: 8.12 (1 H, d, J=8.3 Hz), 7.74-7.66 (5H, m), 7.40 (2 H, dd J=1.8, 8.6Hz), 5.31 (1 H, m), 4.49-4.38 (3H, m) 4.04 (3 H, s), 3.33 (3H, s), 3.10-3.09 (2H, m), 2.17-1.62 (22H, m) and 0.89 (3 H, t, J=7.5 Hz).

### Example 22

### Cyclopentyl (2S)-2-amino-4-{4-{(4-{[(7R)-8-cyclopentyl-7-ethyl-5-methyl-6-oxo-5,6,7,8-tetrahydropteridin-2-yl]amino}-3-methoxybenzoyl)amino]piperidin-1-yl}butanoate

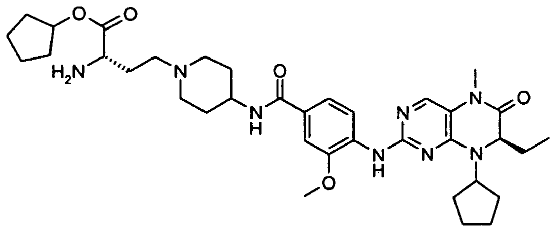

The titled example was prepared according to the general procedure outlined below (Scheme 28):

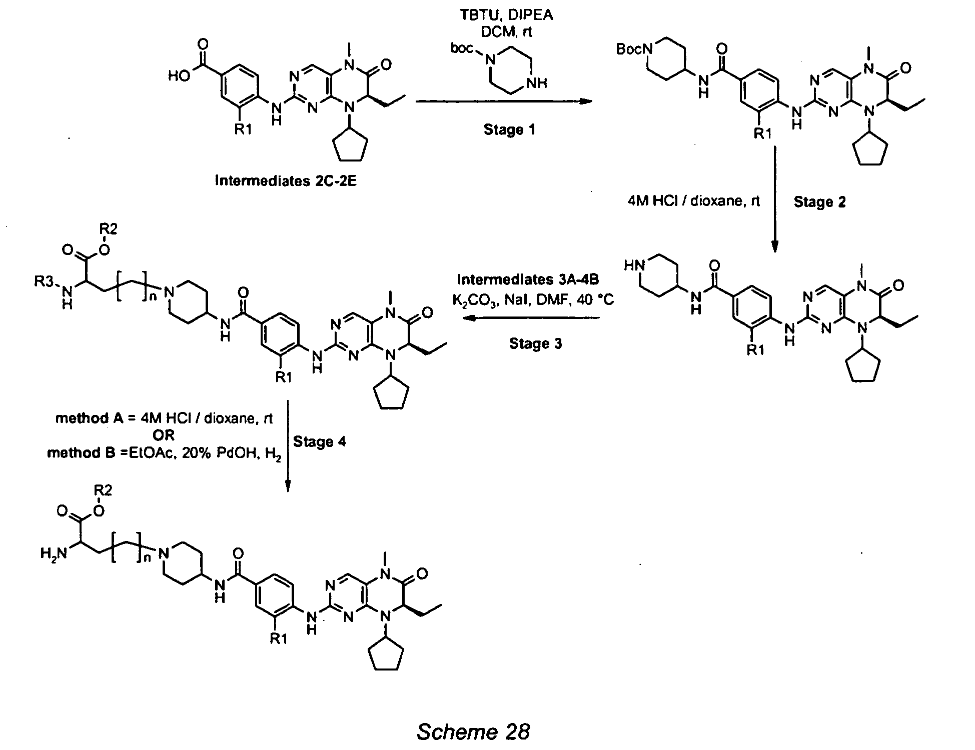

### Stage 1- tert-Butyl 4-[(4-{[(7R)-8-cyclopentyl-7-ethyl-5-methyl-6-oxo-5,6,7,8-tetrahydropteridin-2-yl]amino}-3-methoxybenzoyl)amino]piperidine-1-carboxylate

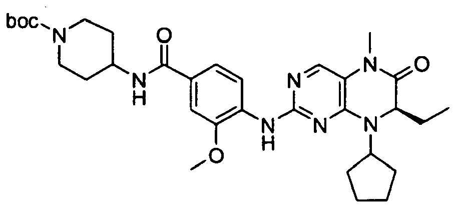

To a suspension of 4-{[(7*R*)-8-Cyclopentyl-7-ethyl-5-methyl-6-oxo-5,6,7,8-tetrahydro pteridin-2-yl] amino}-3-methoxybenzoic acid [Intermediate 2C] (500mg, 1.18mmol) in DCM (20mL) was added TBTU (415mg, 1.29mmol) and DIPEA (0.41mL, 2.35mmol). The reaction mixture was stirred at RT for 30 minutes and then *tert*-butyl 4-aminopiperidine-1-carboxylate (282mg, 1.41 mmol) was added. The reaction mixture was stirred at RT for another 30 minutes and then diluted with DCM (30mL). The solution was washed with water (2 x 30mL), dried (MgSO₄) and concentrated under reduced pressure to leave a thick brown oil. Trituration with Et₂O/heptane (1:3) afforded the product as a beige solid (528mg, 74% yield). ESMS: m/z 608 [M+H]⁺.

### Stage 2- 4-{[(7R)-8-cyclopentyl-7-ethyl-5-methyl-6-oxo-5,6,7,8-tetrahydropteridin-2-yl]amino}-3-methoxy-N-piperidin-4-ylbenzamide

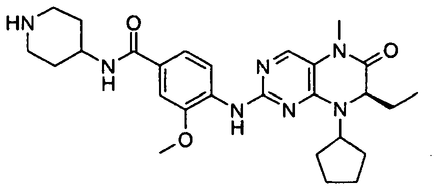

*tert*-Butyl 4-[(4-{[(7*R*)-8-cyclopentyl-7-ethyl-5-methyl-6-oxo-5,6,7,8-tetrahydropteridin-2-yl]amino}-3-methoxybenzoyl)amino]piperidine-1-carboxylate (528mg, 0.87mmol) was suspended in a solution of 4M HCl/dioxane (10mL). The reaction mixture was stirred at RT for 1 hour and concentrated under reduced pressure. The residue was triturated with Et₂O and then partitioned between DCM (100mL) and sat. Na₂CO₃ (50mL). The organic layer was separated, washed with sat. Na₂CO₃ (50mL), dried (MgSO₄) and concentrated under reduced pressure to afford the product as a thick yellow oil, which solidified on standing (407mg, 92% yield). ESMS: m/z 508 [M+H]⁺.

### Stage 3- Cyclopentyl (2S)-2-[(tert-butoxycarbonyl)amino]-4-{4-[(4-{[(7R)-8-cyclopentyl-7-ethyl-5-methyl-6-oxo-5,6,7,8-tetrahydropteridin-2-yl]amino}-3-methoxybenzoyl)amino] piperidin-1-yl}butanoate

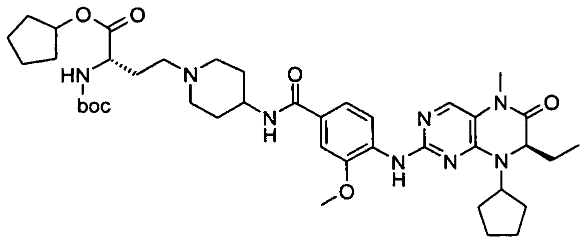

To a solution of 4-{[(7*R*)-8-cyclopentyl-7-ethyl-5-methyl-6-oxo-5,6,7,8-tetrahydropteridin-2-yl]amino}-3-methoxy-*N*-piperidin-4-ylbenzamide (100mg, 0.20mmol) in DMF (2mL) was added cyclopentyl (2*S*)-4-bromo-2-[(*tert*-butoxycarbonyl)amino]butanoate [Intermediate 3A] (103mg, 0.30mmol), K₂CO₃ (109mg, 0.79mmol) and Nal (59mg, 0.40mmol). The reaction mixture was stirred at 80°C for 15 hours, diluted with EtOAc (20mL), washed with water (2 x 20mL), brine (20mL) and dried (MgSO₄). The solvent was concentrated under reduced pressure to leave a yellow oil. Purification by column chromatography (5% MeOH/DCM) afforded the product as a white solid (86 mg, 56% yield). ESMS: m/z 777 [M+H⁺.

### Stage 4 (Method A)- Cyclopentyl (2S)-2-amino-4-{4-[(4-{[(7R)-8-cyclopentyl-7-ethyl-5-methyl-6-oxo-5,6,7,8-tetrahydropteridin-2-yl]amino}-3-methoxybenzoyl)amino]piperidin-1-yl}butanoate.

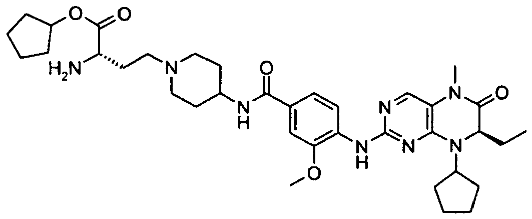

Cyclopentyl (2*S*)-2-[(*tert*-butoxycarbonyl)amino]-4-{4-[(4-{[(7*R*)-8-cyclopentyl-7-ethyl-5-methyl-6-oxo-5,6,7,8-tetrahydropteridin-2-yl]amino}-3-methoxybenzoyl)amino]piperidin-1-yl}butanoate (86mg, 0.11mmol) was suspended in a solution of 4M HCl/dioxane (5mL). The reaction mixture was stirred at RT for 20 minutes and concentrated under reduced pressure. The residue was triturated with Et₂O and then partitioned between DCM (25mL) and sat. Na₂CO₃ (25mL). The organic layer was separated, dried (MgSO₄) and concentrated under reduced pressure to afford the title example as a white solid (49 mg, 65% yield). ESMS m/z 677 [M+H]⁺. ¹H NMR (300 MHz, C*D*₃OD) 8.49 (1H, d, J=9.0 Hz), 7.77 (1H, s), 7.50-7.47 (2H, m), 5.24-5.19 (1H, m), 4.54-4.47 (1H, m), 4.28 (1H, dd, J=3.5, 7.7 Hz), 4.01 (3H, s), 3.95-3.87 (1H, m), 3.66-3.59 (1H, m), 3.32 (3H, s), 3.01 (2H, s), 2.50 (2H, t, J=7.2 Hz), 2.19-2.10 (2H, m), 1.99-1.68 (23H, m) and 0.86 (3H, t, J=7.5 Hz).

### Example 23

### tert-butyl 5-{4-[(4-{[(7R)-8-cyclopentyl-7-ethyl-5-methyl-6-oxo-5,6,7,8-tetrahydropteridin-2-yl]amino}-3-methoxybenzoyl)amino]peridin-1-yl}-L-norvalinate

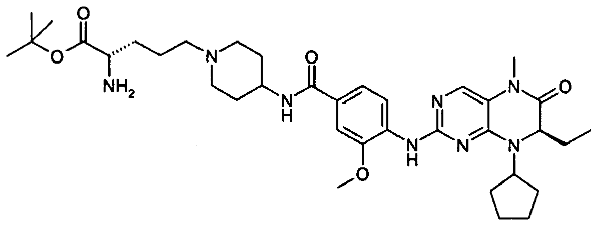

The titled example was prepared according to the general procedure and methodology outlined above (Scheme 28)

### Stages 1-3 As Scheme 28 in using intermediates 2C (stage 1) and 3D (stage 3).

The stage 4 deprotection step was carried out using method B as outlined below.

### Stage 4 (Method B)- tert-butyl 5-{4-[(4-{[(7R)-8-cyclopentyl-7-ethyl-5-methyl-6-oxo-5,6,7,8-tetrahydropteridin-2-yl]amino}-3-methoxybenzoyl)amino]piperidin-1-yl}-L-norvalinate

To a solution of the stage 3 product; *tert*-butyl *N*-[(benzyloxy)carbonyl]-5-{4-[(4-{[(7*R*)-8-cyclopentyl-7-ethyl-5-methyl-6-oxo-5,6,7,8-tetrahydropteridin-2-yl]amino}-3-methoxybenzoyl)amino]piperidin-1-yl}-L-norvalinate (290mg, 0.36mmol) in EtOAc (6mL) was added palladium hydroxide (60mg, 20% wt/wt.). The system was evacuated and put under a hydrogen atmosphere (using a 3-way tap apparatus and hydrogen-filled balloon), this was repeated twice and the mixture was allowed to stir for 90 hour at RT under a hydrogen atmosphere. The system was evacuated of hydrogen and the palladium residues filtered over Celite^{®}. The Celite^{®} was washed thoroughly with EtOAc and the combined filtrates evaporated under reduced pressure. The residue was purified by column chromatography (100% EtOAc to remove impurities followed by 5-10% MeOH / DCM to elute product) to afford the title example as a white solid (37mg, 15% yield). ESMS: m/z 679 [M+H]⁺. ¹H NMR (300 MHz, C*D*Cl₃) δ: 8.53 (1 H, d, J=8.5 Hz), 7.67 (1 H, s), 7.58 (1 H, s), 7.47 (1 H, d, J=1.5 Hz), 7.34 (1 H, dd, J=8.5,1.5 Hz), 6.45 (1 H, d, J=7.5 Hz), 4.50 (1 H, t, J=7.7 Hz), 4.21 (1 H, dd, J=7.8, 3.7 Hz), 4.00 - 4.10 (1H, m), 3.97 (3 H, s), 3.31 - 3.44 (1 H, m), 3.32 (3 H, s), 2.95 (2 H, d, J=8.9 Hz), 2.40 (2 H, t, J=6.7 Hz), 1.59 - 2.21 (20 H, m), 1.45 (9 H, s) and 0.87 (3 H, t, J=7.4 Hz).

The examples in the following table were prepared by methods analogous to the method described above (Scheme 28) using the appropriate intermediates.

| **Example** | **Intermediates used** | | **Stage 4 method** | **Name** | **ESMS** |
|---|---|---|---|---|---|
| | **Stage 1** | **Stage 3** | | | |
| **24** | 2C | 3C | A | Cyclopentyl 5-{4-[(4-{[(7*R*)-8-cyclo pentyl-7-ethyl-5-methyl-6-oxo-5,6,7,8-tetrahydro pteridin-2-yl]amino}-3-methoxybenzoyl) amino]piperidin-1-yl} -L-norvalinate | m/z 691 [M+H]⁺ |
| **25** | 2C | 3B | B | t-Butyl (2*S*)-2-amino-4-{4-[(4-{[(7*R*)-8-cyclopentyl-7-ethyl-5-methyl-6-oxo-5,6,7,8 -tetrahydropteridin-2-yl]amino}-3-methoxy benzoyl) amino] piperidin-1-yl}butanoate | m/z 665 [M+H]⁺ |
| **26** | 2D | 3B | B | t-Butyl (2*S*)-2-amino-4-{4-[(4-{[(7*R*)-8-cyclopentyl-7-ethyl-5-methyl-6-oxo-5,6,7,8-tetra hydropteridin-2-yl]amino}-3-methylbenzoyl) amino] piperidin-1-yl}butanoate | m/z 649 [M+H]⁺ |
| **27** | 2C | 4B | A | cyclopentyl 5-{4-[(4-{[(7*R*)-8-cyclopentyl-7-ethyl-5-methyl-6-oxo-5,6,7,8-tetrahydropteridin-2-yl]amino}-3-methoxybenzoyl)amino]piperidin-1-yl}-D-norvalinate | m/z 691 [M+H]⁺ |
| **28** | 2D | 3A | A | Cyclopentyl (2*S*)-2-amino-4-{4-[(4-{[(7*R*)-8-cyclopentyl-7-ethyl--5-methyl-6-oxo-5,6,7.8-tetra hydropteridin-2-yl]amino}-3-methylbenzoyl) amino] piperidin-1-yl}butanoate | m/z 661 [M+H]⁺ |
| **29** | 2E | 3B | B | t-Butyl (2*S*)-2-amino-4-{4[(4-{[(7*R*)-8-cyclopentyl-7-ethyl-5-ethyl-6-oxo-5,6,7,8-tetrahydropteridin-2-yl]amino}-3-fluorobenzoyl) amino] piperidin-1-yl}butanoate | m/z 653 [M+H]⁺ |
| **30** | 2E | 3A | A | Cyclopentyl (2*S*)-2-amino-4-{4-[(4-{[(7*R*)-8-cyclopentyl-7-ethyl-5-methyl-6-oxo-5,6,7,8-tetra hydropteridin-2-yl]amino}-3-fluorobenzoyl)amino] piperidin-1-yl}butanoate | m/z 665 [M+H]⁺ |
| **31** | 2C | 4A | A | Cyclopentyl (2*R*)-2-amino-4-{4-[(4-{[(7*R*)-8-cyclopentyl-7-ethyl-5-methyl-6-oxo-5,6,7,8-tetra hydropteridin-2-yl]amino)-3-methoxybenzoyl) amino] piperidin-1-yl}butanoate | m/z 677 [M+H]⁺ |

### Example 32

### (1R,2S,5R)-2-isopropyl-5-methylcyclohexyl (2S)-2-amino-4-{4-[(4-{[(7R)-8-cyclopentyl-7-ethyl-5-methyl-6-oxo-5,6,7,8-tetrahydropteridin-2-yl]amino}-3-methoxybenzoyl)amino] piperidin-1-yl}butanoate

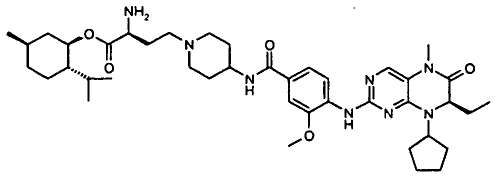

The title compound was prepared by the following methodology (Scheme 29):

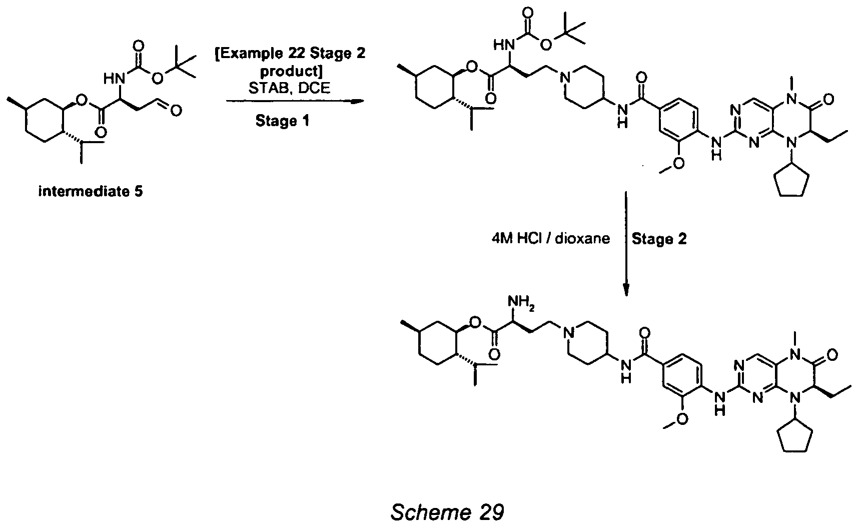

### Stage 1- (1R,2S,5R)-2-isopropyl-5-methylcyclohexyl (2S)-2-[(tert-butoxycarbonyl)amino] -4-{4-[(4-{[(7R)-8-cyclopentyl-7-ethyl-5-methyl-6-oxo-5,6,7,8-tetrahydropteridin-2-yl]amino}-3-methoxybenzoyl)amino]piperidin-1-yl}butanoate

To a solution of (1 R,2S,5R)-2-isopropyl-5-methylcyclohexyl (2S)-2-[(tert-butoxycarbonyl) amino]-4-oxobutanoate [Intermediate 5] (140mg, 0.39mmol) in DCE (15mL) was added 4-{[(7*R*)-8-cyclopentyl-7-ethyl-5-methyl-6-oxo-5,6,7,8-tetrahydropteridin-2-yl]amino}-3-methoxy-*N*-piperidin-4-ylbenzamide [Example 22, Stage 2] (108mg, 0.30mmol). The solution was stirred for 30 min before addition of sodium triacetoxy-borohydride (193mg, 0.91mmol). The reaction stirred for a further 18 hours at RT. Sat NaHCO₃ (10mL) was added and the reaction stirred for 20 minutes. DCM (10mL) was added and the organic layer separated. The aqueous layer was extracted with DCM (2 x 10mL). The combined organic layers were dried (MgSO₄) and concentrated under reduced pressure. Purification by flash column chromatography (2% MeOH / DCM) afforded the product as a clear oil (68mg, 24% yield). ESMS m/z 847 [M+H]⁺.

### Stage 2- (1R,2S,5R)-2-isopropyl-5-methylcyclohexyl (2S)-2-amino-4-{4-[(4-{[(7R)-8-cyclopentyl-7-ethyl-5-methyl-6-oxo-5,6,7,8-tetrahydropteridin-2-yl]amino}-3-methoxybenzoyl)amino] piperidin-1-yl}butanoate

To a solution of (1 R,2S,5R)-2-isopropyl-5-methylcyclohexyl (2*S*)-2-[(*tert*-butoxycarbonyl) amino] -4-{4-[(4-{[(7*R*)-8-cyclopentyl-7-ethyl-5-methyl-6-oxo-5,6,7,8-tetrahydropteridin-2-yl]amino}-3-methoxybenzoyl)amino]piperidin-1-yl}butanoate (11mg, 0.01 mmol) in DCM (1 mL) was added 4M HCl/dioxane (1 mL). The solution was stirred at RT for 3 hours. The mixture was then concentrated under reduced pressure to give a the title example as a white solid (6.1mg, 63% yield). ESMS m/z 747 [M+H]⁺. ¹HNMR (300 MHz, MeO*D*) δ 7.90 (1H, d, J=8.3Hz), 7.68-7.55 (3H, m), 4.56-4.47 (1H, m), 4.45-4.07 (4H, m), 4.01 (3H, s), 3.75 (2H, m), 3.67 (2H, s), 2.77 (1H, m), 2.64-1.06 (38H, m) and 082 (3H, d, J=7.0Hz).

### Example 33

### 4-[(4-{[(7R)-8-cyclopentyl-7-ethyl-5-methyl-6-oxo-5,6,7,8-tetrahydropteridin-2-yl]amino}-3-methoxybenzoyl)amino}-L-phenylalanine

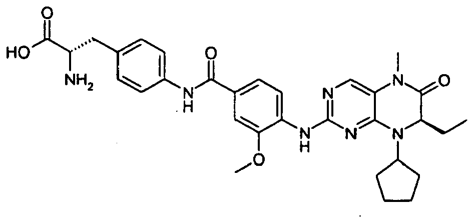

The title compound was prepared by the following methodology (Scheme 30):

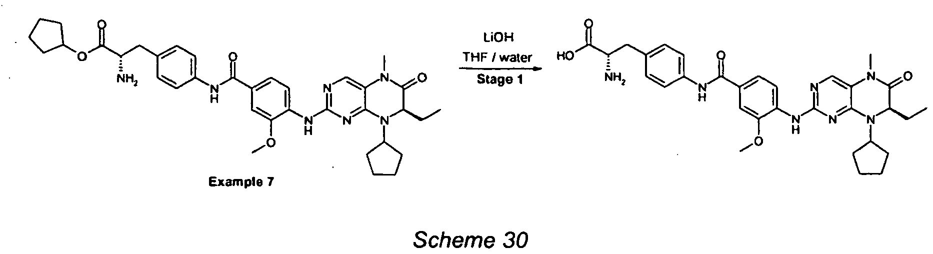

### Stage 1- 4-[(4-{[(7R)-8-cyclopentyl-7-ethyl-5-methyl-6-oxo-5,6,7,8-tetrahydropteridin-2-yl]amino}-3-methoxybenzoyl)amino]-L-phenylalanine

To cyclopentyl 4-[(4-{[(7*R*)-8-cyclopentyl-7-ethyl-5-methyl-6-oxo-5,6,7,8-tetrahydro pteridin-2-yl]amino}-3-methoxybenzoyl)amino]-L-phenylalaninate [Example 7] (45mg, 70µmol) in THF (3mL) was added to a solution of lithium hydroxide (8.4mg, 0.35mmol) in water (3mL). The reaction mixture was stirred at RT overnight and concentrated under reduced pressure. Water (4mL) was added and the pH was adjusted to pH=5-6 with 1 M HCl. The aqueous was extracted with n-butanol (3 x10mL). The combined organic layers were washed with water (5mL), brine (5mL), dried (MgSO₄) and concentrated under reduced pressure. Purification by preparative HPLC afforded the title example as a white solid (37mg, 90% yield). ESMS m/z: 588 [M+H]⁺. ¹H NMR (300 MHz, DMSO-*d6*) δ: 10.17 (1H, s), 8.48 - 8.75 (1H, m), 8.18-8.34 (4H, m), 7.83 (1H, s), 7.73 (2H, d, J=8.7Hz), 7.61-7.67 (2H, m), 7.23 (2H, d, J=8.5 Hz), 4.36 (1H, dd, J=6.8, 3.2 Hz), 4.14-4.30 (2H, m), 3.96 (3H, s), 3.23 (3H, s), 3.07 (2H, d, J=6.4 Hz), 1.43-2.04 (10 H, m) and 0.76 (3 H, t, J=7.4 Hz).

The examples in the following table were prepared by the ester hydrolysis method described above (Scheme 30).

| **Ester Example No.** | **Acid Name** | **Acid Example No.** | **ESMS** |
|---|---|---|---|
| **1** | 4-{[(7*R*)-8-Cyclopentyl-7-ethyl-5-methyl-6-oxo-5,6,7,8-tetrahydropteridin-2-yl]amino}-L-phenylalanine | **34** | m/z 439 [M+H]+ |
| **2** | (2*S*,4*E*)-2-Amino-5-(4-{[(7*R*)-8-cyclopentyl-7-ethyl-5-methyl-6-oxo-5,6,7,8-tetrahydropteridin-2-yl]amino}phenyl)pent-4-enoic acid | **35** | m/z: 465 [M+H]+ |
| **3** | *O*-(4-{[(7*R*)-8-Cyclopentyl-7-ethyl-5-methyl-6-oxo-5,6,7,8-tetrahydropteridin-2-yl]amino}phenyl)-L-homoserine | **36** | m/z 469 [M+H]+ |
| **4** | *O*-(4-{[(7*R*)-8-Cyclopentyl-7-ethyl-5-methyl-6-oxo-5,6,7,8-tetrahydropteridin-2-yl]amino}-3-methoxyphenyl)-L-homoserine | **37** | m/z 499 [M+H]+ |
| **5** | (2*S*)-2-Amino-4-[(4-{[(7*R*)-8-cyclopentyl-7-ethyl-5-methyl-6-oxo-5,6,7,8-tetrahydropteridin-2-yl]amino}-3-methoxybenzoyl)amino]butananoic acid | **38** | m/z 526 [M+H]+ |
| **6** | (4*S*)-4-[(4-{[(7*R*)-8-Cyclopentyl-7-ethyl-5-methyl-6-oxo-5,6,7,8-tetrahydropteridin-2-yl]amino}-3-methoxybenzoyl)amino]-L-proline | **39** | m/z 538 [M+H]+ |
| **8** | *N*⁶-(4-{[(7*R*)-8-cyclopentyl-7-ethyl-5-methyl-6-oxo-5,6,7,8-tetrahydropteridin-2-yl]amino}-3-methoxybenzoyl)lysine | **40** | m/z 554 [M+H]+ |
| **9** | *O*-(4-{[(4-{[(7*R*)-8-Cyclopentyl-7-ethyl-5-methyl-6-oxo-5,6,7,8-tetrahydropteridin-2-yl]amino}-3-methoxybenzoyl)amino]methyl}phenyl)-L-homoserine | **41** | m/z 632 [M+H]+ |
| **13** | 4-(4-{[(7*R*)-8-Cyclopentyl-7-ethyl-5-methyl-6-oxo-5,6,7,8-tetrahydropteridin-2-yl]amino}-3-methoxybenzoyl)piperazine-2-carboxylic acid | **42** | m/z 538 [M+H]+ |
| **14** | 4-{2-[(4-{[(7*R*)-8-Cyclopentyl-7-ethyl-5-methyl-6-oxo-5,6,7,8-tetrahydro pteridin-2-yl]amino}-3-methoxybenzoyl) amino]ethyl}piperazine-2-carboxylic acid | **43** | m/z 581 [M+H]+ |
| **16** | (2*S*)-2-amino-4-{3-[(4-{[(7*R*)-8-Cyclopentyl-7-ethyl-5-methyl-6-oxo-5,6,7,8-tetrahydropteridin-2-yl]amino}-3-methoxybenzoyl)amino]pyrrolidin-1-yl}butanoic acid | **44** | m/z 595 [M+H]+ |
| **18** | (2*S*)-2-Amino-4-{6-[(4-{[(7*R*)-8-cyclopentyl-7-ethyl-5-methyl-6-oxo-5,6,7,8-tetrahydropteridin-2-yfjamino}-3-methoxybenzoyl}amino]-3-azabicyclo[3.1.0]hex-3-yl} butanoic acid | **45** | m/z 607 [M+H]+ |
| **24** | 5-{4-[(4-{[(7*R*)-8-Cyclo pentyl-7-ethyl-5-methyl-6-oxo-5,6,7,8-tetrahydro pteridin-2-yl]amino}-3-methoxybenzoyl)amino]piperidin-1-yl} -L-norvaline | **46** | m/z 623 [M+H]+ |
| **27** | 5-{4-[(4-{[(7*R*)-8-Cyclopentyl-7-ethyl-5-methyl-6-oxo-5,6,7,8-tetrahydropteridin-2-yl]amino}-3-methoxybenzoyl)amino]piperidin-1-yl}-D-norvaline | **47** | m/z 623 [M+H]+ |
| **28** | (2*S*)-2-Amino-4-{4-[(4-{[(7*R*)-8-cyclopentyl-7-ethyl-5-methyl-6-oxo-5,6,7,8-tetra hydropteridin-2-yl]amino}-3-methylbenzoyl)amino] piperidin-1-yl}butanoic acid | **48** | m/z 593 [M+H]+ |
| **30** | (2*S*)-2-Amino-4-{4-[(4-{[(7*R*)-8-cyclopentyl-7-ethyl-5-methyl-6-oxo-5,6,7,8-tetra hydropteridin-2-yl]amino}-3-fluorobenzoyl)amino] piperidin-1-yl}butanoic acid | **49** | m/z 598 [M+H]+ |
| **31** | (2*R*)-2-Amino-4-{4-[(4-{[(7*R*)-8-cyclopentyl-7-ethyl-5-methyl-6-oxo-5,6,7,8-tetra hydropteridin-2-yl]amino}-3methoxybenzoyl)amino] piperidin-1-yl}butanoic acid | **50** | m/z 609 [M+H]+ |
| **7** | 4-[(4-{[(7*R*)-8-cyclopentyl-7-ethyl-5-methyl-6-oxo-5,6,7,8-tetrahydropteridin-2-yl]amino}-3-methoxybenzoyl)amino]-L-phenylalanine | **51** | m/z 588 [M+H]+ |
| **13** | 4-(4-{[(7*R*)-8-cyclopentyl-7-ethyl-5-methyl-6-oxo-5,6,7,8-tetrahydropteridin-2-yl]amino}-3-methoxybenzoyl)piperazine-2-carboxylic acid | **52** | m/z 539 [M+H]+ |
| **22** | (2*S*)-2-amino-4-{4-[(4-{[(7*R*)-8-cyclopentyl-7-ethyl-5methyl-6-oxo-5,6.7,8-tetrahydropteridin-2-yl]amino}-3-methoxybenzoyl)amino]piperidin-1-yl}butanoic acid | **53** | m/z 609 [M+H]+ |

### Measurement of Biological Activity

### PLK1 Enzyme Assay

The ability of compounds to inhibit PLK-1 kinase activity was measured in an assay performed by Invitrogen (Paisley, UK). The Z'-LYTE™ biochemical assay employs a fluorescence-based, coupled-enzyme format and is based on the differential sensitivity of phosphorylated and non-phosphorylated peptides to proteolytic cleavage. The peptide substrate is labelled with two fluorophores-one at each end-that make up a FRET pair. In the primary reaction, the kinase transfers the gamma-phosphate of ATP to a single serine or threonine residue in a synthetic FRET-peptide. In the secondary reaction, a site-specific protease recognizes and cleaves non-phosphorylated FRET-peptides. Phosphorylation of FRET-peptides suppresses cleavage by the Development Reagent. Cleavage disrupts FRET between the donor (i.e., coumarin) and acceptor (i.e., fluorescein) fluorophores on the FRET-peptide, whereas uncleaved, phosphorylated FRET-peptides maintain FRET. A radiometric method, which calculates the ratio (the Emission Ratio) of donor emission to acceptor emission after excitation of the donor fluorophore at 400nm, is used to quantitate reaction progress.

The final 10 µL Kinase Reaction consists of 2.8-25.3ng PLK1, 2µM Ser/Thr 16 Peptide substrate and ATP in 50mM HEPES pH 7.5, 0.01% BRIJ-35, 10mM MgCl2, 1mM EGTA. The assay is performed at an ATP concentration at, or close to, the Km. After the 60 minute Kinase Reaction incubation at RT, 5µL of a 1:8 dilution of Development Reagent is added. The assay plate is incubated for a further 60 minutes at RT and read on a fluorescence plate reader.

Duplicate data points are generated from a 1/3 log dilution series of a stock solution of test compound in DMSO. Nine dilutions steps are made from a top concentration of 10µM, and a 'no compound' blank is included. Data is collected and analysed using XL*fit* software from IDBS. The dose response curve is curve fitted to model number 205 (sigmoidal dose-response model). From the curve generated, the concentration giving 50% inhibition is determined and reported. IC50 results were allocated to one of 3 ranges as follows:
Range A: IC50<100nM
Range B: IC50 from 100nM to 500nM
Range C: IC50 >500nM
NT = Not tested

The results obtained for compounds of the Examples herein are given in the table below.

### Cell inhibition Assay

### Cell inhibition assays were carried out using either method A or method B

### Method A

Cells were seeded in 96W tissue culture plates (1 well = 30mm²) at a density of 500 cells per well in 50µl of the appropriate culture medium (see below). 24hrs later 50µl of the compound prepared in the same medium was added as 4 fold dilutions to give final concentrations in the range 0.15nM-2500nM (n=6 for each concentration). The plates were then incubated at 37°C, 5% CO₂ for 120hrs. Cell proliferation was assessed using WST-1 (a metabolic indicator dye, Roche Cat no. 1 644 807) according to the manufacturers instructions. The results were calculated as percentage of vehicle response and IC50 values represent the concentration of compound that inhibited the vehicle response by 50%.

HCT-116 culture medium - Dulbeccos MEM (Sigma D6546) plus 10% heat inactivated fetal calf serum (Hyclone SH30071 Thermo Fischer Scientific) containing 2mM Glutamine (Sigma cat no G-7513) and 50U/ml Penicillin and Streptomycin Sulphate (Sigma Cat no P-0781).

### Method B

Cells were seeded in 96W tissue culture plates in 50µl of the appropriate culture medium (1 well = 30mm²) at a density according to cell type [HCT-116. 750cells/well, Hut-78 & U937, 1500cells/well].

24hrs later 50µl of the compound prepared in the same medium was added, made as 12 fold dilutions to give final concentrations from 10000nM to 0.28pM (n=6 for each concentration).

The plates were then incubated at 37°C, 5% CO₂ for 72hrs.

A tritiated thymidine incorporation assay was used as a measure of cell proliferation. In short, cells were incubated with 0.4µCi/well for 4hrs before harvesting onto filtermats. These were dried, meltilex scintillation sheets melted on, then sealed in bags and ³H emission counted on a Trilux microbeta counter.

The results are calculated as percentage of vehicle response and IC50 values represent the concentration of compound that inhibits the vehicle response by 50%.

IC50 results were allocated to one of 3 ranges as follows:
Range A: IC50 <100nM
Range B: IC50 from 100nM to 500nM
Range C: IC50 >500nM
NT = Not tested

The results obtained for compounds of the Examples herein are given in the table below.

| **Example Number** | **Inhibitor Activity vs PLK1** | **Inhibitor Activity vs HCT 116 cell line (method A)** |
|---|---|---|
| **1** | A | B |
| **2** | A | A |
| **3** | A | B |
| **4** | A | A |
| **5** | A | A |
| **6** | A | A |
| **7** | B | A |
| **8** | A | A |
| **9** | B | A |
| **10** | A | A |
| **11** | A | B |
| **12** | A | A |
| **13** | A | B |
| **14** | A | A |
| **15** | A | A |
| **16** | A | A |
| **17** | A | A |
| **18** | A | A |
| **19** | A | A |
| **20** | A | A |
| **21** | NT | NT |
| **22** | A | A |
| **23** | A | A |
| **24** | A | A |
| **25** | A | A |
| **26** | A | A |
| **27** | A | A |
| **28** | A | A |
| **29** | A | A |
| **30** | A | A |
| **31** | A | A |
| **32** | A | NT |
| **33** | A | NT |
| **34** | A | NT |
| **35** | A | NT |
| **36** | A | NT |
| **37** | A | NT |
| **38** | A | NT |
| **39** | A | NT |
| **40** | A | NT |
| **41** | A | NT |
| **42** | A | NT |
| **43** | A | NT |
| **44** | A | NT |
| **45** | A | NT |
| **46** | A | NT |
| **47** | A | NT |
| **48** | A | NT |
| **49** | A | NT |
| **50** | A | NT |
| **51** | A | NT |
| **52** | A | NT |
| **53** | A | NT |

### Broken Cell Carboxylesterase Assay

Any given compound of the present invention wherein R₇ is an ester group, may be tested to determine whether it meets the requirement that it be hydrolysed by intracellular esterases, by testing in the following assay.

### Preparation of cell extract

U937 or HCT 116 tumour cells (~ 10⁹) were washed in 4 volumes of Dulbeccos PBS (~ 1 litre) and pelleted at 525 g for 10 min at 4°C. This was repeated twice and the final cell pellet was resuspended in 35 mL of cold homogenising buffer (Trizma 10 mM, NaCl 130 mM, CaCl₂ 0.5 mM pH 7.0 at 25°C). Homogenates were prepared by nitrogen cavitation (700 psi for 50 min at 4°C). The homogenate was kept on ice and supplemented with a cocktail of inhibitors at final concentrations of:
Leupeptin 1 µM
Aprotinin 0.1 µM
E64 8 µM
Pepstatin 1.5 µM
Bestatin 162 µM
Chymostatin 33 µM

After clarification of the cell homogenate by centrifugation at 525g for 10 min, the resulting supernatant was used as a source of esterase activity and was stored at -80°C until required.

### Measurement of ester cleavage

Hydrolysis of esters to the corresponding carboxylic acids can be measured using the cell extract, prepared as above. To this effect cell extract (∼30 µg / total assay volume of 0.5 mL) was incubated at 37°C in a Tris- HCl 25 mM, 125 mM NaCl buffer, pH 7.5 at 25°C. At zero time the ester (substrate) was then added at a final concentration of 2.5 µM and the samples were incubated at 37°C for the appropriate time (usually 0 or 80 min). Reactions were stopped by the addition of 3 x volumes of acetonitrile. For zero time samples the acetonitrile was added prior to the ester compound. After centrifugation at 12000 g for 5 min, samples were analysed for the ester and its corresponding carboxylic acid at RT by LCMS (Sciex API 3000, HP1100 binary pump, CTC PAL). Chromatography was based on an AceCN (75x2.1 mm) column and a mobile phase of 5-95 % acetonitrile in water /0.1 % formic acid.

The table below presents data showing that several amino acid ester motifs, conjugated to various intracellular enzyme inhibitors by several different linker chemistries are all hydrolysed by intracellular carboxyesterases to the corresponding acid.

| **Structure of amino acid ester conjugate** | **R** | **Linker** | **Hydrolysis Rate Range U937Cells(pg/mL/min)** | **Preparation of amino ester conjugate** |
|---|---|---|---|---|
| 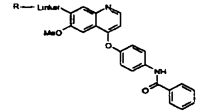 | 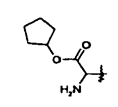 | -CH2CH2O- | 100-1000 | WO2006117552 |
| 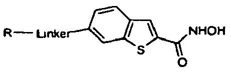 | 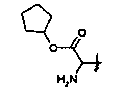 | 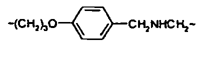 | 1000-50000 | WO2006117548 |
| 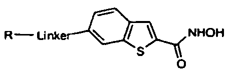 | 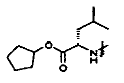 | 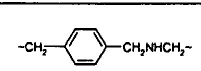 | >50000 | W02006117549 |
| 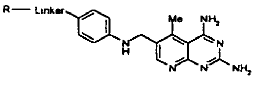 | 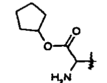 | -CH2CH2O- | >50000 | W02006117567 |
| 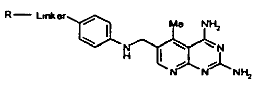 | 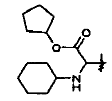 | -CH2CH2O- | 1000-50000 | WO2006117567 |
| 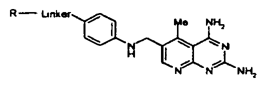 | 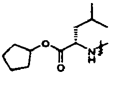 | -CH2- | 1000-50000 | W02006117567 |
| 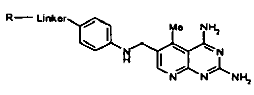 | 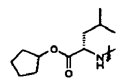 | -CO- | >50000 | WO2006117567 |
| 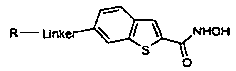 | 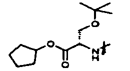 | 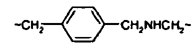 | >50000 | WO2006117549 |
| 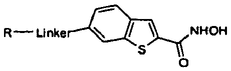 | 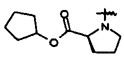 | 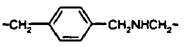 | >50000 | W02006117549 |

The table below shows that Example 22 containing a cleavable esterase motif has much greater activity in cells than the compound lacking the esterase motif, compound I (Example 46 in WO04076454), even though both have similar enzyme activities.

| **Compound** | **Structure** | **Inhibition of PLK (IC50, nM)** | | **Inhibition of U937 cell proliferation (IC50, nM) (method B)** | **Ratio cell/enzyme** |
|---|---|---|---|---|---|
| | | **ester** | **acid** | | |
| Compound I | 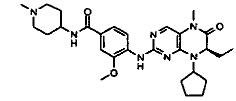 | 4 | | 1.6 | 0.4 |
| Example 22 | 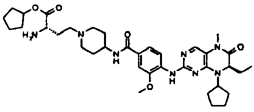 | 6 | 6 (Example 53) | 0.09 | 0.015 |

## Claims

1. A compound of formula (I), or a salt, N-oxide, hydrate or solvate thereof: 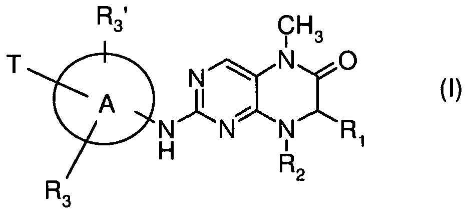 wherein
**R₁** is hydrogen, or an optionally substituted (C₁-C₆)alkyl, (C₂-C₆)alkenyl, (C₂-C₆)alkynyl or (C₃-C₆)cycloalkyl group;
**R₂** is hydrogen, or an optionally substituted (C₁-C₆)alkyl, (C₂-C₆)alkenyl, (C₂-C₆)alkynyl or (C₃-C₆)cycloalkyl group;
**R₃** and **R₃**' are independently selected from hydrogen, -CN, hydroxyl, halogen, optionally substituted (C₁-C₆)alkyl, (C₂-C₆)alkenyl, (C₂-C₆)alkynyl or (C₃-C₆)cycloalkyl, -NR₅R₆ or C₁-C4 alkoxy, wherein R₅ and R₆ are independently hydrogen or optionally substituted (C₁-C₆)alkyl;
ring **A** is an optionally substituted phenyl, pyridinyl or pyrimidinyl ring;
**T** is a radical of formula R-L¹-Y¹- wherein
**Y¹** is a bond, -O-, -S-, -NR₆-, -(C=O)-, -S(O₂)- -(C=O)NR₆-, -NR₆(C=O)-, -S(O₂)NR₆-, -NR₆S(O₂)-, or -NR₆(C=O)NR₉-, wherein R₆ and R₉ are independently hydrogen or optionally substituted (C₁-C₆)alkyl;
**L¹** is a divalent radical of formula -(Alk¹)ₘ(Q)ₙ(Alk²)ₚ- wherein
**m**, **n** and **p** are independently 0 or 1,
**Q** is (i) an optionally substituted divalent mono- or bicyclic carbocyclic or heterocyclic radical having 5-13 ring members, or (ii), in the case where p is 0, a divalent radical of formula -Q¹-X²- wherein X² is -O-, -S- or NR^{A}- wherein
R^{A} is hydrogen or optionally substituted C₁-C₃ alkyl, and Q¹ is an optionally substituted divalent mono- or bicyclic carbocyclic or heterocyclic radical having 5-13 ring members,
**Alk¹** and **Alk²** independently represent optionally substituted divalent (C₃-C₆)cycloalkyl radicals, or optionally substituted straight or branched, (C₁-C₆)alkylene, (C₂-C₆)alkenylene, or (C₂-C₆)alkynylene radicals which may optionally contain or terminate in an ether (-O-), thioether (-S-) or amino (-NR^{A}-) link wherein R^{A} is hydrogen or optionally substituted (C₁-C₃)alkyl;
**R** is a radical of formula (X) or (Y) 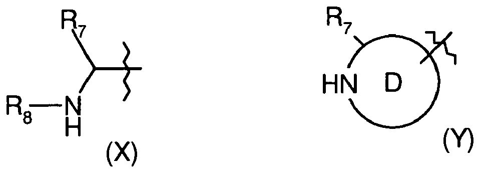 wherein
**R₇** is a carboxylic acid group (-COOH), or an ester group which is hydrolysable by one or more intracellular carboxylesterase enzymes to a carboxylic acid group;
**R₈** is hydrogen; or optionally substituted C₁-C₆ alkyl, C₃-C₇cycloalkyl, phenyl or pyridyl or -(C=O)R₆, -(C=O)OR₆, or -(C=O)NR₆ wherein R₆ is hydrogen or optionally substituted (C₁-C₆)alkyl; and
**D** is a monocyclic heterocyclic ring of 5 or 6 ring atoms wherein R₇ is linked to a ring carbon adjacent the ring nitrogen shown, and ring D is optionally fused to a second carbocyclic or heterocyclic ring of 5 or 6 ring atoms in which case the bond shown intersected by a wavy line may be from a ring atom in said second ring; and wherein, unless otherwise specified, the term "substituted" means substituted with up to four compatible substituents, each of which independently selected from: (C₁-C₆)alkyl, (C₁-C₆)alkoxy, hydroxy, hydroxy(C₁-C₆)alkyl, mercapto, mercapto(C₁-C₆)alkyl, (C₁-C₆)alkylthio, phenyl, halo (including fluoro, bromo and chloro), trifluoromethyl, trifluoromethoxy, nitro, nitrile (-CN), oxo, -COOH, -COOR^{A}, -COR^{A}, -SO₂R^{A},-CONH₂, -SO₂NH₂, -CONHR^{A}, -SO₂NHR^{A},
-CONR^{A}R^{B}, -SO₂NR^{A}R^{B}, -NH₂, -NHR^{A}, -NR^{A}R^{B}, -OCONH₂, -OCONHR^{A},-OCONR^{A}R^{B},
-NHCOR^{A}, -NHCOOR^{A}, -NR^{B}COOR^{A}, -NHSO₂OR^{A}, -NR^{B}SO₂OH, -NR^{B}SO₂OR^{A},
-NHCONH₂, -NR^{A}CONH₂,-NHCONHR^{B},-NR^{A}CONHR^{B}, -NHCONR^{A}R^{B}, or
-NR^{A}CONR^{A}R^{B} wherein R^{A} and R^{B} are independently a (C₁-C₆)alkyl, (C₃-C₆) cycloalkyl, phenyl or monocyclic heteroaryl having 5 or 6 ring atoms, or R^{A} and R^{B} when attached to the same nitrogen atom form a cyclic amino group.

2. A compound as claimed in claim 1 wherein R₁ is ethyl.

3. A compound as claimed in claim 1 or claim 2 wherein R₂ is cyclopentyl.

4. A compound as claimed in any of the preceding claims wherein ring A is a phenyl ring.

5. A compound as claimed in any of claims 1 to 4 wherein R₃ is methoxy, fluoro or chloro, and R'₃ is hydrogen, fluoro or chloro.

6. A compound as claimed in claim 1 having formula (IA): 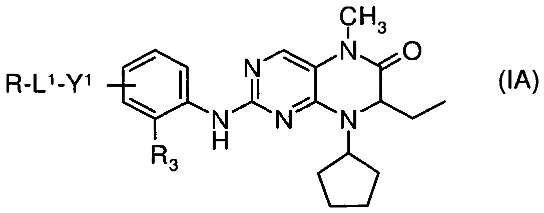 wherein R₃ is methoxy, fluoro or chloro, and the remaining variables are as defined in claim 1.

7. A compound as claimed in any of the preceding claims wherein R₇ is of formula -(C=O)OR₁₀ wherein R₁₀ is R₁₁R₁₂R₁₃C- wherein
(i) R₁₁ is hydrogen or optionally substituted (C₁-C₃)alkyl-(Z¹)ₐ [(C₁-C₃)alkyl]_{b}- or (C₂-C₃)alkenyl-(Z¹)ₐ-[(C₁-C₃)alkyl]_{b}- wherein a and b are independently 0 or 1 and Z¹ is -O-, -S-, or -NR₁₄- wherein R₁₄ is hydrogen or (C₁-C₃)alkyl; and R₁₂ and R₁₃ are independently hydrogen or (C₁-C₃)alkyl-;
(ii) R₁₁ is hydrogen or optionally substituted R₁₅R₁₆N-(C₁-C₃)alkyl- wherein R₁₅ is hydrogen or (C₁-C₃)alkyl and R₁₆ is hydrogen or (C₁-C₃)alkyl; or R₁₅ and R₁₆ together with the nitrogen to which they are attached form an optionally substituted monocyclic heterocyclic ring of 5- or 6- ring atoms or bicyclic heterocyclic ring system of 8 to 10 ring atoms, and R₁₂ and R₁₃ are independently hydrogen or (C₁-C₃)alkyl-;or
(iii) R₁₁ and R₁₂ taken together with the carbon to which they are attached form an optionally substituted monocyclic carbocyclic ring of from 3 to 7 ring atoms or bicyclic carbocyclic ring system of 8 to 10 ring atoms, and R₁₃ is hydrogen.

8. A compound as claimed in claim 7 wherein R₁₀ is cyclopentyl or tert-butyl.

9. A compound as claimed in any of the preceding claims wherein R is a radical of formula (X) and R₈ is hydrogen.

10. A compound as claimed in any of the preceding claims wherein, in the radical L¹, Y¹ is -NHC(=O)-.

11. A compound as claimed in any of the preceding claims wherein, in the radical L¹, Alk¹ and Alk² radicals, when present, are selected from -CH₂-, -CH₂CH₂- -CH₂CH₂CH₂-, -CH₂CH(OH)CH₂-, -CH₂CH₂CH₂CH₂-, -CH=CH-, -CH=CHCH₂-, -CH₂CH=CH-, -CH₂CH=CHCH₂-, -C≡C-, -C≡CCH₂-, -CH₂C≡C-, CH₂C≡CCH₂ -CH₂W-, -CH₂CH₂W-, -CH₂CH₂WCH₂-, -CH₂CH₂WCH(CH₃)-, -CH₂WCH₂CH₂-, -CH₂WCH₂CH₂WCH₂-, -WCH₂CH₂-, -CH₂CH₂N(CH₂CH₂OH)CH₂, and divalent cyclopropyl, cyclopentyl and cyclohexyl radicals; W being -O-, -S-, -NH-, or -N(CH₃)-.

12. A compound as claimed in any of claims 1 to 11 wherein Q, when present, is a divalent 1,4-phenylene, 1,4-piperidinylene, or 1,4- piperazinylene radical.

13. A compound as claimed in claim 1 selected from the group consisting of:
Cyclopentyl 4-[(4-{[(7*R*)-8-cyclopentyl-7-ethyl-5-methyl-6-oxo-5,6,7,8-tetrahydro pteridin-2-yl]amino}-3-methoxybenzoyl)amino]-L-phenylalaninate,
Cyclopentyl *O*-(4-{[(4-{[(7*R*)-8-cyclopentyl-7-ethyl-5-methyl-6-oxo-5,6,7,8-tetrahydro pteridin-2-yl]amino}-3-methoxybenzoyl)amino]methyl} phenyl)-L-homoserinate,
*tert*-butyl 4-[(4-{[(7*R*)-8-cyclopentyl-7-ethyl-5-methyl-6-oxo-5,6,7,8-tetrahydropteridin-2-yl]amino}-3-methoxybenzoyl)amino]-L-phenylalaninate,
*tert*-Butyl *O*-(4-{[(4-{[(7*R*)-8-cyclopentyl-7-ethyl-5-methyl-6-oxo-5,6,7,8-tetrahydro pteridin-2-yl]amino}-3-methoxybenzoyl)amino]methyl}phenyl)-L-homoserinate,
Cyclopentyl 4-{2-[(4-{[(7*R*)-8-cyclopentyl-7-ethyl-5-methyl-6-oxo-5,6,7,8-tetrahydro pteridin-2-yl]amino}-3-methoxybenzoyl) amino]ethyl}piperazine-2-carboxylate,
te*r*t-butyl 4-{2-[(4-{[(7*R*)-8-cyclopentyl-7-ethyl-5-methyl-6-oxo-5,6,7,8-tetrahydro pteridin-2-yl]amino}-3-methoxybenzoyl) amino]ethyl}piperazine-2-carboxylate,
*C*yclopentyl (2*S*)-2-amino-4-{4-[(4-{[(7*R*)-8-cyclopentyl-7-ethyl-5-methyl-6-oxo-5,6,7,8-tetrahydropteridin-2-yl]amino}-3-methoxybenzoyl)amino]piperidin-1-yl}butanoate,
*tert*-butyl 5-{4-[(4-{[(7*R*)-8-cyclopentyl-7-ethyl-5-methyl-6-oxo-5,6,7,8-tetrahydro pteridin-2-yl]amino}-3-methoxybenzoyl)amino]piperidin-1-yl}-L-norvalinate,
Cyclopentyl 5-{4-[(4-{[(7*R*)-8-cyclo pentyl-7-ethyl-5-methyl-6-oxo-5,6,7,8-tetrahydro pteridin-2-yl]amino}-3-methoxybenzoyl) amino]piperidin-1-yl}-L-norvalinate,
t-butyl (2*S*)-2-amino-4-{4-[(4-{[(7*R*)-8-cyclopentyl-7-ethyl-5-methyl-6-oxo-5,6,7,8-tetrahydropteridin-2-yl]amino}-3-methoxy benzoyl) amino] piperidin-1-yl}butanoate,
t-butyl (2*S*)-2-amino-4-{4-[(4-{[(7R)-8-cyclopentyl-7-ethyl-5-methyl-6-oxo-5,6,7,8-tetrahydropteridin-2-yl]amino}-3-methylbenzoyl)amino]piperidin-1-yl}butanoate,
Cyclopentyl (2*S*)-2-amino-4-{4-[(4-{[(7*R*)-8-cyclopentyl-7-ethyl-5-methyl-6-oxo-5,6,7,8-tetra hydropteridin-2-yl]amino}-3-methylbenzoyl)amino]piperidin-1-yl}butanoate,
t-butyl (2*S*)-2-amino-4-{4-[(4-{[(7*R*)-8-cyclopentyl-7-ethyl-5-methyl-6-oxo-5,6,7,8-tetrahydropteridin-2-yl]amino}-3-fluorobenzoyl)amino]piperidin-1-yl}butanoate, Cyclopentyl (2*S*)-2-amino-4-{4-[(4-{[(7*R*)-8-cyclopentyl-7-ethyl-5-methyl-6-oxo-5,6,7,8-tetra hydropteridin-2-yl]amino}-3-fluorobenzoyl)amino]piperidin-1-yl}butanoate,
and salts, N-oxides, hydrates or solvates thereof.

14. A pharmaceutical composition comprising a compound as claimed in any of the preceding claims, together with a pharmaceutically acceptable carrier.

15. A compound as claimed in any of claims 1 to 13 for use in the treatment of cell proliferative diseases.

## Patentansprüche

1. Verbindung der Formel (I) oder ein Salz, N-Oxid, Hydrat oder Solvat davon: 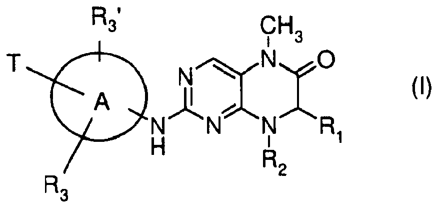 wobei
R₁ Wasserstoff oder eine gegebenenfalls substituierte (C₁-C₆)-Alkyl-, (C₂-C₆)-Alkenyl-, (C₂-C₆)-Alkinyl- oder (C₃-C₆)-Cycloalkylgruppe darstellt;
R₂ Wasserstoff oder eine gegebenenfalls substituierte (C₁-C₆)-Alkyl-, (C₂-C₆)-Alkenyl-, (C₂-C₆)-Alkinyl- oder (C₃-C₆)-Cycloalkylgruppe darstellt;
R₃ und R_{3'} unabhängig voneinander aus Wasserstoff, -CN, Hydroxyl, Halogen, gegebenenfalls substituiertem (C₁-C₆)-Alkyl, (C₂-C₆)-Alkenyl, (C₂-C₆)-Alkinyl oder (C₃-C₆)-Cycloalkyl, -NR₅R₆ oder C₁-C₄-Alkoxy ausgewählt sind, wobei R₅ und R₆ unabhängig voneinander Wasserstoff oder gegebenenfalls substituiertes (C₁-C₆)-Alkyl darstellen;
Ring A ein gegebenenfalls substituierter Phenyl-, Pyridinyl- oder Pyrimidinylring ist;
T ein Rest der Formel R-L¹-Y¹- ist, wobei
Y¹ eine Bindung, -O-, -S-, -NR₆-, -(C=O)-, -S(O₂)-, - (C=O)NR₆-, -NR₆(C=O)-, -S(O₂)NR₆-, -NR₆S(O₂)- oder -NR₆(C=O)NR₉- darstellt, wobei R₆ und R₉ unabhängig voneinander Wasserstoff oder gegebenenfalls substituiertes (C₁-C₆)-Alkyl darstellen;
L¹ ein divalenter Rest der Formel -(Alk¹)ₘ(Q)ₙ(Alk²)ₚ- darstellt, wobei
m, n und p unabhängig voneinander 0 oder 1 sind,
Q (i) ein gegebenenfalls substituierter, divalenter mono- oder bicyclischer, carbocyclischer oder heterocyclischer, 5- bis 13-gliedriger Rest ist oder (ii) falls p 0 ist, einen divalenten Rest der Formel -Q¹-X²- darstellt, wobei X² -O-, -S- oder NR^{A}- ist, wobei R^{A} Wasserstoff oder gegebenenfalls substituiertes C₁-C₃-Alkyl darstellt, und Q¹ ein gegebenenfalls substituierter, divalenter mono- oder bicyclischer, carbocyclischer oder heterocyclischer, 5- bis 13-gliedriger Rest ist,
Alk¹ und Alk² unabhängig voneinander gegebenenfalls substituierte divalente (C₃-C₆)-Cycloalkylreste oder gegebenenfalls substituierte, gerade oder verzweigte, (C₁-C₆)-Alkylen-, (C₂-C₆)-Alkenylen- oder (C₂-C₆)-Alkinylenreste darstellen, die gegebenenfalls eine Ether (-0-)-, Thioether (-S-)- oder Amino (-NR^{A}-)-Verknüpfung enthalten oder damit enden, wobei R^{A} Wasserstoff oder gegebenenfalls substituiertes (C₁-C₃)-Alkyl darstellt;
R einen Rest der Formel (X) oder (Y) darstellt 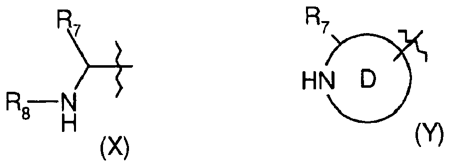 wobei
R₇ eine Carbonsäuregruppe (-COOH) oder eine Estergruppe darstellt, die durch ein oder mehrere intrazelluläre Carboxylesterase-Enzyme zu einer Carbonsäuregruppe hydrolysiert werden können;
R₆ Wasserstoff; oder gegebenenfalls substituiertes C₁-C₆-Alkyl, C₃-C₇-Cycloalkyl, Phenyl oder Pyridyl oder -(C=O)R₆, -(C=O)OR₆ oder -(C=)ONR₆ darstellt, wobei R₆ Wasserstoff oder gegebenenfalls substituiertes (C₁-C₆)-Alkyl darstellt; und
D ein monocyclischer, heterocyclischer Ring mit 5 oder 6 Ringatomen darstellt, wobei R₇ mit einem Ringkohlenstoff verknüpft ist, der an den dargestellten Ringstickstoff angrenzt, und Ring D gegebenenfalls mit einem zweiten carbocyclischen oder heterocyclischen Ring mit 5 oder 6 Ringatomen kondensiert ist, wobei die mit einer Wellenlinie durchschnittene Bindung von einem Ringatom in dem zweiten Ring stammen kann; und wobei, sofern nichts anderes angegeben ist, der Begriff "substituiert" mit bis zu vier passenden Substituenten substituiert bedeutet, jeweils unabhängig ausgewählt aus: (C₁-C₆)-Alkyl, (C₁-C₆)-Alkoxy, Hydroxy, Hydroxy-(C₁-C₆)-Alkyl, Mercapto, Mercapto-(C₁-C₆)-Alkyl, (C₁-C₆)-Alkylthio, Phenyl, Halo (inklusive Fluor, Brom und Chlor), Trifluormethyl, Trifluormethoxy, Nitro, Nitril (-CN), Oxo, -COOH, -COOR^{A}, -COR^{A}, -SO₂R^{A}, -CONH₂, -SO₂NH₂, -CONHR^{A}, -SO₂NHR^{A}, -CONR^{A}R^{B}, -SO₂NR^{A}R^{B}, -NH₂, -NHR^{A}, -NR^{A}R^{B}, -OCONH₂, -OCONHR^{A}, -OCONR^{A}R^{B},
-NHCOR^{A}, -NHCOOR^{A}, -NR^{B}COOR^{A}, -NHSO₂OR^{A}, -NR^{B}SO₂OR^{A}, -NHCONH₂, -NR^{A}CONH₂, -NHCONHR^{B}, -NR^{A}CONHR^{B}, -NHCONR^{A}R^{B} oder -NR^{A}CONR^{A}R^{B}, wobei R^{A} und R^{B} unabhängig voneinander (C₁-C₆)-Alkyl, (C₃-C₆)-Cycloalkyl, Phenyl oder monocyclisches Heteroaryl mit 5 oder 6 Ringatomen darstellen oder R^{A} und R^{B}, falls sie an dasselbe Stickstoffatom gebunden sind, eine cyclische Aminogruppe bilden.

2. Verbindung gemäß Anspruch 1, wobei R₁ Ethyl ist,

3. Verbindung gemäß Anspruch 1 oder Anspruch 2, wobei R₂ Cyclopentyl ist.

4. Verbindung gemäß einem der vorhergehenden Ansprüche, wobei Ring A ein Phenylring ist.

5. Verbindung gemäß einem der Ansprüche 1 bis 4, wobei R₃ Methoxy, Fluor oder Chlor ist und R'₃ Wasserstoff, Fluor oder Chlor ist.

6. Verbindung gemäß Anspruch 1 mit der Formel (IA): 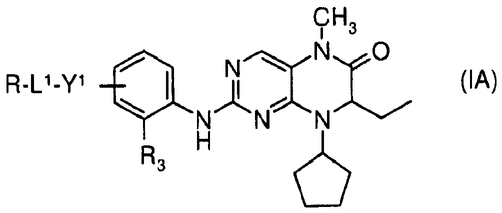 wobei R₃ Methoxy, Fluor oder Chlor ist und die übrigen Variablen wie in Anspruch 1 definiert sind.

7. Verbindung gemäß einem der vorhergehenden Ansprüche, wobei R₇ die Formel -(C=O)OR₁₀ besitzt, wobei R₁₀ R₁₁R₁₂R₁₃C- darstellt, wobei
(i) R₁₁ Wasserstoff oder gegebenenfalls substituiertes (C₁-C₃)-Alkyl-(Z¹)ₐ-[(C₁-C₃-alkyl]_{b}- oder (C₂-C₃)-Alkenyl-(Z¹)ₐ-[(C₁-C₃-alkyl]_{b}- darstellt, wobei a und b unabhängig voneinander 0 oder 1 sind und Z¹ -O-, -S- oder -NR₁₄ ist, wobei R₁₄ Wasserstoff oder (C₁-C₃)-Alkyl- darstellt; und R₁₂ und R₁₃ unabhängig voneinander Wasserstoff oder (C₁-C₃)-Alkyl darstellen;
(ii) R₁₁ Wasserstoff oder gegebenenfalls substituiertes R₁₅R₁₆N-(C₁-C₃)-Alkyl- darstellt, wobei R₁₅ Wasserstoff oder (C₁-C₃)-Alkyl ist und R₁₆ Wasserstoff oder (C₁-C₃)-Alkyl ist; oder R₁₅ und R₁₆ zusammen mit dem Stickstoff, an den sie gebunden sind, einen gegebenenfalls substituierten, monocyclischen, heterocyclischen Ring mit 5 oder 6 Ringatomen oder ein bicyclisches, heterocyclisches Ringsystem mit 8 bis 10 Ringatomen bilden und R₁₂ und R₁₃ unabhängig voneinander Wasserstoff oder (C₁-C₃)-Alkyl sind; oder
(iii) R₁₁ und R₁₂ zusammen mit dem Kohlenstoff, an den sie gebunden sind, einen gegebenenfalls substituierten, monocyclischen, carbocyclischen Ring mit 3 bis 7 Ringatomen oder ein bicyclisches, carbocyclisches Ringsystem mit 8 bis 10 Ringatomen bilden und R₁₃ Wasserstoff ist.

8. Verbindung gemäß Anspruch 7, wobei R₁₀ Cyclopentyl oder tert-Butyl ist.

9. Verbindung gemäß einem der vorhergehenden Ansprüche, wobei R ein Rest der Formel (X) und R₈ Wasserstoff ist.

10. Verbindung gemäß einem der vorhergehenden Ansprüche, wobei in dem Rest L¹, Y¹ -NHC(=O)- ist.

11. Verbindung gemäß einem der vorhergehenden Ansprüche, wobei in dem Rest L¹, Alk¹ und Alk², falls vorhanden, Reste ausgewählt aus -CH₂-, -CH₂CH₂-, -CH₂CH₂CH₂-, -CH₂CH(OH)CH₂-, -CH₂CH₂CH₂CH₂-, -CH=CH-, -CH=CHCH₂-, -CH₂CH=CH-, -CH₂CH=CHCH₂-, -C≡C-, -C≡CCH₂-, -CH₂C≡C-, -CH₂C≡CCH₂ -CH₂W-, -CH₂CH₂W-, -CH₂CH₂WCH₂-, -CH₂CH₂WCH(CH₃)-, -CH₂WCH₂CH₂-, -CH₂WCH₂CH₂WCH₂-, -WCH₂CH₂-, -CH₂CH₂N(CH₂CH₂OH)CH₂ und divalente Cyclopropyl-, Cyclopentyl- und Cyclohexylreste sind, wobei W -O-, -S-, -NH- oder -N(CH₃)- ist.

12. Verbindung gemäß einem der Ansprüche 1 bis 11, wobei Q, falls vorhanden, ein divalenter 1,4-Phenylen-, 1,4-Piperidinylen- oder 1,4-Piperazinylenrest ist.

13. Verbindung gemäß Anspruch 1, ausgewählt aus der Gruppe bestehend aus:
Cyclopentyl-4-[(4-{[(7R)-8-cyclopentyl-7-ethyl-5-methyl-6-oxo-5,6,7,8-tetrahydropteridin-2-yl]amino}-3-methoxybenzoyl)amino]-L-phenylalaninat,
Cyclopentyl-O-(4-{[4-{[(7R)-8-cyclopentyl-7-ethyl-5-methyl-6-oxo-5,6,7,8-tetrahydropteridin-2-yl]amino}-3-methoxybenzoyl)amino]methyl}phenyl)-L-homoserinat,
tert-Butyl-4-[(4-{[(7R)-8-cyclopentyl-7-ethyl-5-methyl-6-oxo-5,6,7,8-tetrahydropteridin-2-yl]amino}-3-methoxybenzoyl)amino]-L-phenylalaninat, tert-Butyl-O-(4-{[4-{[(7R)-8-cyclopentyl-7-ethyl-5-methyl-6-oxo-5,6,7,8-tetrahydropteridin-2-yl]amino}-3-methoxybenzoyl)amino]methyl}phenyl)-L-homoserinat,
Cyclopentyl-4-{2-[(4-{[(7R)-8-cyclopentyl-7-ethyl-5-methyl-6-oxo-5,6,7,8-tetrahydropteridin-2-yl]amino}-3-methoxybenzoyl)amino]ethyl}piperazin-2-carboxylat,
tert-Butyl-4-{2-[(4-{[(7R)-8-cyclopentyl-7-ethyl-5-methyl-6-oxo-5,6,7,8-tetrahydropteridin-2-yl]amino}-3-methoxybenzoyl)amino]-ethyl}piperazin-2-carboxylat,
Cyclopentyl-(2S)-2-amino-4-{4-[(4{[(7R)-8-cyclopentyl-7-ethyl-5-methyl-6-oxo-5,6,7,8-tetrahydropteridin-2-yl]amino}-3-methoxybenzoyl)amino]piperidin-1-yl}butanoat,
tert-Butyl-5-{4-[(4-{[(7R)-8-cyclopentyl-7-ethyl-5-methyl-6-oxo-5,6,7,8-tetrahydropteridin-2-yl]amino}-3-methoxybenzoyl)amino]piperidin-1-yl}-L-norvalinat,
Cyclopentyl-5-{4-[(4-{[(7R)-8-cyclopentyl-7-ethyl-5-methyl-6-oxo-5,6,7,8-tetrahydropteridin-2-yl]amino}-3-methoxybenzoyl)amino]piperidin-1-yl}-L-norvalinat,
t-Butyl-(2S)-2-amino-4-{4-[(4-{[(7R)-8-cyclopentyl-7-ethyl-5-methyl-6-oxo-5,6,7,8-tetrahydropteridin-2-yl]amino}-3-methoxybenzoyl)amino]piperidin-1-yl}butanoat,
t-Butyl-(2S)-2-amino-4-{4-[(4-{[(7R)-8-cyclopentyl-7-ethyl-5-methyl-6-oxo-5,6,7,8-tetrahydropteridin-2-yl]amino}-3-methylbenzoyl)amino]piperidin-1-yl}butanoat,
Cyclopentyl-(2S)-2-amino-4-{4-[(4-{[(7R)-8-cyclopentyl-7-ethyl-5-methyl-6-oxo-5,6,7,8-tetrahydropteridin-2-yl]amino}-3-methylbenzoyl)amino]piperidin-1-yl}butanoat, t-Butyl-(2S)-2-amino-4-{4-[(4-{[(7R)-8-cyclopentyl-7-ethyl-5-methyl-6-oxo-5,6,7,8-tetrahydropteridin-2-yl]amino}-3-fluorbenzoyl)amino]piperidin-1-yl}butanoat,
Cyclopentyl-(2S)-2-amino-4-{4-[(4-{[(7R)-8-cyclopentyl-7-ethyl-5-methyl-6-oxo-5,6,7,8-tetrahydropteridin-2-yl]amino}-3-fluorbenzoyl)amino]piperidin-1-yl}butanoat,
und Salze, N-Oxide, Hydrate oder Solvate davon.

14. Pharmazeutische Zusammensetzung, umfassend eine Verbindung gemäß einem der vorhergehenden Ansprüche zusammen mit einem pharmazeutisch akzeptablen Träger.

15. Verbindung gemäß einem der Ansprüche 1 bis 13 zur Anwendung bei der Behandlung von Zellproliferationskrankheiten.

## Revendications

1. Composé de formule (I), ou sel, N-oxyde, hydrate ou solvate de celui-ci : 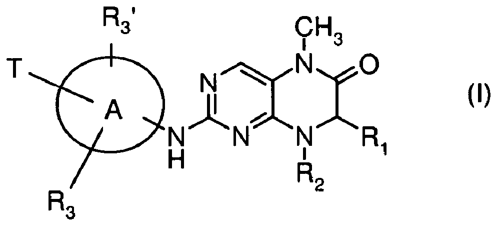 dans lequel
**R₁** est un hydrogène, ou un groupe alkyle en (C₁-C₆), alcényle en (C₂-C₆), alcynyle en (C₂-C₆) ou cycloalkyle en (C₃-C₆) éventuellement substitué ;
**R₂** est un hydrogène, ou un groupe alkyle en (C₁-C₆), alcényle en (C₂-C₆), alcynyle en (C₂-C₆) ou cycloalkyle en (C₃-C₆) éventuellement substitué ;
**R₃** et **R₃** sont, indépendamment l'un de l'autre, choisis parmi un hydrogène, -CN, un hydroxyle, un halogène, un alkyle en (C₁-C₆), alcényle en (C₂-C₆), alcynyle en (C₂-C₆) ou cycloalkyle en (C₃-C₆) éventuellement substitué, -NR₅R₆ ou un alcoxy en C₁-C₄, où R₅ et R₆ sont, indépendamment l'un de l'autre, un hydrogène ou un alkyle en (C₁-C₆) éventuellement substitué ;
le cycle **A** est un cycle phényle, pyridinyle ou pyrimidinyle éventuellement substitué ;
**T** est un radical de formule R-L¹-Y¹- dans lequel
**Y¹** est une liaison, -O-, -S-, -NR₆-, -(C=O)-, -S(O)₂-, -(C=O)NR₆-, -NR₆(C=O)-, -S(O₂)NR₆-, -NR₆S(O₂)-, ou -NR₆(C=O)NR₉-, où R₆ et R₉ sont, indépendamment l'un de l'autre, un hydrogène ou un alkyle en (C₁-C₆) éventuellement substitué ;
**L¹** est un radical divalent de formule -(Alk¹)ₘ(Q)ₙ(Alk²)ₚ- dans laquelle
**m, n** et **p** sont, indépendamment les uns des autres, 0 ou 1,
**Q** est (i) un radical divalent mono- ou bicyclique carbocyclique ou hétérocyclique éventuellement substitué comportant 5 à 13 chaînons de cycle, ou (ii), lorsque p est égal à 0, un radical divalent de formule -Q¹-X²- dans laquelle X² est -O-, S- ou NR^{A}- où R^{A} est un hydrogène ou un alkyle en C₁-C₃ éventuellement substitué, et Q¹ est un radical divalent mono- ou bicyclique, carbocyclique ou hétérocyclique,
éventuellement substitué comportant, 5 à 13 chaînons de cycle, **Alk¹** et **Alk²** représentent, indépendamment l'un de l'autre, des radicaux divalents cycloalkyle en (C₃-C₆) éventuellement substitués,
ou des radicaux linéaires ou ramifiés éventuellement substitués alkylène en (C₁-C₆), alcénylène en (C₂-C₆) ou alcynylène en (C₂-C₆) qui peuvent éventuellement contenir ou se terminer par une liaison éther (-O-), thioéther (-S-) ou amino (-NR^{A}-) où R^{A} est un hydrogène ou un alkyle en (C₁-C₃) éventuellement substitué ;
**R** est un radical de formule (X) ou (Y) 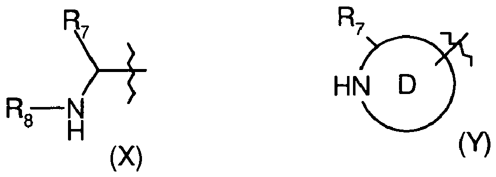 dans laquelle
**R₇** est un groupe acide carboxylique (-COOH), ou un groupe ester qui est hydrolysable, par une ou plusieurs enzymes carboxylestérase intracellulaires, en un groupe acide carboxylique ;
**R₈** est un hydrogène ; ou un groupe éventuellement substitué alkyle en C₁-C₆, cycloalkyle en C₃-C₇, phényle ou pyridyle ou -(C=O)R₆,-(C=O)OR₆, ou -(C=O)NR₆ où R₆ est un hydrogène ou un alkyle en (C₁-C₆) éventuellement substitué ; et
**D** est un cycle monocyclique hétérocyclique à 5 ou 6 atomes de cycle dans lequel R₇ est lié à un carbone de cycle adjacent à l'azote de cycle représenté, et le cycle D est éventuellement condensé à un second cycle carbocyclique ou hétérocyclique à 5 ou 6 atomes de cycle, auquel cas la liaison représentée, coupée par une ligne ondulée, peut provenir d'un atome de cycle dudit second cycle ; et où, sauf mention contraire, le terme « substitué » signifie substitué par jusqu'à quatre substituants compatibles, chacun d'entre eux, indépendamment des autres, étant choisi parmi : un alkyle en (C₁-C₆), un alcoxy en (C₁-C₆), un hydroxy, un hydroxy-alkyle en (C₁-C₆), un mercapto, un mercapto-alkyle en (C₁-C₆), un alkyl en (C₁-C₆)-thio, un phényle, un halo (comprenant fluoro, bromo et chloro), un trifluorométhyle, un trifluorométhoxy, un nitro, un nitrile (-CN), un oxo, -COOH, -COOR^{A}, -COR^{A}, SO₂R^{A}, -CONH₂, -SO₂NH₂, -CONHR^{A}, -SO₂NHR^{A},-CONR^{A}R^{B}, -SO₂NR^{A}R^{B}, -NH₂, -NHR^{A}, -NR^{A}R^{B}, -OCONH₂, -OCONHR^{A}, -OCONR^{A}R^{B}, -NHCOR^{A}, -NHCOOR^{A}, -NR^{B}COOR^{A}, -NHSO₂OR^{A},-NR^{B}SO₂OH, -NR^{B}SO₂OR^{A}, -NHCONH₂, -NR^{A}CONH₂, -NHCONHR^{B}, - NR^{A}CONHR^{B}, -NHCONR^{A}R^{B}, ou -NR^{A}CONR^{A}R^{B} où R^{A} et R^{B} sont, indépendamment l'un de l'autre, un alkyle en (C₁-C₆), un cycloalkyle en (C₃-C₆), un phényle ou un hétéroaryle monocyclique comportant 5 ou 6 atomes de cycle, ou R^{A} et R^{B}, lorsqu'ils sont liés au même atome d'azote, forment un groupe amino cyclique.

2. Composé selon la revendication 1, dans lequel R₁ est un éthyle.

3. Composé selon la revendication 1 ou la revendication 2, dans lequel R₂ est un cyclopentyle.

4. Composé selon l'une quelconque des revendications précédentes, dans lequel le cycle A est un cycle phényle.

5. Composé selon l'une quelconque des revendications 1 à 4, dans lequel R₃ est un méthoxy, un fluoro ou un chloro, et R'₃ est un hydrogène, un fluoro ou un chloro.

6. Composé selon la revendication 1 répondant à la formule (IA) : 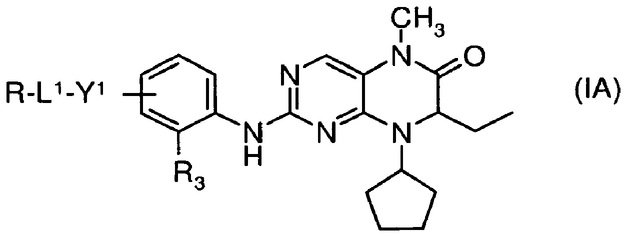 dans laquelle R₃ est un méthoxy, un fluoro ou un chloro, et les variables restantes sont telles que définies dans la revendication 1.

7. Composé selon l'une quelconque des revendications précédentes, dans lequel R₇ a la formule -(C=O)OR₁₀ où R₁₀ est R₁₁R₁₂R₁₃C- où
(i) R₁₁ est un hydrogène ou un alkyle en (C₁-C₃)-(Z¹)ₐ-[alkyle en (C₁-C₃)]_{b}- ou alcényle en (C₂-C₃)-(Z¹)ₐ-[alkyle en (C₁-C₃)]_{b}- éventuellement substitué, où a et b sont, indépendamment l'un de l'autre, 0 ou 1, et Z¹ est-O-, -S-, ou -NR₁₄- où R₁₄ est un hydrogène ou un alkyle en (C₁-C₃) ; et R₁₂ et R₁₃ sont, indépendamment l'un de l'autre, un hydrogène ou un alkyle en (C₁-C₃) ;
(ii) R₁₁ est un hydrogène ou un R₁₅R₁₆N-alkyle en (C₁-C₃)-éventuellement substitué où R₁₅ est un hydrogène ou un alkyle en (C₁-C₃) et R₁₆ est un hydrogène ou un alkyle en (C₁-C₃) ; ou R₁₅ et R₁₆, conjointement avec l'azote auquel ils sont liés, forment un cycle monocyclique hétérocyclique à 5 ou 6 atomes de cycle ou système cyclique hétérocyclique bicyclique à 8 à 10 atomes de cycle éventuellement substitué, et R₁₂ et R₁₃ sont, indépendamment l'un de l'autre, un hydrogène ou un alkyle en (C₁-C₃) ; ou
(iii) R₁₁ et R₁₂, considérés conjointement avec le carbone auquel ils sont liés, forment un cycle monocyclique carbocyclique à 3 à 7 atomes de cycle ou système cyclique bicyclique carbocyclique à 8 à 10 atomes de cycle éventuellement substitué, et R₁₃ est un hydrogène.

8. Composé selon la revendication 7, dans lequel R₁₀ est un cyclopentyle ou un tert-butyle.

9. Composé selon l'une quelconque des revendications précédentes, dans lequel R est un radical de formule (X) et R₈ est un hydrogène.

10. Composé selon l'une quelconque des revendications précédentes, dans lequel, dans le radical L¹, Y¹ est -NHC(=O)-.

11. Composé selon l'une quelconque des revendications précédentes, dans lequel, dans le radical L¹, les radicaux Alk¹ et Alk², lorsqu'ils sont présents, sont choisis parmi -CH₂-, -CH₂CH₂-, -CH₂CH₂CH₂-,-CH₂CH(OH)CH₂-, -CH₂CH₂CH₂CH₂-, -CH=CH-, -CH=CHCH₂-, -CH₂CH=CH-,-CH₂CH=CHCH₂-, -C≡C-, -C≡CCH₂-, -CH₂C≡C-, CH₂C≡CCH₂ -CH₂W-, -CH₂CH₂W-, -CH₂CH₂WCH₂-, -CH₂CH₂WCH(CH₃)-, -CH₂WCH₂CH₂-, -CH₂WCH₂CH₂WCH₂-,-WCH₂CH₂-, -CH₂CH₂N(CH₂CH₂OH)CH₂, et les radicaux divalents cyclopropyle, cyclopentyle et cyclohexyle ; W étant -O-, -S-, -NH-, ou-N(CH₃)-.

12. Composé selon l'une quelconque des revendications 1 à 11, dans lequel Q, lorsqu'il est présent, est un radical divalent 1,4-phénylène, 1,4-pipéridinylène, ou 1,4-pipérazinylène.

13. Composé selon la revendication 1, choisi dans le groupe constitué par :
le 4-[(4-{[(*7R*)-8-cyclopentyl-7-éthyl-5-méthyl-6-oxo-5,6,7,8-tétrahydroptéridin-2-yl]amino}-3-méthoxybenzoyl)amino]-L-phényl-alaninate de cyclopentyle,
le *O*-(4-{[(4-{[(*7R*)-8-cyclopentyl-7-éthyl-5-méthyl-6-oxo-5,6,7,8-tétrahydroptéridin-2-yl]amino}-3-méthoxybenzoyl)amino]méthyl}phényl)-L-homosérinate de cyclopentyle,
le 4-[(4-{[(*7R*)-8-cyclopentyl-7-éthyl-5-méthyl-6-oxo-5,6,7,8-tétrahydroptéridin-2-yl]amino}-3-méthoxybenzoyl)amino]-L-phényl-alaninate de *tert*-butyle,
le *O*-(4-{[(4-{[(*7R*)-8-cyclopentyl-7-éthyl-5-méthyl-6-oxo-5,6,7,8-tétrahydroptéridin-2-yl]amino}-3-méthoxybenzoyl)amino]méthyl}phényl)-L-homosérinate de *tert*-butyle,
le 4-{2-[(4-{[(*7R*)-8-cyclopentyl-7-éthyl-5-méthyl-6-oxo-5,6,7,8-tétrahydroptéridin-2-yl]amino}-3-méthoxybenzoyl)amino]éthyl}pipérazine-2-carboxylate de cyclopentyle,
le 4-{2-[(4-{[(*7R*)-8-cyclopentyl-7-éthyl-5-méthyl-6-oxo-5,6,7,8-tétrahydroptéridin-2-yl]amino}-3-méthoxybenzoyl)amino]éthyl}pipérazine-2-carboxylate de *tert*-butyle,
le (*2S*)-2-amino-4-{4-[(4-{[(*7R*)-8-cyclopentyl-7-éthyl-5-méthyl-6-oxo-5,6,7,8-tétrahydroptéridin-2-yl]amino}-3-méthoxybenzoyl)amino]-pipéridin-1-yl}butanoate de cyclopentyle,
le 5-{4-[(4-{[(*7R*)-8-cyclopentyl-7-éthyl-5-méthyl-6-oxo-5,6,7,8-tétrahydroptéridin-2-yl]amino}-3-méthoxybenzoyl)amino]pipéridin-1-yl}-L-norvalinate de *tert*-butyle,
le 5-{4-[(4-{[(*7R*)-8-cyclopentyl-7-éthyl-5-méthyl-6-oxo-5,6,7,8-tétrahydroptéridin-2-yl]amino}-3-méthoxybenzoyl)amino]pipéridin-1-yl}-L-norvalinate de cyclopentyle,
le (*2S*)-2-amino-4-{4-[(4-{[(*7R*)-8-cyclopentyl-7-éthyl-5-méthyl-6-oxo-5,6,7,8-tétrahydroptéridin-2-yl]amino}-3-méthoxybenzoyl)amino]-pipéridin-1-yl}butanoate de t-butyle,
le (*2S*)-2-amino-4-{4-[(4-{[(*7R*)-8-cyclopentyl-7-éthyl-5-méthyl-6-oxo-5,6,7,8-tétrahydroptéridin-2-yl]amino}-3-méthylbenzoyl)amino]-pipéridin-1-yl}butanoate de t-butyle,
le (*2S*)-2-amino-4-{4-[(4-{[(*7R*)-8-cyclopentyl-7-éthyl-5-méthyl-6-oxo-5,6,7,8-tétrahydroptéridin-2-yl]amino}-3-méthylbenzoyl)amino]-pipéridin-1-yl}butanoate de cyclopentyle,
le (*2S*)-2-amino-4-{4-[(4-{[(*7R*)-8-cyclopentyl-7-éthyl-5-méthyl-6-oxo-5,6,7,8-tétrahydroptéridin-2-yl]amino}-3-fluorobenzoyl)amino]-pipéridin-1-yl}butanoate de t-butyle,
le (*2S*)-2-amino-4-{4-[(4-{[(*7R*)-8-cyclopentyl-7-éthyl-5-méthyl-6-oxo-5,6,7,8-tétrahydroptéridin-2-yl]amino}-3-fluorobenzoyl)amino]-pipéridin-1-yl}butanoate de cyclopentyle,
et les sels, N-oxydes, hydrates ou solvates de ceux-ci.

14. Composition pharmaceutique comprenant un composé selon l'une quelconque des revendications précédentes, conjointement avec un véhicule pharmaceutiquement acceptable.

15. Composé selon l'une quelconque des revendications 1 à 13, destiné à être utilisé dans le traitement de maladies liées à une prolifération des cellules.
